# EUROPEAN PATENT APPLICATION

(11) **EP 4 501 925 A1**
(43) Date of publication of application: **05.02.2025**
(21) Application number: 23778158.8
(22) Date of filing: 28.03.2023
(51) Int. Cl.: C07D 403/12, C07D 487/04, A61K 31/517, A61K 31/519, A61P 35/00

(54) **NITROGEN-CONTAINING HETEROCYCLIC COMPOUND, PREPARATION METHOD THEREFOR, AND PHARMACEUTICAL APPLICATION THEREOF**

(30) Priority: 28.03.2022 CN 202210330007; 05.08.2022 CN 202210944231; 13.10.2022 CN 202211255163
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); WANG, Bin, Shanghai 200245 (CN); DONG, Huaide, Shanghai 200245 (CN); HE, Feng, Shanghai 200245 (CN); TAO, Weikang, Shanghai 200245 (CN)
(74) Representative: Potter Clarkson
(86) International application number: PCT/CN2023/084265
(87) International publication number: WO 2023/185793

(57) **Abstract**

The present disclosure relates to a nitrogen-containing heterocyclic compound, a preparation method therefor, and a pharmaceutical application thereof. Specifically, the present disclosure relates to a nitrogen-containing heterocyclic compound represented by general formula (I), a preparation method therefor, a pharmaceutical composition containing same, and a use thereof as a therapeutic agent, especially a use as an HER2 inhibitor and a use in the preparation of a drug for treating and/or preventing diseases or disorders by inhibiting HER2.

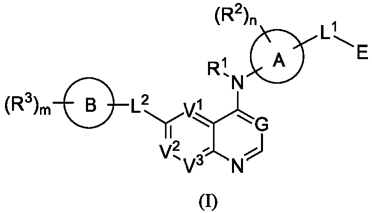

## Description

### TECHNICAL FIELD

The present disclosure belongs to the field of pharmaceutics, and relates to a nitrogen heterocyclic compound, a preparation method therefor, and pharmaceutical use thereof. Specifically, the present disclosure relates to a nitrogen-containing heterocyclic compound of general formula (I), a preparation method therefor, a pharmaceutical composition comprising the compound, and use thereof as a therapeutic agent, particularly use thereof as a HER2 inhibitor and use thereof in the preparation of a drug for treating and/or preventing diseases or disorders by inhibiting HER2.

### BACKGROUND

Human epidermal growth factor receptor 2 (HER2; Neu, ERBB2) is a member of the type I receptor tyrosine kinase family, which also includes EGFR (ERBB1), HER3 (ERBB3), and HER4 (ERBB4). To date, no ligand that can directly bind to HER2 has yet been found in humans, and HER2 must form a homodimer or heterodimer with other members of the family (e.g., HER3). HER2 undergoes conformational changes after dimerization, activating intracellular tyrosine kinase activity, subsequently reactivating downstream pathways (MAPK signaling pathway and PI3K/AKT signaling pathway), thereby exerting corresponding physiological effects.

Abnormal HER2 signaling has been found in a variety of human malignancies. The extracellular region, juxtamembrane region, and intracellular region of HER2 can have oncogenic mutations. Overall, these mutations enable HER2 to have the sustained activity, promoting the development of cancers, as well as maintenance and growth of tumors. It is the basis of tumor transformation and tumor maintenance in various tumors including breast cancer, gastric cancer, or lung cancer. HER2 overexpression increases HER2 signaling, particularly in breast cancer, where HER2 amplification is correlated with poor survival outcomes. HER2 mutations are present in 6-7% of all cancers in humans. Thus, disruption of HER2 oncogenic signals can be effective in treating tumors driven by HER2 oncogenic mutations or HER2 wild-type amplification. Currently, there are a number of drugs against HER2 that have been approved by the FDA for treating breast cancer, including antibodies against HER2 (trastuzumab and pertuzumab), antibody-drug conjugates against HER2 (trastuzumab-DM1 (T-DM1, ado-trastuzumab emtansine), and small molecules that inhibit HER2 kinase domains (afatinib, neratinib, lapatinib, tucatinib, and pyrotinib).

Although these drugs can inhibit the HER2 wild type, e.g., tucatinib, these inhibitors are not effective against HER2 carrying exon 20 mutations. Mutations in exon 20 of the HER2 gene lead to enhanced kinase activity (Wang et al., Cancer Cell, 2006, 10(1): 25-38). This enhanced HER2 kinase activity enters downstream signaling cascade and stimulates tumor transformation by promoting the growth, proliferation, and survival of cells. Genetic studies in mouse models have shown that non-small cell lung cancer is the most common exon 20 mutation of HER2, which is an insertion of 4 amino acids YVMA (p.A775_G776insYVMA) and can drive oncogenic growth. Reduction of the HER2-YVMA expression can result in tumor shrinkage, which indicates that this oncogenic variant of HER2 is essential for tumor maintenance. In addition, a broad-spectrum ERBB inhibitor, afatinib, can effectively interfere with the oncogenic signaling of HER2-YVMA *in vivo.*

According to statistics, about 2-4% of patients with lung cancer carry activating mutations in exon 20 of HER2. Currently, clinically approved ERBB-targeted tyrosine kinase inhibitors are almost ineffective in these patients, primarily due to EGFR wild-type-mediated dose-limiting toxicity. Allitinib, ibrutinib, neratinib, poziotinib, and pyrotinib are known broad-spectrum ERBB inhibitors of mutant HER2 exon 20. However, clinically, afatinib and other broad-spectrum ERBB inhibitors show only limited efficacy in NSCLC patients with HER2 exon 20 mutations, due to the limitation of effective dose. Therefore, there is an urgent medical need to develop a molecule that specifically inhibits HER2 exon 20 mutant proteins but has no effect on the EGFR wild type, thereby overcoming the disadvantages of EGFR wild-type-mediated dose-limiting toxicity. The present invention is intended to provide a novel inhibitor of mutant HER2 exon 20 that is selective for the EGFR wild type.

A series of patent applications for HER2 inhibitors are currently disclosed, including WO2007059257A1, WO2017148391A1, WO2021213800A1, and WO2021156178A1.

### SUMMARY

The present disclosure aims to provide a compound of general formula (I) or a pharmaceutically acceptable salt thereof: 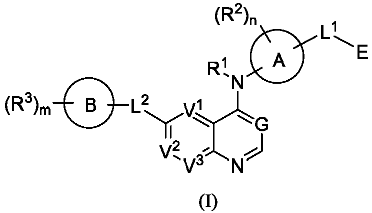 wherein:
ring A is aryl or heteroaryl;
ring B is 7- to 10-membered fused heterocyclyl or 7- to 10-membered bridged heterocyclyl;
G is N or C(R^{A});
R^{A} is selected from the group consisting of a hydrogen atom, halogen, alkyl, haloalkyl, hydroxyalkyl, alkoxy, haloalkoxy, cyano, hydroxy, and amino;
V¹ is C(R^{a}) or N;
V² and V³ are identical or different and are each independently C(R^{a}) or N; or V² is C(R^{bb}), V³ is C(R^{cc}), and R^{bb} and R^{cc}, together with the carbon atom to which they are each attached, form cycloalkyl, heterocyclyl, aryl, or heteroaryl, wherein the cycloalkyl, heterocyclyl, aryl, or heteroaryl is optionally substituted with one or more substituents selected from the group consisting of halogen, alkyl, haloalkyl, hydroxyalkyl, alkoxy, haloalkoxy, cyano, hydroxy, amino, nitro, cycloalkyl, heterocyclyl, aryl, and heteroaryl;
L¹ is selected from the group consisting of O, NR^{b1}, C(O), S, S(O), and S(O)₂;
L² is selected from the group consisting of O, NR^{b2}, C(O), (CR^{c}R^{d})ᵤ, (CR^{c}R^{d})ᵤO, O(CR^{c}R^{d})ᵤ, (CR^{c}R^{d})ᵤNR^{b2}, NR^{b2}(CR^{c}R^{d})ᵤ, C(O)NR^{b2}, and NR^{b2}C(O);
E is 9- to 10-membered heteroaryl, wherein the 9- to 10-membered heteroaryl is optionally substituted with one or more R¹⁶;
R¹⁶ is selected from the group consisting of halogen, alkyl, alkenyl, alkynyl, cyano, nitro, -OR⁴, -NR⁵R⁶, -C(O)R⁴, -C(O)OR⁴, -OC(O)R⁴, -C(O)NR⁵R⁶, -S(O)ₚR⁴, -S(O)ₚNR⁵R⁶, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, aminoalkyl, cyano, -OR^{4a}, -NR^{5a}R^{6a}, cycloalkyl, heterocyclyl, aryl, and heteroaryl;
R^{a} is selected from the group consisting of a hydrogen atom, halogen, alkyl, alkenyl, alkynyl, cyano, nitro, haloalkyl, hydroxyalkyl, -OR^{e}, -(CH₂)ₛ-NR^{f}R^{g}, cycloalkyl, heterocyclyl, aryl, and heteroaryl;
R^{b1} and R^{b2} are identical or different and are each independently selected from the group consisting of a hydrogen atom, alkyl, haloalkyl, hydroxyalkyl, cycloalkyl, and heterocyclyl;
R^{c} and R^{d} are identical or different and are each independently selected from the group consisting of a hydrogen atom, halogen, alkyl, hydroxy, and hydroxyalkyl;
R¹ is selected from the group consisting of a hydrogen atom, alkyl, and cycloalkyl;
each R² is identical or different and is independently selected from the group consisting of halogen, alkyl, alkenyl, alkynyl, cyano, nitro, haloalkyl, hydroxyalkyl, -OR⁷, -(CH₂)ᵥ-NR⁸R⁹, cycloalkyl, heterocyclyl, aryl, and heteroaryl;
each R³ is identical or different and is independently selected from the group consisting of oxo, halogen, alkyl, alkenyl, alkynyl, cyano, nitro, -OR¹⁰, -NR¹¹R¹², -C(O)R¹⁰, - C(O)OR¹⁰, -OC(O)R¹⁰, -C(O)NR¹¹R¹², -NR¹³C(O)R¹⁰, -NR¹³C(O)OR¹⁰, - NR¹³C(O)NR¹¹R¹², -S(O)ₚR¹⁰, -S(O)ₚNR¹¹R¹², -NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl;
R¹⁰ are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more R^{B};
R^{B} is selected from the group consisting of oxo, halogen, alkyl, alkenyl, alkynyl, cyano, -OR^{10b}, -NR^{11b}R^{12b}, -C(O)R^{10b}, -C(O)NR^{11b}R^{12b}, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, alkoxy, hydroxy, cyano, and amino;
R^{e}, R⁴, R^{4a}, R⁷, R^{10a}, and R^{10b} are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, amino, cycloalkyl, heterocyclyl, aryl, and heteroaryl;
R^{f}, R^{g}, R⁵, R⁶, R⁸, R⁹, R¹¹, and R¹² are identical or different at each occurrence and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
or R^{f} and R^{g}, together with the nitrogen atom to which they are attached, form heterocyclyl, or R⁵ and R⁶, together with the nitrogen atom to which they are attached, form heterocyclyl, or R⁸ and R⁹, together with the nitrogen atom to which they are attached, form heterocyclyl, or R¹¹ and R¹², together with the nitrogen atom to which they are attached, form heterocyclyl, wherein the heterocyclyl is optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
R¹³ are identical or different at each occurrence and are each independently selected from the group consisting of a hydrogen atom, alkyl, and cycloalkyl;
R^{5a}, R^{6a} , R^{11a}, R^{12a} , R^{11b}, and R^{12b} are identical or different at each occurrence and are each independently selected from the group consisting of a hydrogen atom, alkyl, haloalkyl, hydroxyalkyl, aminoalkyl, cycloalkyl, heterocyclyl, aryl, heteroaryl, cycloalkylalkyl, heterocyclylalkyl, arylalkyl, and heteroarylalkyl;
s is 0, 1, or 2;
v is 0, 1, or 2;
u is 1, 2, 3, or 4;
p is 0, 1, or 2;
n is 0, 1, 2, 3, or 4; and
m is an integer from 0 to 10.

In some embodiments of the present disclosure, the compound of general formula (I) or the pharmaceutically acceptable salt thereof is provided, wherein:
ring A is aryl or heteroaryl;
ring B is 7- to 10-membered fused heterocyclyl or 7- to 10-membered bridged heterocyclyl;
G is N or C(R^{A});
R^{A} is selected from the group consisting of a hydrogen atom, halogen, alkyl, haloalkyl, hydroxyalkyl, alkoxy, haloalkoxy, cyano, hydroxy, and amino;
V¹, V², and V³ are identical or different and are each independently C(R^{a}) or N;
L¹ is selected from the group consisting of O, NR^{b1}, C(O), S, S(O), and S(O)₂;
L² is selected from the group consisting of O, NR^{b2}, C(O), (CR^{c}R^{d})ᵤ, (CR^{c}R^{d})ᵤO, O(CR^{c}R^{d})ᵤ, (CR^{c}R^{d})ᵤNR^{b2}, NR^{b2}(CR^{c}R^{d})ᵤ, C(O)NR^{b2}, and NR^{b2}C(O);
E is 9- to 10-membered heteroaryl, wherein the 9- to 10-membered heteroaryl is optionally substituted with one or more R¹⁶;
R¹⁶ is selected from the group consisting of halogen, alkyl, alkenyl, alkynyl, cyano, nitro, -OR⁴, -NR⁵R⁶, -C(O)R⁴, -C(O)OR⁴, -OC(O)R⁴, -C(O)NR⁵R⁶, -S(O)ₚR⁴, -S(O)ₚNR⁵R⁶, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, aminoalkyl, cyano, -OR^{4a}, -NR^{5a}R^{6a}, cycloalkyl, heterocyclyl, aryl, and heteroaryl;
R^{a} is selected from the group consisting of a hydrogen atom, halogen, alkyl, alkenyl, alkynyl, cyano, nitro, haloalkyl, hydroxyalkyl, -OR^{e}, -(CH₂)ₛ-NR^{f}R^{g}, cycloalkyl, heterocyclyl, aryl, and heteroaryl;
R^{b1} and R^{b2} are identical or different and are each independently selected from the group consisting of a hydrogen atom, alkyl, haloalkyl, hydroxyalkyl, cycloalkyl, and heterocyclyl;
R^{c} and R^{d} are identical or different and are each independently selected from the group consisting of a hydrogen atom, halogen, alkyl, hydroxy, and hydroxyalkyl;
R¹ is selected from the group consisting of a hydrogen atom, alkyl, and cycloalkyl;
each R² is identical or different and is independently selected from the group consisting of halogen, alkyl, alkenyl, alkynyl, cyano, nitro, haloalkyl, hydroxyalkyl, -OR⁷, -(CH₂)ᵥ-NR⁸R⁹, cycloalkyl, heterocyclyl, aryl, and heteroaryl;
each R³ is identical or different and is independently selected from the group consisting of oxo, halogen, alkyl, alkenyl, alkynyl, cyano, nitro, -OR¹⁰, -NR¹¹R¹², -C(O)R¹⁰, - C(O)OR¹⁰, -OC(O)R¹⁰, -C(O)NR¹¹R¹², -NR¹³C(O)R¹⁰, -NR¹³C(O)OR¹⁰, - NR¹³C(O)NR¹¹R¹², -S(O)ₚR¹⁰, -S(O)ₚNR¹¹R¹², -NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl;
R¹⁰ are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more R^{B};
R^{B} is selected from the group consisting of oxo, halogen, alkyl, alkenyl, alkynyl, cyano, -OR^{10b}, -NR^{11b}R^{12b}, -C(O)R^{10b}, -C(O)NR^{11b}R^{12b}, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, alkoxy, hydroxy, cyano, and amino;
R^{e}, R⁴, R^{4a}, R⁷, R^{10a}, and R^{10b} are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, amino, cycloalkyl, heterocyclyl, aryl, and heteroaryl;
R^{f}, R^{g}, R⁵, R⁶, R⁸, R⁹, R¹¹, and R¹² are identical or different at each occurrence and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
or R^{f} and R^{g}, together with the nitrogen atom to which they are attached, form heterocyclyl, or R⁵ and R⁶, together with the nitrogen atom to which they are attached, form heterocyclyl, or R⁸ and R⁹, together with the nitrogen atom to which they are attached, form heterocyclyl, or R¹¹ and R¹², together with the nitrogen atom to which they are attached, form heterocyclyl, wherein the heterocyclyl is optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
R¹³ are identical or different at each occurrence and are each independently selected from the group consisting of a hydrogen atom, alkyl, and cycloalkyl;
R^{5a}, R^{6a} , R^{11a}, R^{12a} , R^{11b}, and R^{12b} are identical or different at each occurrence and are each independently selected from the group consisting of a hydrogen atom, alkyl, haloalkyl, hydroxyalkyl, aminoalkyl, cycloalkyl, heterocyclyl, aryl, heteroaryl, cycloalkylalkyl, heterocyclylalkyl, arylalkyl, and heteroarylalkyl;
s is 0, 1, or 2;
v is 0, 1, or 2;
u is 1, 2, 3, or 4;
p is 0, 1, or 2;
n is 0, 1, 2, 3, or 4; and
m is an integer from 0 to 10.

In some embodiments of the present disclosure, the compound of general formula (I) or the pharmaceutically acceptable salt thereof is provided, wherein L¹ is O.

In some embodiments of the present disclosure, the compound of general formula (I) or the pharmaceutically acceptable salt thereof is provided, wherein ring A is 6- to 10-membered aryl or 5- to 10-membered heteroaryl; preferably, ring A is phenyl.

In some embodiments of the present disclosure, the compound of general formula (I) or the pharmaceutically acceptable salt thereof is provided, wherein R^{A} is selected from the group consisting of a hydrogen atom, halogen, C₁₋₆ alkyl, and cyano; preferably, R^{A} is cyano.

In some embodiments of the present disclosure, the compound of general formula (I) or the pharmaceutically acceptable salt thereof is provided, wherein G is N or C-CN; preferably, G is N.

In some embodiments of the present disclosure, the compound of general formula (I) or the pharmaceutically acceptable salt thereof is provided, wherein R¹ is a hydrogen atom. In some embodiments of the present disclosure, the compound of general formula (I) or the pharmaceutically acceptable salt thereof is provided, wherein R^{c} and R^{d} are both hydrogen atoms.

In some embodiments of the present disclosure, the compound of general formula (I) or the pharmaceutically acceptable salt thereof is provided, wherein u is 1 or 2; preferably, u is 1.

In some embodiments of the present disclosure, the compound of general formula (I) or the pharmaceutically acceptable salt thereof is provided, wherein L² is O or (CR^{c}R^{d})ᵤO, and R^{c}, R^{d}, and u are as defined in general formula (I); preferably, L² is O or CH₂O; more preferably, L² is O.

In some embodiments of the present disclosure, the compound of general formula (I) or the pharmaceutically acceptable salt thereof is a compound of general formula (II) or a pharmaceutically acceptable salt thereof: 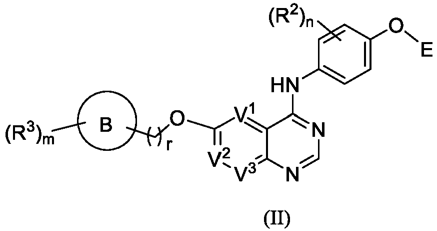 wherein:
r is 0 or 1;
ring B, E, R², R³, V¹, V², V³, m, and n are as defined in general formula (I).

In some embodiments of the present disclosure, the compound of general formula (II) or the pharmaceutically acceptable salt thereof is provided, wherein V¹, V², and V³ are identical or different and are each independently C(R^{a}) or N; R^{a} is as defined in general formula (I).

In some embodiments of the present disclosure, the compound of general formula (I) or general formula (II) or the pharmaceutically acceptable salt thereof is provided, wherein ring B is 8-membered fused heterocyclyl or 7- to 8-membered bridged heterocyclyl; preferably, ring B is 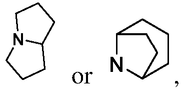 and ring B may be substituted with R³ at any substitutable position.

In some embodiments of the present disclosure, the compound of general formula (I) or general formula (II) or the pharmaceutically acceptable salt thereof is provided, wherein 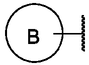 is selected from the group consisting of 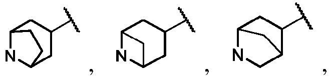 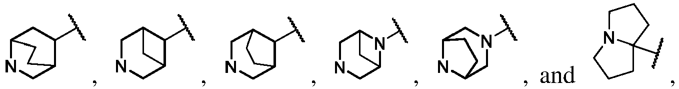 and ring B may be substituted with R³ at any substitutable position; preferably, 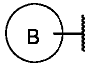 is selected from the group consisting of 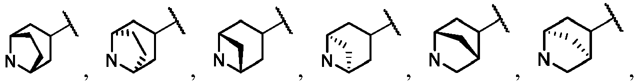 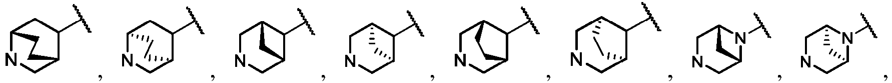 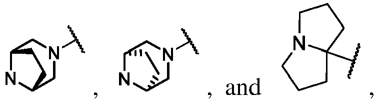 and ring B may be substituted with R³ at any substitutable position; more preferably, 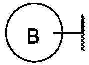 is

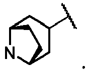

In some embodiments of the present disclosure, the compound of general formula (I) or general formula (II) or the pharmaceutically acceptable salt thereof is provided, wherein 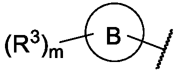 is selected from the group consisting of 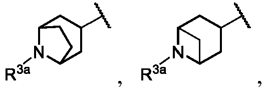 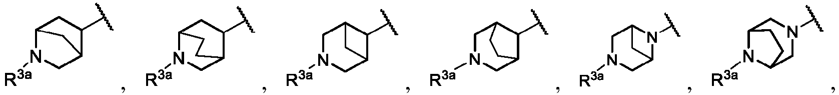 and 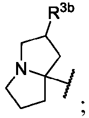 R^{3a} is selected from the group consisting of a hydrogen atom, alkyl, alkenyl, alkynyl, -C(O)R¹⁰, -C(O)OR¹⁰, -C(O)NR¹¹R¹², -S(O)ₚR¹⁰, -S(O)ₚNR¹¹R¹², cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl; R^{3b} is selected from the group consisting of a hydrogen atom, oxo, halogen, alkyl, alkenyl, alkynyl, cyano, nitro, -OR¹⁰, -NR¹¹R¹², -C(O)R¹⁰, - C(O)OR¹⁰, -OC(O)R¹⁰, -C(O)NR¹¹R¹², -NR¹³C(O)R¹⁰, -NR¹³C(O)OR¹⁰, - NR¹³C(O)NR¹¹R¹², -S(O)ₚR¹⁰, -S(O)ₚNR¹¹R¹², -NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl; R¹⁰, R¹¹, R¹², R^{10a}, R^{11a}, R^{12a}, and p are as defined in general formula (I); preferably, 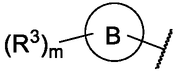 is selected from the group consisting of 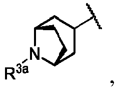 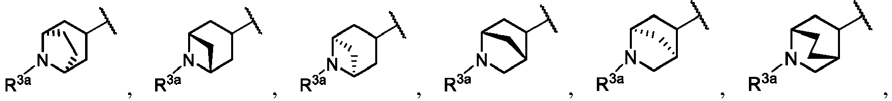 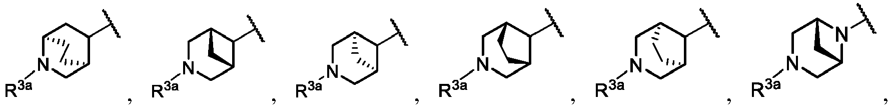 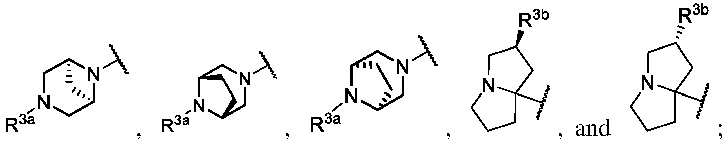 R^{3a} is selected from the group consisting of a hydrogen atom, alkyl, alkenyl, alkynyl, -C(O)R¹⁰, - C(O)OR¹⁰, -C(O)NR¹¹R¹², -S(O)ₚR¹⁰, -S(O)ₚNR¹¹R¹², cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl; R^{3b} is selected from the group consisting of halogen, alkyl, alkenyl, alkynyl, cyano, nitro, -OR¹⁰, -NR¹¹R¹², -C(O)R¹⁰, -C(O)OR¹⁰, -OC(O)R¹⁰, -C(O)NR¹¹R¹², - NR¹³C(O)R¹⁰, -NR¹³C(O)OR¹⁰, -NR¹³C(O)NR¹¹R¹², -S(O)ₚR¹⁰, -S(O)ₚNR¹¹R¹², - NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, - NR^{11a}R^{12a}, cycloalkyl, heterocyclyl, aryl, and heteroaryl; R¹⁰, R¹¹, R¹², R^{10a}, R^{11a}, R^{12a}, and p are as defined in general formula (I).

In some embodiments of the present disclosure, the compound of general formula (I) or general formula (II) or the pharmaceutically acceptable salt thereof is provided, wherein each R³ is identical or different and is independently selected from the group consisting of halogen, C₁₋₆ alkyl, -OR¹⁰, and -C(O)R¹⁰, wherein the C₁₋₆ alkyl is optionally substituted with one or more substituents selected from the group consisting of halogen, cyano, -OR^{10a}, -NR^{11a}R^{12a}, 3- to 8-membered cycloalkyl, 3- to 8-membered heterocyclyl, 6- to 10-membered aryl, and 5- to 10-membered heteroaryl; and R¹⁰, R^{10a}, R^{11a}, and R^{12a} are as defined in general formula (I); preferably, R³ is halogen or -C(O)R¹⁰; and R¹⁰ is as defined in general formula (I); more preferably, R³ is -C(O)R¹⁰; and R¹⁰ is as defined in general formula (I).

In some embodiments of the present disclosure, the compound of general formula (I) or general formula (II) or the pharmaceutically acceptable salt thereof is provided, wherein R^{3a} is C₁₋₆ alkyl or -C(O)R¹⁰, wherein the C₁₋₆ alkyl is optionally substituted with one or more substituents selected from the group consisting of halogen, cyano, -OR^{10a}, - NR^{11a}R^{12a}, 3- to 8-membered cycloalkyl, 3- to 8-membered heterocyclyl, 6- to 10-membered aryl, and 5- to 10-membered heteroaryl; and R¹⁰, R^{10a}, R^{11a}, and R^{12a} are as defined in general formula (I); preferably, R^{3a} is -C(O)R¹⁰, and R¹⁰ is as defined in general formula (I).

In some embodiments of the present disclosure, the compound of general formula (I) or general formula (II) or the pharmaceutically acceptable salt thereof is provided, wherein R^{3b} is selected from the group consisting of halogen, C₁₋₆ alkyl, -OR¹⁰, and -C(O)R¹⁰, wherein the C₁₋₆ alkyl is optionally substituted with one or more substituents selected from the group consisting of halogen, cyano, -OR^{10a}, -NR^{11a}R^{12a}, 3- to 8-membered cycloalkyl, 3- to 8-membered heterocyclyl, 6- to 10-membered aryl, and 5- to 10-membered heteroaryl; and R¹⁰, R^{10a}, R^{11a}, and R^{12a} are as defined in general formula (I); preferably, R^{3b} is halogen; more preferably, R^{3b} is fluorine.

In some embodiments of the present disclosure, the compound of general formula (I) or the pharmaceutically acceptable salt thereof is provided, wherein 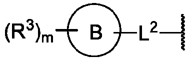 is 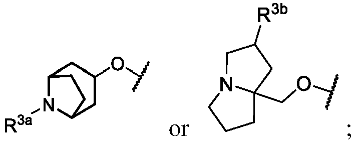 R^{3a} is -C(O)R¹⁰, and R¹⁰ is as defined in general formula (I); R^{3b} is halogen; preferably, 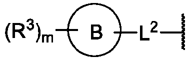 is selected from the group consisting of 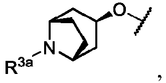 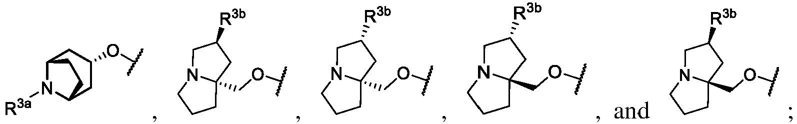 R^{3a} is - C(O)R¹⁰, and R¹⁰ is as defined in general formula (I); R^{3b} is halogen; more preferably, 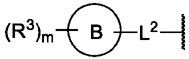 is selected from the group consisting of 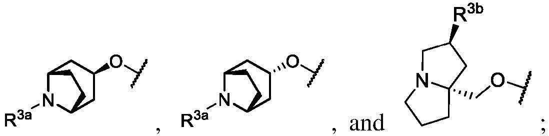 R^{3a} is -C(O)R¹⁰, and R¹⁰ is as defined in general formula (I); R^{3b} is halogen; further preferably, 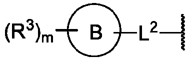 is 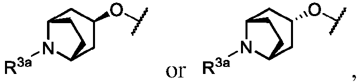 and R^{3a} is -C(O)R¹⁰; R¹⁰ is C₂₋₆ alkenyl (preferably ethenyl); most preferably, 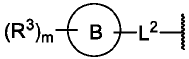 is 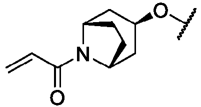 or

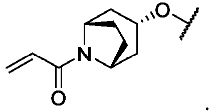

In some embodiments of the present disclosure, the compound of general formula (II) or the pharmaceutically acceptable salt thereof is provided, wherein r is 0.

In some embodiments of the present disclosure, the compound of general formula (II) or the pharmaceutically acceptable salt thereof is provided, wherein 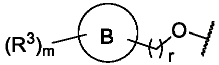 is 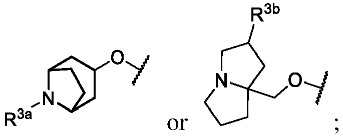 R^{3a} is -C(O)R¹⁰, and R¹⁰ is as defined in general formula (I); R^{3b} is halogen; preferably, 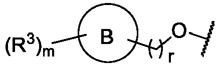 is selected from the group consisting of 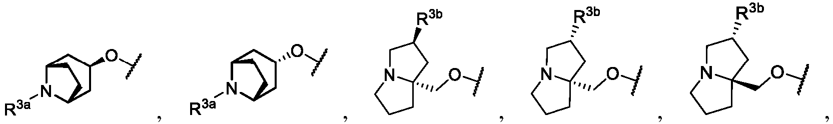 and 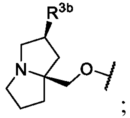 R^{3a} is -C(O)R¹⁰, and R¹⁰ is as defined in general formula (I); R^{3b} is halogen; more preferably, 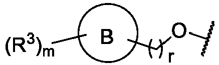 is selected from the group consisting of 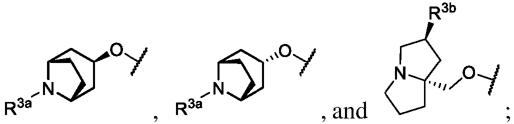 R^{3a} is -C(O)R¹⁰, and R¹⁰ is as defined in general formula (I); R^{3b} is halogen; further preferably, 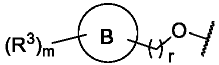 is 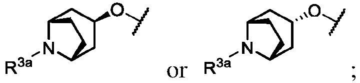 R^{3a} is -C(O)R¹⁰; and R¹⁰ is C₂₋₆ alkenyl (preferably ethenyl); most preferably, 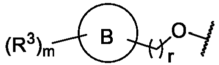 is 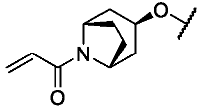 or

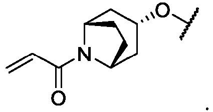

In some embodiments of the present disclosure, the compound of general formula (I) or general formula (II) or the pharmaceutically acceptable salt thereof is provided, wherein R¹⁰ are identical or different at each occurrence and are each independently selected from the group consisting of a hydrogen atom, C₁₋₆ alkyl, C₂₋₆ alkenyl, C₂₋₆ alkynyl, 3- to 8-membered cycloalkyl, and 3- to 8-membered heterocyclyl, wherein the C₁₋₆ alkyl, C₂₋₆ alkenyl, C₂₋₆ alkynyl, 3- to 8-membered cycloalkyl, and 3- to 8-membered heterocyclyl are each independently optionally substituted with one or more R^{B}; R^{B} is selected from the group consisting of oxo, halogen, C₁₋₆ alkyl, -OR^{10b}, -NR^{11b}R^{12b}, -C(O)R^{10b}, - C(O)NR^{11b}R^{12b}, 3- to 8-membered cycloalkyl, and 3- to 8-membered heterocyclyl, wherein the C₁₋₆ alkyl, 3- to 8-membered cycloalkyl, and 3- to 8-membered heterocyclyl are each independently optionally substituted with one or more substituents selected from the group consisting of oxo, halogen, C₁₋₆ alkyl, C₁₋₆ haloalkyl, C₁₋₆ hydroxyalkyl, C₁₋₆ alkoxy, hydroxy, cyano, and amino; R^{10b}, R^{11b}, and R^{12b} are as defined in general formula (I);
preferably, R¹⁰ is C₂₋₆ alkenyl, wherein the C₂₋₆ alkenyl is optionally substituted with one or more R^{B}; R^{B} is selected from the group consisting of halogen, C₁₋₆ alkyl, 3- to 8-membered cycloalkyl, and 3- to 8-membered heterocyclyl, wherein the C₁₋₆ alkyl, 3- to 8-membered cycloalkyl, and 3- to 8-membered heterocyclyl are each independently optionally substituted with one or more substituents selected from the group consisting of oxo, halogen, C₁₋₆ alkyl, C₁₋₆ haloalkyl, C₁₋₆ alkoxy, hydroxy, cyano, and amino;
more preferably, R¹⁰ is C₂₋₆ alkenyl, wherein the C₂₋₆ alkenyl is optionally substituted with one or more substituents selected from the group consisting of halogen and C₁₋₆ alkyl;
further preferably, R¹⁰ is C₂₋₆ alkenyl;
most preferably, R¹⁰ is ethenyl.

In some embodiments of the present disclosure, the compound of general formula (I) or general formula (II) or the pharmaceutically acceptable salt thereof is provided, wherein R^{10a} and R^{10b} are identical or different at each occurrence and are each independently a hydrogen atom or C₁₋₆ alkyl.

In some embodiments of the present disclosure, the compound of general formula (I) or general formula (II) or the pharmaceutically acceptable salt thereof is provided, wherein R^{11a}, R^{12a}, R^{11b}, and R^{12b} are identical or different at each occurrence and are each independently a hydrogen atom or C₁₋₆ alkyl.

In some embodiments of the present disclosure, the compound of general formula (I) or general formula (II) or the pharmaceutically acceptable salt thereof is provided, wherein m is 0, 1, or 2; preferably, m is 1.

In some embodiments of the present disclosure, the compound of general formula (I) or general formula (II) or the pharmaceutically acceptable salt thereof is provided, wherein R³ is 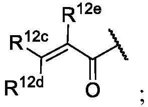 R^{12c}, R^{12d}, and R^{12e} are identical or different and are each independently selected from the group consisting of a hydrogen atom, halogen, C₁₋₆ alkyl, 3- to 8-membered cycloalkyl, and 3- to 8-membered heterocyclyl, wherein the C₁₋₆ alkyl, 3- to 8-membered cycloalkyl, and 3- to 8-membered heterocyclyl are each independently optionally substituted with one or more substituents selected from the group consisting of oxo, halogen, C₁₋₆ alkyl, C₁₋₆ haloalkyl, C₁₋₆ alkoxy, hydroxy, cyano, and amino.

In some embodiments of the present disclosure, the compound of general formula (I) or the pharmaceutically acceptable salt thereof is a compound of general formula (III) or a pharmaceutically acceptable salt thereof: 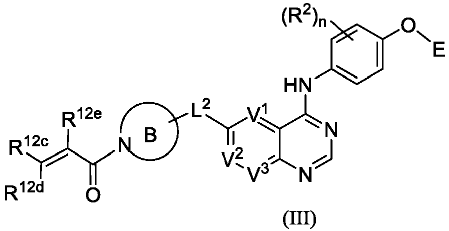 wherein:
ring B is 7- to 10-membered nitrogen-containing fused heterocyclyl or 7- to 10-membered nitrogen-containing bridged heterocyclyl;
R^{12c}, R^{12d}, and R^{12e} are identical or different and are each independently selected from the group consisting of a hydrogen atom, halogen, C₁₋₆ alkyl, 3- to 8-membered cycloalkyl, and 3- to 8-membered heterocyclyl, wherein the C₁₋₆ alkyl, 3- to 8-membered cycloalkyl, and 3- to 8-membered heterocyclyl are each independently optionally substituted with one or more substituents selected from the group consisting of oxo, halogen, C₁₋₆ alkyl, C₁₋₆ haloalkyl, C₁₋₆ alkoxy, hydroxy, cyano, and amino; and
E, R², L², V¹, V², V³, and n are as defined in general formula (I). In some embodiments of the present disclosure, the compound of general formula (III) or the pharmaceutically acceptable salt thereof is provided, wherein V¹, V², and V³ are identical or different and are each independently C(R^{a}) or N; R^{a} is as defined in general formula (I).

In some embodiments of the present disclosure, the compound of general formula (III) or the pharmaceutically acceptable salt thereof is provided, wherein L² is O.

In some embodiments of the present disclosure, the compound of general formula (III) or the pharmaceutically acceptable salt thereof is provided, wherein ring B is 8-membered nitrogen-containing fused heterocyclyl or 7- to 8-membered nitrogen-containing bridged heterocyclyl; preferably, ring B is 7- to 8-membered nitrogen-containing bridged heterocyclyl; more preferably, ring B is 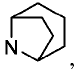 and ring B may be substituted with R³ at any substitutable position.

In some embodiments of the present disclosure, the compound of general formula (III) or the pharmaceutically acceptable salt thereof is provided, wherein 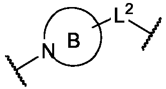 is 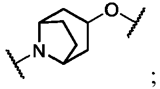 preferably

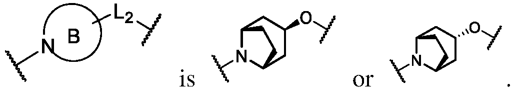

In some embodiments of the present disclosure, the compound of general formula (I), general formula (II), or general formula (III) or the pharmaceutically acceptable salt thereof is provided, wherein E is selected from the group consisting of 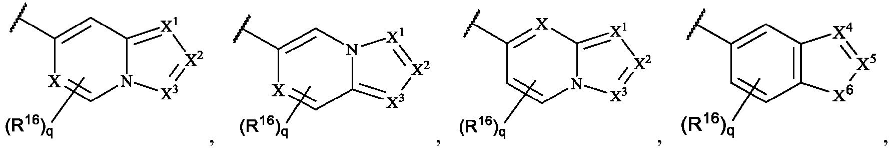 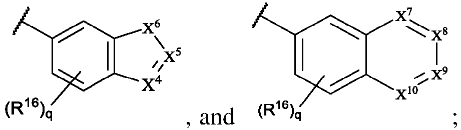 X is N or CR^{16a}; X¹, X², and X³ are each independently N or CR^{16b}; X⁴ and X⁵ are identical or different and are each independently N or CR^{16c}; X⁶ is selected from the group consisting of O, S, and NR^{16d} X⁷, X⁸, X⁹, and X¹⁰ are identical or different and are each independently N or CR^{16e}, and at least one of X⁷, X⁸, X⁹, and X¹⁰ is N; R^{16a}, R^{16b}, R^{16c}, and R^{16e} are identical or different and are each independently selected from the group consisting of selected from the group consisting of a hydrogen atom, halogen, alkyl, alkenyl, alkynyl, cyano, nitro, -OR⁴, -NR⁵R⁶, - C(O)R⁴, -C(O)OR⁴, -OC(O)R⁴, -C(O)NR⁵R⁶, -S(O)ₚR⁴, -S(O)ₚNR⁵R⁶, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, aminoalkyl, cyano, -OR^{4a}, -NR^{5a}R^{6a}, cycloalkyl, heterocyclyl, aryl, and heteroaryl; R^{16d} is selected from the group consisting of a hydrogen atom, alkyl, and cycloalkyl, wherein the alkyl and cycloalkyl are each independently optionally substituted with one or more substituents selected from the group consisting of oxo, halogen, alkyl, cyano, -OR^{4a}, and -NR^{5a}R^{6a}; q is 0, 1, 2, or 3; and R⁴, R^{4a}, R⁵, R⁶, R^{5a}, R^{6a}, R¹⁶, and p are as defined in general formula (I).

In some embodiments of the present disclosure, the compound of general formula (I), general formula (II), or general formula (III) or the pharmaceutically acceptable salt thereof is provided, wherein R¹⁶ is selected from the group consisting of halogen, C₁₋₆ alkyl, cyano, -OR⁴, and 3- to 8-membered cycloalkyl, wherein the C₁₋₆ alkyl is optionally substituted with one or more halogens, and R⁴ is as defined in general formula (I); preferably, R¹⁶ is halogen or C₁₋₆ alkyl; more preferably, R¹⁶ is C₁₋₆ alkyl; most preferably, R¹⁶ is methyl.

In some embodiments of the present disclosure, the compound of general formula (I), general formula (II), or general formula (III) or the pharmaceutically acceptable salt thereof is provided, wherein R⁴ is a hydrogen atom or C₁₋₆ alkyl.

In some embodiments of the present disclosure, the compound of general formula (I), general formula (II), or general formula (III) or the pharmaceutically acceptable salt thereof is provided, wherein R^{4a} is a hydrogen atom or C₁₋₆ alkyl.

In some embodiments of the present disclosure, the compound of general formula (I), general formula (II), or general formula (III) or the pharmaceutically acceptable salt thereof is provided, wherein R⁵ and R⁶ are identical or different at each occurrence and are each independently a hydrogen atom or C₁₋₆ alkyl.

In some embodiments of the present disclosure, the compound of general formula (I), general formula (II), or general formula (III) or the pharmaceutically acceptable salt thereof is provided, wherein R^{5a} and R^{6a} are identical or different at each occurrence and are each independently a hydrogen atom or C₁₋₆ alkyl.

In some embodiments of the present disclosure, the compound of general formula (I), general formula (II), or general formula (III) or the pharmaceutically acceptable salt thereof is provided, wherein R^{16a}, R^{16b}, R^{16c}, and R^{16e} are identical or different and are each independently selected from the group consisting of a hydrogen atom, halogen, and C₁₋₆ alkyl; preferably, R^{16a}, R^{16b}, R^{16c}, and R^{16e} are each independently a hydrogen atom.

In some embodiments of the present disclosure, the compound of general formula (I), general formula (II), or general formula (III) or the pharmaceutically acceptable salt thereof is provided, wherein R^{16d} is selected from the group consisting of a hydrogen atom, C₁₋₆ alkyl, and 3- to 8-membered cycloalkyl; preferably, R^{16d} is C₁₋₆ alkyl; more preferably, R^{16d} is methyl.

In some embodiments of the present disclosure, the compound of general formula (I), general formula (II), or general formula (III) or the pharmaceutically acceptable salt thereof is provided, wherein E is selected from the group consisting of 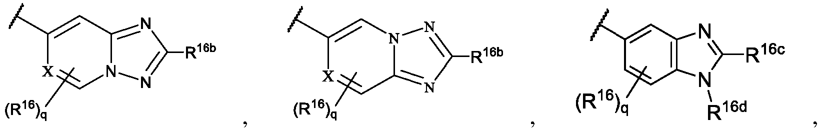 and 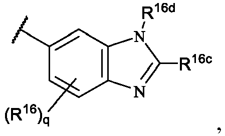 preferably 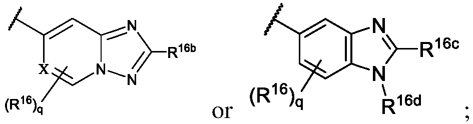 X is N or CR^{16a}; R^{16a}, R^{16b}, and R^{16c} are identical or different and are each independently selected from the group consisting of a hydrogen atom, halogen, and C₁₋₆ alkyl; R^{16d} is selected from the group consisting of a hydrogen atom, C₁₋₆ alkyl, and 3- to 8-membered cycloalkyl; R¹⁶ is halogen or C₁₋₆ alkyl; q is 0, 1, 2, or 3.

In some embodiments of the present disclosure, the compound of general formula (I), general formula (II), or general formula (III) or the pharmaceutically acceptable salt thereof is provided, wherein q is 0 or 1; preferably, q is 0.

In some embodiments of the present disclosure, the compound of general formula (I), general formula (II), or general formula (III) or the pharmaceutically acceptable salt thereof is provided, wherein E is selected from the group consisting of

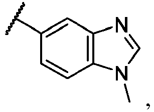

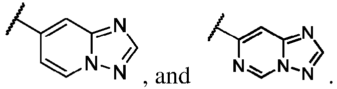

In some embodiments of the present disclosure, the compound of general formula (I), general formula (II), or general formula (III) or the pharmaceutically acceptable salt thereof is provided, wherein each R² is identical or different and is independently C₁₋₆ alkyl or halogen; preferably, R² is C₁₋₆ alkyl; more preferably, R² is methyl.

In some embodiments of the present disclosure, the compound of general formula (I), general formula (II), or general formula (III) or the pharmaceutically acceptable salt thereof is provided, wherein each R² is identical or different and is independently methyl or fluorine.

In some embodiments of the present disclosure, the compound of general formula (I), general formula (II), or general formula (III) or the pharmaceutically acceptable salt thereof is provided, wherein n is 0, 1, or 2; preferably, n is 1 or 2; more preferably, n is 1. In some embodiments of the present disclosure, the compound of general formula (II) or general formula (III) or the pharmaceutically acceptable salt thereof is provided, wherein 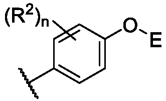 is 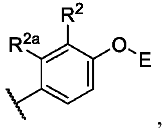 and R^{2a} is a hydrogen atom or halogen; E and R² are as defined in general formula (I); preferably, 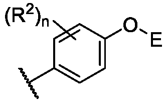 is 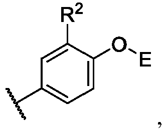 and E and R² are as defined in general formula (I); more preferably, 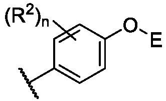 is 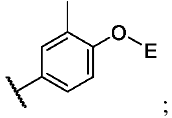 E is as defined in general formula (I).

In some embodiments of the present disclosure, the compound of general formula (II) or general formula (III) or the pharmaceutically acceptable salt thereof is provided, wherein 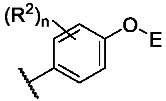 is 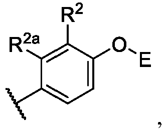 and R^{2a} is a hydrogen atom or halogen; R² is C₁₋₆ alkyl; E is as defined in general formula (I); preferably 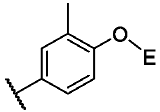 or 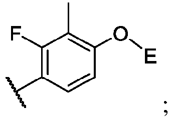 E is as defined in general formula (I); more preferably 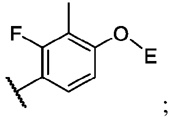 E is as defined in general formula (I).

In some embodiments of the present disclosure, the compound of general formula (II) or general formula (III) or the pharmaceutically acceptable salt thereof is provided, wherein 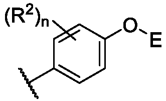 is selected from the group consisting of 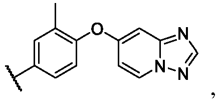 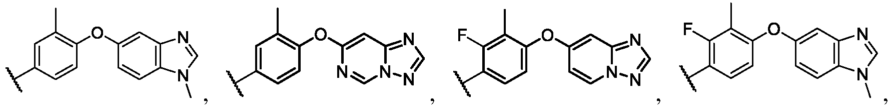 and 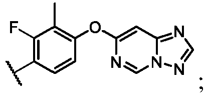 preferably

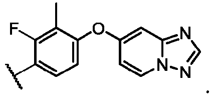

In some embodiments of the present disclosure, the compound of general formula (I), general formula (II), or general formula (III) or the pharmaceutically acceptable salt thereof is provided, wherein R^{e} is a hydrogen atom or C₁₋₆ alkyl.

In some embodiments of the present disclosure, the compound of general formula (I), general formula (II), or general formula (III) or the pharmaceutically acceptable salt thereof is provided, wherein R^{a} is selected from the group consisting of a hydrogen atom, halogen, C₁₋₆ alkyl, cyano, and -OR^{e}, and R^{e} is as defined in general formula (I); preferably, R^{a} is a hydrogen atom or C₁₋₆ alkoxy; further preferably, R^{a} is selected from the group consisting of a hydrogen atom, methoxy, and ethoxy; more preferably, R^{a} is a hydrogen atom or methoxy.

In some embodiments of the present disclosure, the compound of general formula (I), general formula (II), or general formula (III) or the pharmaceutically acceptable salt thereof is provided, wherein V¹ is C(R^{a}) or N; V² and V³ are identical or different and are each independently C(R^{a}) or N; or V² is C(R^{bb}), V³ is C(R^{cc}), and R^{bb} and R^{cc}, together with the carbon atom to which they are each attached, form 5- or 6-membered cycloalkyl or 5- or 6-membered heterocyclyl; R^{a} is a hydrogen atom or C₁₋₆ alkoxy; preferably, V¹, V², and V³ are identical or different and are each independently C(R^{a}) or N; R^{a} is a hydrogen atom or C₁₋₆ alkoxy; more preferably, V¹ is N or CH; V² is C(R^{a}) or N, and R^{a} is a hydrogen atom or C₁₋₆ alkoxy; V³ is CH.

In some embodiments of the present disclosure, the compound of general formula (I), general formula (II), or general formula (III) or the pharmaceutically acceptable salt thereof is provided, wherein V¹, V², and V³ are each independently C(R^{a}), and R^{a} is as defined in general formula (I); or V¹ and V² are both N, V³ is C(R^{a}), and R^{a} is as defined in general formula (I);
preferably, V¹, V², and V³ are each independently C(R^{a}), and R^{a} is a hydrogen atom or C₁₋₆ alkoxy; or V¹ and V² are both N, V³ is C(R^{a}), and R^{a} is a hydrogen atom or C₁₋₆ alkoxy;
more preferably, V¹, V², and V³ are all CH; or V¹ and V³ are both CH, and V² is C-C₁₋₆ alkoxy; or V¹ and V² are both N, and V³ is CH;
most preferably, V¹, V², and V³ are all CH.

In some embodiments of the present disclosure, the compound of general formula (I), general formula (II), or general formula (III) or the pharmaceutically acceptable salt thereof is provided, wherein V¹ is C(R^{a}); V² and V³ are identical or different and are each independently C(R^{a}) or N; R^{a} is as defined in general formula (I); preferably, V¹, V², and V³ are identical or different and are each independently C(R^{a}); R^{a} is as defined in general formula (I); more preferably, V¹, V², and V³ are all CH.

In some embodiments of the present disclosure, the compound of general formula (III) or the pharmaceutically acceptable salt thereof is provided, wherein R^{12c}, R^{12d}, and R^{12e} are identical or different and are each independently selected from the group consisting of a hydrogen atom, halogen, and C₁₋₆ alkyl; preferably, R^{12c}, R^{12d}, and R^{12e} are all hydrogen atoms.

In some embodiments of the present disclosure, the compound of general formula (II) or the pharmaceutically acceptable salt thereof is provided, wherein B is 8-membered fused heterocyclyl or 7- to 8-membered bridged heterocyclyl; R² is C₁₋₆ alkyl or halogen; n is 1 or 2; R³ is halogen or -C(O)R¹⁰; R¹⁰ is C₂₋₆ alkenyl, wherein the C₂₋₆ alkenyl is optionally substituted with one or more substituents selected from the group consisting of halogen and C₁₋₆ alkyl; m is 1; E is selected from the group consisting of 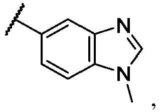 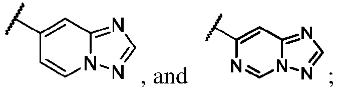 r is 0 or 1; V¹ is C(R^{a}) or N; V² and V³ are identical or different and are each independently C(R^{a}) or N; or V² is C(R^{bb}), V³ is C(R^{cc}), and R^{bb} and R^{cc}, together with the carbon atom to which they are each attached, form 5- or 6-membered cycloalkyl or 5- or 6-membered heterocyclyl; R^{a} is a hydrogen atom or C₁₋₆ alkoxy.

In some embodiments of the present disclosure, the compound of general formula (II) or the pharmaceutically acceptable salt thereof is provided, wherein B is 8-membered fused heterocyclyl or 7- to 8-membered bridged heterocyclyl; R² is C₁₋₆ alkyl or halogen; n is 1; R³ is halogen or -C(O)R¹⁰; R¹⁰ is C₂₋₆ alkenyl, wherein the C₂₋₆ alkenyl is optionally substituted with one or more substituents selected from the group consisting of halogen and C₁₋₆ alkyl; m is 1; E is selected from the group consisting of 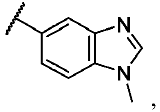 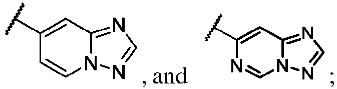 r is 0 or 1; V¹, V², and V³ are each independently C(R^{a}), and R^{a} is a hydrogen atom or C₁₋₆ alkoxy; or V¹ and V² are both N, V³ is C(R^{a}), and R^{a} is a hydrogen atom or C₁₋₆ alkoxy.

In some embodiments of the present disclosure, the compound of general formula (II) or the pharmaceutically acceptable salt thereof is provided, wherein B is 7- to 8-membered bridged heterocyclyl; R² is C₁₋₆ alkyl or halogen; n is 1 or 2; R³ is -C(O)R¹⁰; R¹⁰ is C₂₋₆ alkenyl; m is 1; E is selected from the group consisting of 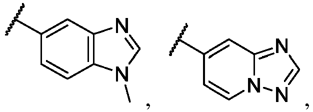 and 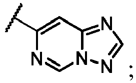 r is 0; V¹, V², and V³ are all CH.

In some embodiments of the present disclosure, the compound of general formula (III) or the pharmaceutically acceptable salt thereof is provided, 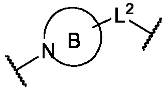 is 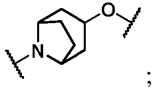 R² is C₁₋₆ alkyl or halogen; n is 1 or 2; R^{12c}, R^{12d}, and R^{12e} are all hydrogen atoms; E is selected from the group consisting of 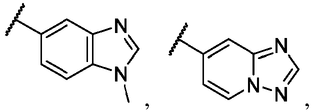 and 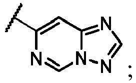 V¹, V², and V³ are identical or different and are each independently C(R^{a}) or N; R^{a} is a hydrogen atom or C₁₋₆ alkoxy.

In some embodiments of the present disclosure, the compound of general formula (III) or the pharmaceutically acceptable salt thereof is provided, wherein 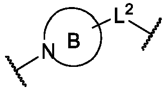 is 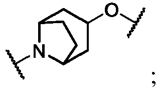 R² is C₁₋₆ alkyl or halogen; n is 1; R^{12c}, R^{12d}, and R^{12e} are identical or different and are each independently selected from the group consisting of a hydrogen atom, halogen, and C₁₋₆ alkyl; E is selected from the group consisting of 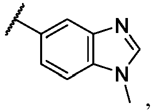 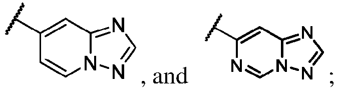 V¹, V², and V³ are each independently C(R^{a}), and R^{a} is a hydrogen atom or C₁₋₆ alkoxy; or V¹ and V² are both N, V³ is C(R^{a}), and R^{a} is a hydrogen atom or C₁₋₆ alkoxy.

In some embodiments of the present disclosure, the compound of general formula (III) or the pharmaceutically acceptable salt thereof is provided, wherein 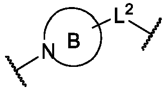 is 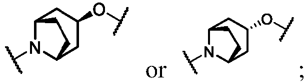 R² is C₁₋₆ alkyl or halogen; n is 1 or 2; R^{12c}, R^{12d}, and R^{12e} are all hydrogen atoms; E is selected from the group consisting of 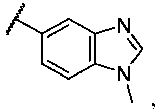 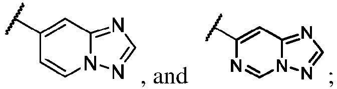 V¹, V², and V³ are all CH.

**Table A. Typical compounds of the present disclosure include, but are not limited to:**

| Example No. | Compound structure | Name |
|---|---|---|
| **1** | 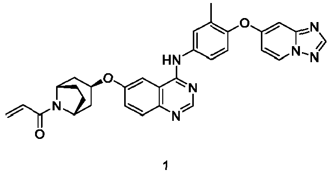 | 1-(*endo*-3-((4-((4-([1,2,4]Triazolo[1,5-*a*]pyridin-7-yloxy)-3-methylphenyl)amino)quinazolin-6-yl)oxy)-8-azabicyclo[3.2.1]octan-8-yl)prop-2-en-1-one **1** |
| | 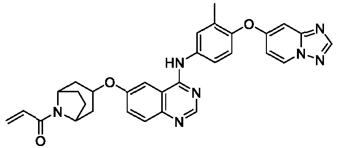 | 1-(3-((4-((4-([1,2,4]Triazolo[1,5-*a*]pyridin-7-yloxy)-3-methylphenyl)amino)quinazolin-6-yl)oxy)-8-azabicyclo[3.2.1]octan-8-yl)prop-2-en-1-one |
| **2** | 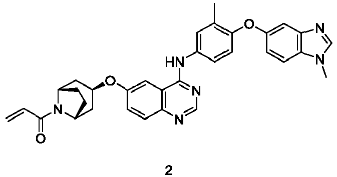 | 1-(*endo*-3-((4-((3-Methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one **2** |
| | 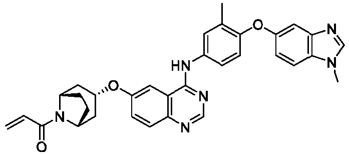 | 1-(*exo*-3-((4-((3-Methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one |
| | 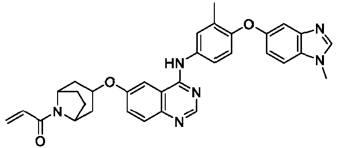 | 1-(3-((4-((3-Methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one |
| **3** | 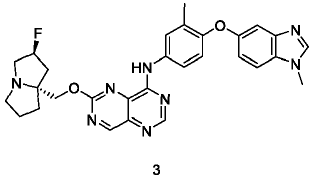 | 6-(((2*S*,7*aR*)-2-Fluorotetrahydro-1*H-*pyrrolizin-7*a*(5*H*)-yl)methoxy)-*N*-(3-methyl-4-((1-methyl-1*H-*benzo[*d*]imidazol-5-yl)oxy)phenyl)pyrimido[5,4-*d*]pyrimidin-4-amine **3** |
| | 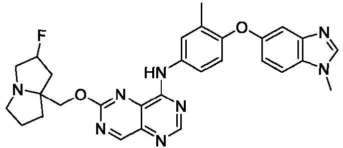 | 6-((2-Fluorotetrahydro-1*H-*pyrrolizin-7*a*(5*H*)-yl)methoxy)-*N*-(3-methyl-4-((1-methyl-1*H-*benzo[*d*]imidazol-5-yl)oxy)phenyl)pyrimido[5,4-*d*]pyrimidin-4-amine |
| | 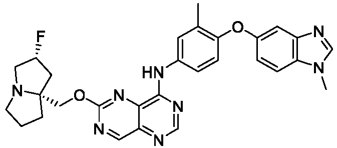 | 6-(((2*R*,7*aR*)-2-Fluorotetrahydro-1*H-*pyrrolizin-7*a*(5*H*)-yl)methoxy)-*N*-(3-methyl-4-((1-methyl-1*H-*benzo[*d*]imidazol-5-yl)oxy)phenyl)pyrimido[5,4-*d*]pyrimidin-4-amine |
| | 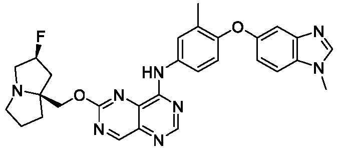 | 6-(((2*S*,7*aS*)-2-Fluorotetrahydro-1*H-*pyrrolizin-7*a*(5*H*)-yl)methoxy)-*N*-(3-methyl-4-((1-methyl-1*H-*benzo[*d*]imidazol-5-yl)oxy)phenyl)pyrimido[5,4-*d*]pyrimidin-4-amine |
| | 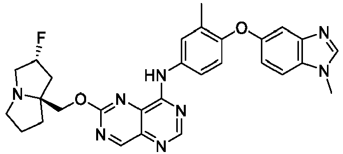 | 6-(((2*R*,7*aS*)-2-Fluorotetrahydro-1*H-*pyrrolizin-7*a*(5*H*)-yl)methoxy)-*N*-(3-methyl-4-((1-methyl-1*H-*benzo[*d*]imidazol-5-yl)oxy)phenyl)pyrimido[5,4-*d*]pyrimidin-4-amine |
| **4** | 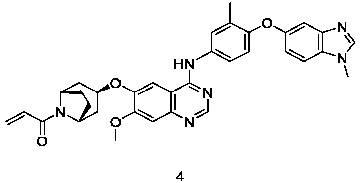 | 1-(*endo*-3-((7-Methoxy-4-((3-methyl-4-((1-methyl-1*H-*benzo[*d*]imidazol-5-yl)oxy)phenyl)amino)quinazolin-6-yl)oxy)-8-azabicyclo[3.2.1]octan-8-yl)prop-2-en-1-one **4** |
| | 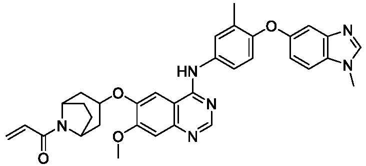 | 1-(3-((7-Methoxy-4-((3-methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one |
| **5** | 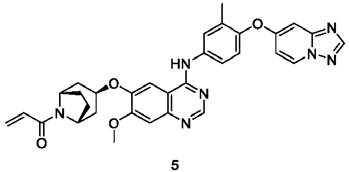 | 1-(*endo*-3-((4-((4-([1,2,4]Triazolo[1,5-*a*]pyridin-7-yloxy)-3-methylphenyl)amino)-7-methoxyquinazolin-6-yl)oxy)-8-azabicyclo[3.2.1]octan-8-yl)prop-2-en-1-one **5** |
| | 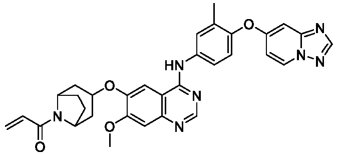 | 1-(3-((4-((4-([1,2,4]Triazolo[1,5-*a*]pyridin-7-yloxy)-3-methylphenyl)amino)-7-methoxyquinazolin-6-yl)oxy)-8-azabicyclo[3.2.1]octan-8-yl)prop-2-en-1-one |
| **6** | 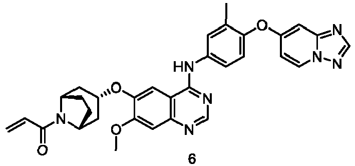 | 1-(*exo*-3-((4-((4-([1,2,4]Triazolo[1,5-*a*]pyridin-7-yloxy)-3-methylphenyl)amino)-7-methoxyquinazolin-6-yl)oxy)-8-azabicyclo[3.2.1]octan-8-yl)prop-2-en-1-one **6** |
| **7** | 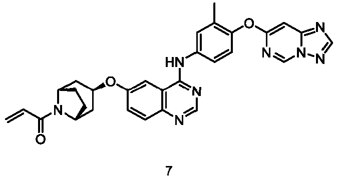 | 1-(*endo*-3-((4-((4-([1,2,4]Triazolo[1,5-*c*]pyrimidin-7-yloxy)-3-methylphenyl)amino)quinazolin-6-yl)oxy)-8-azabicyclo[3.2.1]octan-8-yl)prop-2-en-1-one **7** |
| **8** | 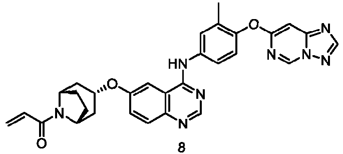 | 1-(*exo*-3-((4-((4-([1,2,4]Triazolo[1,5-*c*]pyrimidin-7-yloxy)-3-methylphenyl)amino)quinazolin-6-yl)oxy)-8-azabicyclo[3.2.1]octan-8-yl)prop-2-en-1-one **8** |
| | 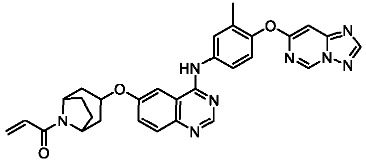 | 1-(3-((4-((4-([1,2,4]Triazolo[1,5-*c*]pyrimidin-7-yloxy)-3-methylphenyl)amino)quinazolin-6-yl)oxy)-8-azabicyclo[3.2.1]octan-8-yl)prop-2-en-1-one |
| **9** | 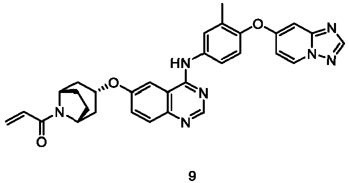 | 1-(*exo-*3-((4-((4-([1,2,4]Triazolo[1,5-*a*]pyridin-7-yloxy)-3-methylphenyl)amino)quinazolin-6-yl)oxy)-8-azabicyclo[3.2.1]octan-8-yl)prop-2-en-1-one **9** |
| **10** | 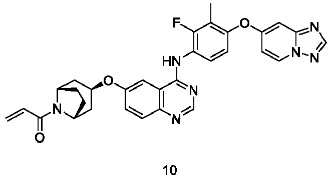 | 1-(*endo*-3-((4-((4-([1,2,4]Triazolo[1,5-*a*]pyridin-7-yloxy)-2-fluoro-3-methylphenyl)amino)quinazolin-6-yl)oxy)-8-azabicyclo[3.2.1]octan-8-yl)prop-2-en-1-one **10** |
| | 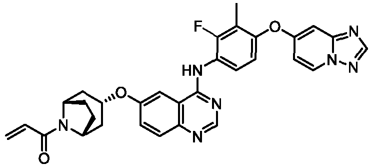 | 1-(*exo-*3-((4-((4-([1,2,4]Triazolo[1,5-*a*]pyridin-7-yloxy)-2-fluoro-3-methylphenyl)amino)quinazolin-6-yl)oxy)-8-azabicyclo[3.2.1]octan-8-yl)prop-2-en-1-one |
| | 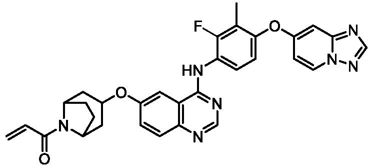 | 1-(3-((4-((4-([1,2,4]Triazolo[1,5-*a*]pyridin-7-yloxy)-2-fluoro-3-methylphenyl)amino)quinazolin-6-yl)oxy)-8-azabicyclo[3.2.1]octan-8-yl)prop-2-en-1-one |
| **11** | 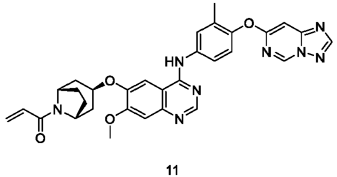 | 1-(*endo*-3-((4-((4-([1,2,4]Triazolo[1,5-*c*]pyrimidin-7-yloxy)-3-methylphenyl)amino)-7-methoxyquinazolin-6-yl)oxy)-8-azabicyclo[3.2.1]octan-8-yl)prop-2-en-1-one **11** |
| **12** | 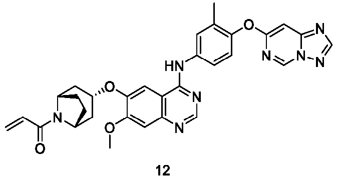 | 1-(*exo*-3-((4-((4-([1,2,4]Triazolo[1,5-*c*]pyrimidin-7-yloxy)-3-methylphenyl)amino)-7-methoxyquinazolin-6-yl)oxy)-8-azabicyclo[3.2.1]octan-8-yl)prop-2-en-1-one **12** |
| | 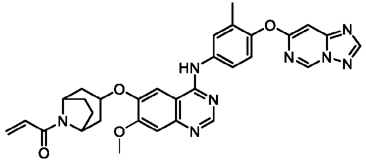 | 1-(3-((4-((4-([1,2,4]Triazolo[1,5-*c*]pyrimidin-7-yloxy)-3-methylphenyl)amino)-7-methoxyquinazolin-6-yl)oxy)-8-azabicyclo[3.2.1]octan-8-yl)prop-2-en-1-one |
| **13** | 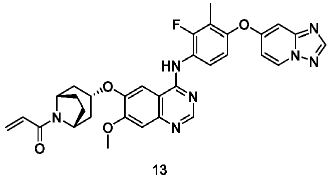 | 1-(*exo*-3-((4-((4-([1,2,4]Triazolo[1,5-*a*]pyridin-7-yloxy)-2-fluoro-3-methylphenyl)amino)-7-methoxyquinazolin-6-yl)oxy)-8-azabicyclo[3.2.1]octan-8-yl)prop-2-en-1-one **13** |
| | 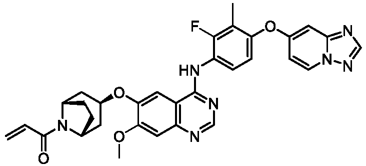 | 1-(*endo*-3-((4-((4-([1,2,4]Triazolo[1,5-*a*]pyridin-7-yloxy)-2-fluoro-3-methylphenyl)amino)-7-methoxyquinazolin-6-yl)oxy)-8-azabicyclo[3.2.1]octan-8-yl)prop-2-en-1-one |
| | 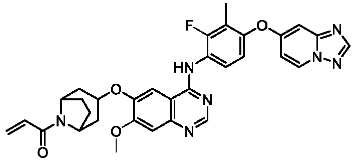 | 1-(3-((4-((4-([1,2,4]Triazolo[1,5-*a*]pyridin-7-yloxy)-2-fluoro-3-methylphenyl)amino)-7-methoxyquinazolin-6-yl)oxy)-8-azabicyclo[3.2.1]octan-8-yl)prop-2-en-1-one |
| **14** | 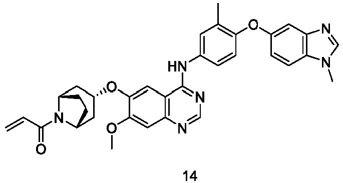 | 1-(*exo*-3-((7-Methoxy-4-((3-methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one **14** |
| **15** | 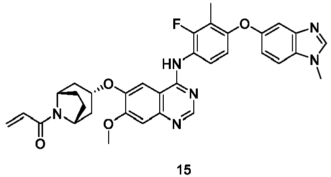 | 1-(*exo*-3-((4-((2-Fluoro-3-methyl-4-((1-methyl-1*H*-benzo[*d*]imidazol-5-yl)oxy)phenyl)amino)-7-methoxyquinazolin-6-yl)oxy)-8-azabicyclo[3.2.1]octan-8-yl)prop-2-en-1-one **15** |
| | 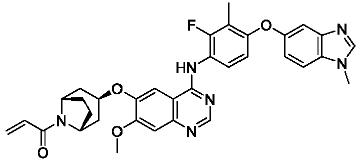 | 1-(*endo*-3-((4-((2-Fluoro-3-methyl-4-((1-methyl-1*H*-benzo[*d*]imidazol-5-yl)oxy)phenyl)amino)-7-methoxyquinazolin-6-yl)oxy)-8-azabicyclo[3.2.1]octan-8-yl)prop-2-en-1-one |
| | 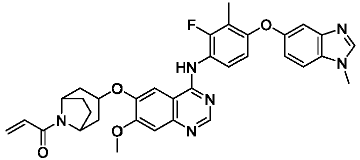 | 1-(3-((4-((2-Fluoro-3-methyl-4-((1-methyl-1*H*-benzo[*d*]imidazol-5-yl)oxy)phenyl)amino)-7-methoxyquinazolin-6-yl)oxy)-8-azabicyclo[3.2.1]octan-8-yl)prop-2-en-1-one |
| **16** | 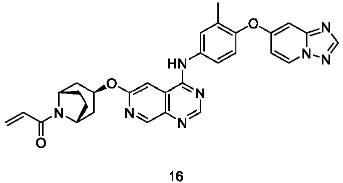 | 1-(*endo*-3-((4-((4-([1,2,4]Triazolo[1,5-*a*]pyridin-7-yloxy)-3-methylphenyl)amino)pyrido[3,4-*d*]pyrimidin-6-yl)oxy)-8-azabicyclo[3.2.1]octan-8-yl)prop-2-en-1-one **16** |
| | 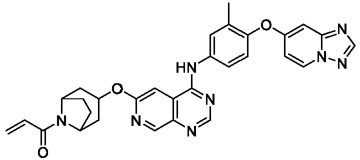 | 1-(3-((4-((4-([1,2,4]Triazolo[1,5-*a*]pyridin-7-yloxy)-3-methylphenyl)amino)pyrido[3,4-*d*]pyrimidin-6-yl)oxy)-8-azabicyclo[3.2.1]octan-8-yl)prop-2-en-1-one |
| **17** | 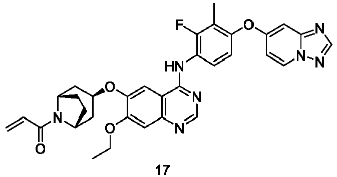 | 1-(*endo*-3-((4-((4-([1,2,4]Triazolo[1,5-*a*]pyridin-7-yloxy)-2-fluoro-3-methylphenyl)amino)-7-ethoxyquinazolin-6-yl)oxy)-8-azabicyclo[3.2.1]octan-8-yl)prop-2-en-1-one **17** |
| | 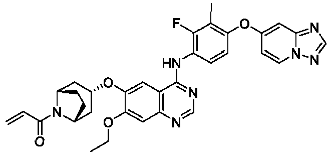 | 1-(*exo*-3-((4-((4-([1,2,4]Triazolo[1,5-*a*]pyridin-7-yloxy)-2-fluoro-3-methylphenyl)amino)-7-ethoxyquinazolin-6-yl)oxy)-8-azabicyclo[3.2.1]octan-8-yl)prop-2-en-1-one |
| | 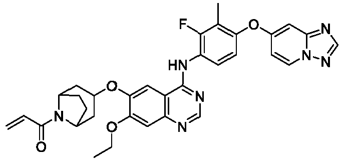 | 1-(3-((4-((4-([1,2,4]Triazolo[1,5-*a*]pyridin-7-yloxy)-2-fluoro-3-methylphenyl)amino)-7-ethoxyquinazolin-6-yl)oxy)-8-azabicyclo[3.2.1]octan-8-yl)prop-2-en-1-one |
| **18** | 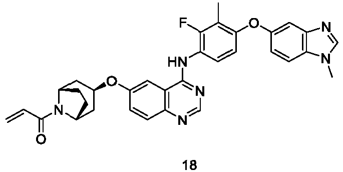 | 1-(*endo*-3-((4-((2-Fluoro-3-methyl-4-((1-methyl-1*H*-benzo[d]imidazol-5-yl)oxy)phenyl)amino)quinazolin-6-yl)oxy)-8-azabicyclo[3.2.1]octan-8-yl)prop-2-en-1-one **18** |
| **19** | 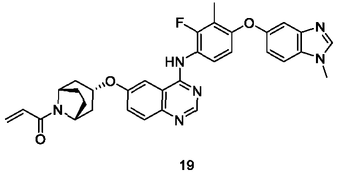 | 1-(*exo*-3-((4-((2-Fluoro-3-methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one **19** |
| | 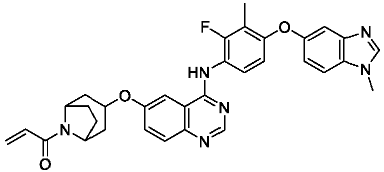 | 1-(3-((4-((2-Fluoro-3-methyl-4-((1-methyl-1*H*-benzo[*d*]imidazol-5-yl)oxy)phenyl)amino)quinazolin-6-yl)oxy)-8-azabicyclo[3.2.1]octan-8-yl)prop-2-en-1-one |
| **20** | 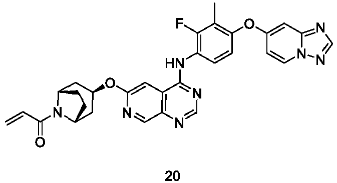 | 1-(*endo*-3-((4-((4-([1,2,4]Triazolo[1,5-*a*]pyridin-7-yloxy)-2-fluoro-3-methylphenyl)amino)pyrido[3,4-*d*]pyrimidin-6-yl)oxy)-8-azabicyclo[3.2.1]octan-8-yl)prop-2-en-1-one **20** |
| **21** | 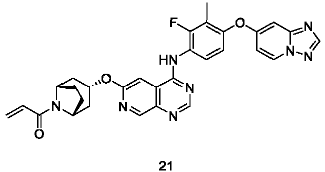 | 1-(*exo*-3-((4-((4-([1,2,4]Triazolo[1,5-*a*]pyridin-7-yloxy)-2-fluoro-3-methylphenyl)amino)pyrido[3,4-*d*]pyrimidin-6-yl)oxy)-8-azabicyclo[3.2.1]octan-8-yl)prop-2-en-1-one **21** |
| | 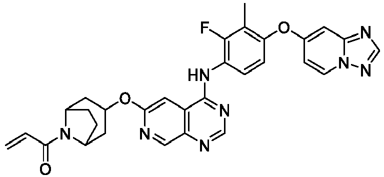 | 1-(3-((4-((4-([1,2,4]Triazolo[1,5-*a*]pyridin-7-yloxy)-2-fluoro-3-methylphenyl)amino)pyrido[3,4-*d*]pyrimidin-6-yl)oxy)-8-azabicyclo[3.2.1]octan-8-yl)prop-2-en-1-one |
| 22 | 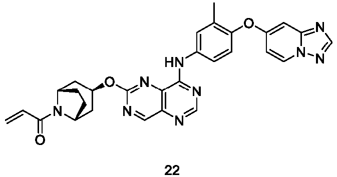 | 1-(*endo*-3-((8-((4-([1,2,4]Triazolo[1,5-*a*]pyridin-7-yloxy)-3-methylphenyl)amino)pyrimido[5,4-*d*]pyrimidin-2-yl)oxy)-8-azabicyclo[3.2.1]octan-8-yl)prop-2-en-1-one **22** |
| | 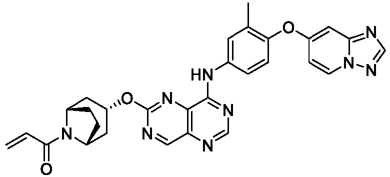 | 1-(*exo*-3-((8-((4-([1,2,4]Triazolo[1,5-*a*]pyridin-7-yloxy)-3-methylphenyl)amino)pyrimido[5,4-*d*]pyrimidin-2-yl)oxy)-8-azabicyclo[3.2.1]octan-8-yl)prop-2-en-1-one |
| | 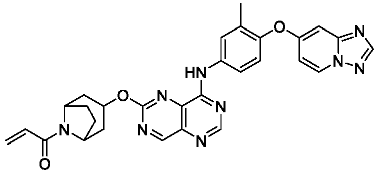 | 1-(3-((8-((4-([1,2,4]Triazolo[1,5-*a*]pyridin-7-yloxy)-3-methylphenyl)amino)pyrimido[5,4-*d*]pyrimidin-2-yl)oxy)-8-azabicyclo[3.2.1]octan-8-yl)prop-2-en-1-one |
| 23 | 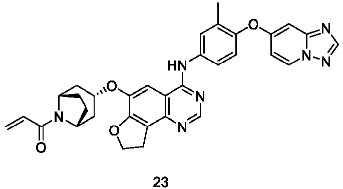 | 1-(*exo*-3-((4-((4-([1,2,4]Triazolo[1,5-*a*]pyridin-7-yloxy)-3-methylphenyl)amino)-8,9-dihydrofuro[2,3-*h*]quinazolin-6-yl)oxy)-8-azabicyclo[3.2.1]octan-8-yl)prop-2-en-1-one **23** |
| | 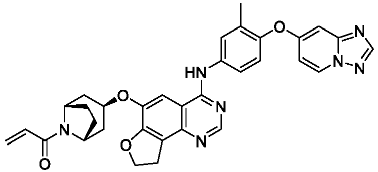 | 1-(*endo*-3-((4-((4-([1,2,4]Triazolo[1,5-*a*]pyridin-7-yloxy)-3-methylphenyl)amino)-8,9-dihydrofuro[2,3-*h*]quinazolin-6-yl)oxy)-8-azabicyclo[3.2.1]octan-8-yl)prop-2-en-1-one |
| | 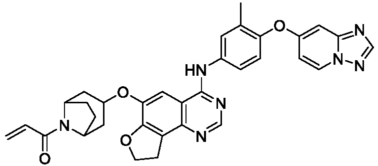 | 1-(3-((4-((4-([1,2,4]Triazolo[1,5-*a*]pyridin-7-yloxy)-3-methylphenyl)amino)-8,9-dihydrofuro[2,3-*h*]quinazolin-6-yl)oxy)-8-azabicyclo[3.2.1]octan-8-yl)prop-2-en-1-one |
| 24 | 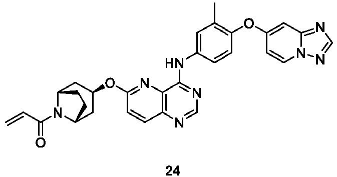 | 1-(*endo*-3-((4-((4-([1,2,4]Triazolo[1,5-*a*]pyridin-7-yloxy)-3-methylphenyl)amino)pyrido[3,2-*d*]pyrimidin-6-yl)oxy)-8-azabicyclo[3.2.1]octan-8-yl)prop-2-en-1-one **24** |
| | 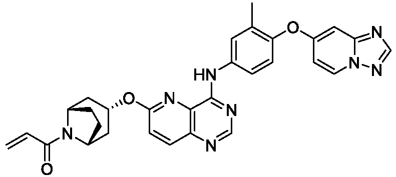 | 1-(*exo*-3-((4-((4-([1,2,4]Triazolo[1,5-*a*]pyridin-7-yloxy)-3-methylphenyl)amino)pyrido[3,2-*d*]pyrimidin-6-yl)oxy)-8-azabicyclo[3.2.1]octan-8-yl)prop-2-en-1-one |
| | 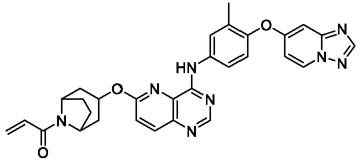 | 1-(3-((4-((4-([1,2,4]Triazolo[1,5-*a*]pyridin-7-yloxy)-3-methylphenyl)amino)pyrido[3,2-*d*]pyrimidin-6-yl)oxy)-8-azabicyclo[3.2.1]octan-8-yl)prop-2-en-1-one |
| 25 | 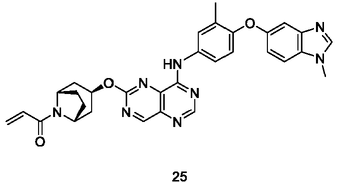 | 1-(*endo*-3-((8-((3-Methyl-4-((1-methyl-1H-benzo[*d*]imidazol-5-yl)oxy)phenyl)amino)pyrimido[5,4-*d*]pyrimidin-2-yl)oxy)-8-azabicyclo[3.2.1]octan-8-yl)prop-2-en-1-one **25** |
| | 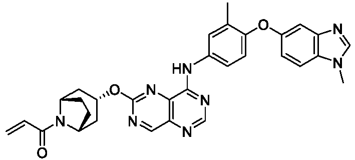 | 1-(*exo*-3-((8-((3-Methyl-4-((1-methyl-1*H*-benzo[*d*]imidazol-5-yl)oxy)phenyl)amino)pyrimido[5,4-*d*]pyrimidin-2-yl)oxy)-8-azabicyclo[3.2.1]octan-8-yl)prop-2-en-1-one |
| | 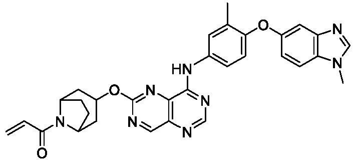 | 1-(3-((8-((3-Methyl-4-((1-methyl-1*H*-benzo[*d*]imidazol-5-yl)oxy)phenyl)amino)pyrimido[5,4-*d*]pyrimidin-2-yl)oxy)-8-azabicyclo[3.2.1]octan-8-yl)prop-2-en-1-one |
| 26 | 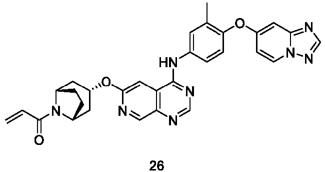 | 1-(*exo*-3-((4-((4-([1,2,4]Triazolo[1,5-*a*]pyridin-7-yloxy)-3-methylphenyl)amino)pyrido[3,4-*d*]pyrimidin-6-yl)oxy)-8-azabicyclo[3.2.1]octan-8-yl)prop-2-en-1-one **26** |
| 27 | 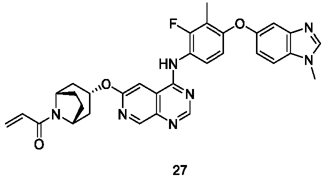 | 1-(*exo*-3-((4-((2-Fluoro-3-methyl-4-((1-methyl-1H-benzo[*d*]imidazol-5-yl)oxy)phenyl)amino)pyrido[3,4-*d*]pyrimidin-6-yl)oxy)-8-azabicyclo[3.2.1]octan-8-yl)prop-2-en-1-one **27** |
| | 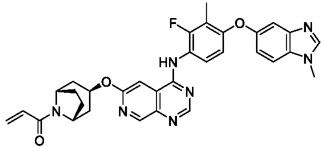 | 1-(*endo*-3-((4-((2-Fluoro-3-methyl-4-((1-methyl-1*H*-benzo[d]imidazol-5-yl)oxy)phenyl)amino)pyrido[3,4-*d*]pyrimidin-6-yl)oxy)-8-azabicyclo[3.2.1]octan-8-yl)prop-2-en-1-one |
| | 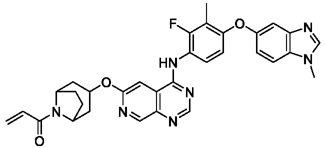 | 1-(3-((4-((2-Fluoro-3-methyl-4-((1-methyl-1*H*-benzo[*d*]imidazol-5-yl)oxy)phenyl)amino)pyrido[3,4-*d*]pyrimidin-6-yl)oxy)-8-azabicyclo[3.2.1]octan-8-yl)prop-2-en-1-one |
| 28 | 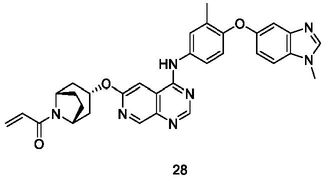 | 1-(*exo*-3-((4-((3-Methyl-4-((1-methyl-1*H*-benzo[*d*]imidazol-5-yl)oxy)phenyl)amino)pyrido[3,4-*d*]pyrimidin-6-yl)oxy)-8-azabicyclo[3.2.1]octan-8-yl)prop-2-en-1-one **28** |
| | 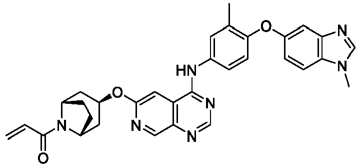 | 1-(*endo*-3-((4-((3-Methyl-4-((1-methyl-1*H*-benzo[*d*]imidazol-5-yl)oxy)phenyl)amino)pyrido[3,4-*d*]pyrimidin-6-yl)oxy)-8-azabicyclo[3.2.1]octan-8-yl)prop-2-en-1-one |
| | 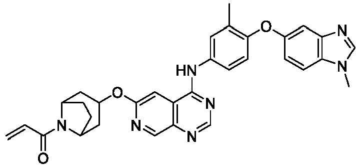 | 1-(3-((4-((3-Methyl-4-((1-methyl-1*H*-benzo[*d*]imidazol-5-yl)oxy)phenyl)amino)pyrido[3,4-*d*]pyrimidin-6-yl)oxy)-8-azabicyclo[3.2.1]octan-8-yl)prop-2-en-1-one |

Another aspect of the present disclosure relates to a compound of general formula (IIIa) or a salt thereof: 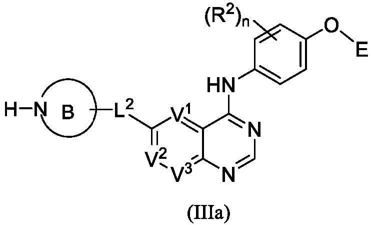 wherein:
E, ring B, R², L², V¹, V², V³, and n are as defined in general formula (III).

In some embodiments of the present disclosure, the compound of general formula (IIIa) or the salt thereof is provided, wherein V¹, V², and V³ are identical or different and are each independently C(R^{a}) or N; R^{a} is as defined in general formula (I).

**Table B. Typical intermediate compounds of the present disclosure include, but are not limited to:**

| Compound No. | Compound structure | Name |
|---|---|---|
| **1f** | 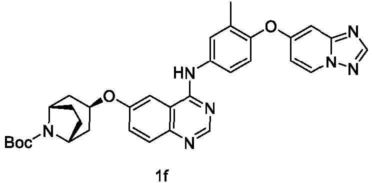 | *tert*-Butyl e*ndo*-3-((4-((4-([1,2,4]triazolo[1,5-*a*]pyridin-7-yloxy)-3-methylphenyl)amino)quinazolin-6-yl)oxy)-8-azabicyclo[3.2.1]octane-8-carboxylate **1f** |
| **1g** | 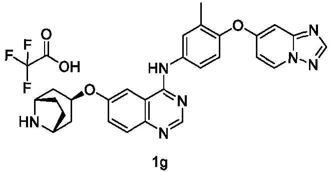 | 6-((*endo*-8-Azabicyclo[3.2.1]octan-3-yl)oxy)-*N*-(4-([1,2,4]triazolo[1,5-*a*]pyridin-7-yloxy)-3-methylphenyl)quinazolin-4-amine 2,2,2-trifluoroacetate **1g** |
| | 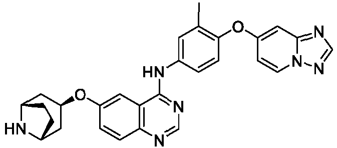 | 6-((*endo*-8-Azabicyclo[3.2.1]octan-3-yl)oxy)-*N*-(4-([1,2,4]triazolo[1,5-*a*]pyridin-7-yloxy)-3-methylphenyl)quinazolin-4-amine |
| | 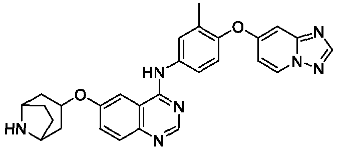 | 6-((8-Azabicyclo[3.2.1]octan-3-yl)oxy)-*N*-(4-([1,2,4]triazolo[1 ,5-*a*]pyridin-7-yloxy)-3-methylphenyl)quinazolin-4-amine |
| | 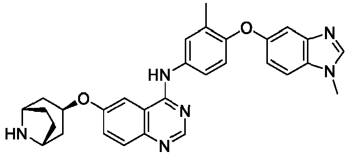 | 6-((*endo-*8-Azabicyclo[3.2.1]octan-3-yl)oxy)-*N*-(3-methyl-4-((1-methyl-1*H*-benzo[*d*])imidazol-5-yl)oxy)phenyl)quinazolin-4-amine |
| | 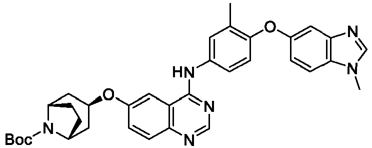 | *tert*-Butyl *endo*-3-((4-((3-methyl-4-((1-methyl-1*H*-benzo[*d*]imidazol-5-yl)oxy)phenyl)amino)quinazolin-6-yl)oxy)-8-azabicyclo[3.2.1]octane-8-carboxylate |
| | 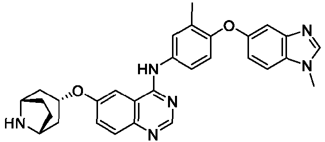 | 6-((*exo*-8-Azabicyclo[3.2.1]octan-3-yl)oxy)-*N*-(3-methyl-4-((1-methyl-1*H*-benzo[*d*])imidazol-5-yl)oxy)phenyl)quinazolin-4-amine |
| | 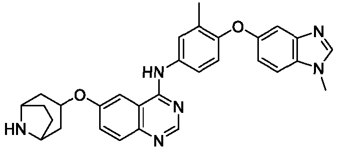 | 6-((8-Azabicyclo[3.2.1]octan-3-yl)oxy)-*N*-(3-methyl-4-((1-methyl-1*H*-benzo[*d*])imidazol-5-yl)oxy)phenyl)quinazolin-4-amine |
| 4c | 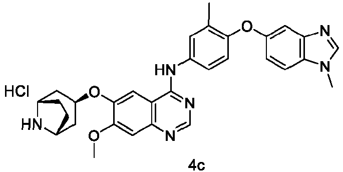 | 6-((*endo*-8-Azabicyclo[3.2.1]octan-3-yl)oxy)-7-methoxy-*N*-(3-methyl-4-((1-methyl-1*H-*benzo[*d*]imidazol-5-yl)oxy)phenyl)quinazolin-4-amine hydrochloride **4c** |
| | 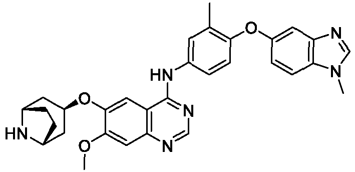 | 6-((*endo*-8-Azabicyclo[3.2.1]octan-3-yl)oxy)-7-methoxy-*N*-(3-methyl-4-((1-methyl-1*H-*benzo[*d*]imidazol-5-yl)oxy)phenyl)quinazolin-4-amine |
| | 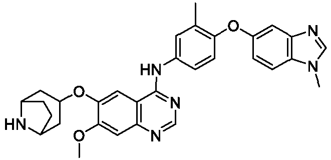 | 6-((8-Azabicyclo[3.2.1]octan-3-yl)oxy)-7-methoxy-*N*-(3-methyl-4-((1-methyl-1*H*-benzo[*d*]imidazol-5-yl)oxy)phenyl)quinazolin-4-amine |
| | 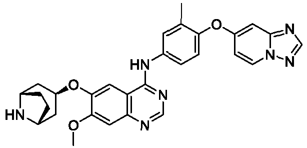 | 6-((*endo*-8-Azabicyclo[3.2.1]octan-3-yl)oxy)-*N*-(4-([1,2,4]triazolo[1,5-*a*]pyridin-7-yloxy)-3-methylphenyl)-7-methoxyquinazolin-4-amine |
| | 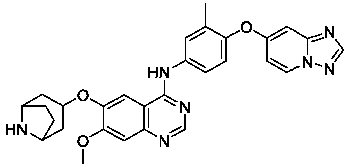 | 6-((8-Azabicyclo[3.2.1]octan-3-yl)oxy)-*N*-(4-([1,2,4]triazolo[1,5-*a*]pyridin-7-yloxy)-3-methylphenyl)-7-methoxyquinazolin-4-amine |
| 6c | 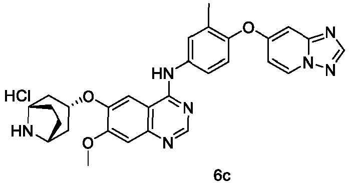 | 6-((*exo*-8-Azabicyclo[3.2. 1]octan-3-yl)oxy)-*N*-(4-([1,2,4]triazolo[1,5-*a*]pyridin-7-yloxy)-3-methylphenyl)-7-methoxyquinazolin-4-amine hydrochloride **6c** |
| | 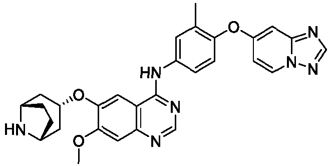 | 6-((*exo-*8-Azabicyclo[3.2.1]octan-3-yl)oxy)-*N*-(4-([1,2,4]triazolo[1,5-*a*]pyridin-7-yloxy)-3-methylphenyl)-7-methoxyquinazolin-4-amine |
| 7c | 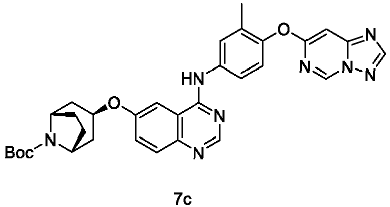 | *tert-*Butyl *endo-*3-((4-((4-([1,2,4]triazolo[1,5-*c*]pyrimidin-7-yloxy)-3-methylphenyl)amino)quinazolin-6-yl)oxy)-8-azabicyclo[3.2.1]octane-8-carboxylate **7c** |
| 7d | 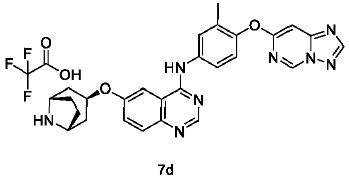 | 6-((*endo*-8-Azabicyclo[3.2.1]octan-3-yl)oxy)-*N*-(4-([1,2,4]triazolo[1,5-*c*]pyrimidin-7-yloxy)-3-methylphenyl)quinazolin-4-amine 2,2,2-trifluoroacetate **7d** |
| | 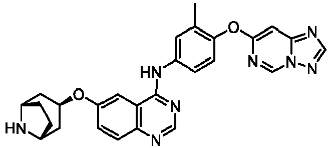 | 6-((*endo*-8-Azabicyclo[3.2.1]octan-3-yl)oxy)-*N*-(4-([1,2,4]triazolo[1,5-*c*]pyrimidin-7-yloxy)-3-methylphenyl)quinazolin-4-amine |
| | 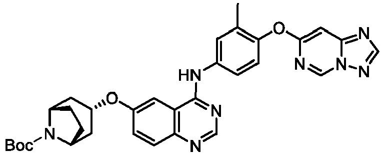 | *tert-*Butyl *exo*-3*-*((4-((4-([1,2,4]triazolo[1,5-c]pyrimidin-7-yloxy)-3-methylphenyl)amino)quinazolin-6-yl)oxy)-8-azabicyclo[3.2.1]octane-8-carboxylate |
| | 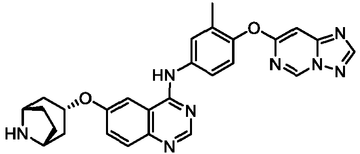 | 6-((*exo-*8-Azabicyclo[3.2.1]octan-3-yl)oxy)-*N*-(4-([1,2,4]triazolo[1,5-*c*]pyrimidin-7-yloxy)-3-methylphenyl)quinazolin-4-amine |
| | 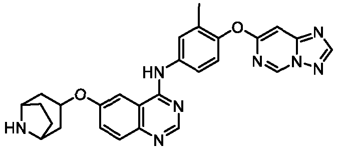 | 6-((8-Azabicyclo[3.2.1]octan-3-yl)oxy)-*N*-(4-([1,2,4]triazolo[1,5-*c*]pyrimidin-7-yloxy)-3-methylphenyl)quinazolin-4-amine |
| | 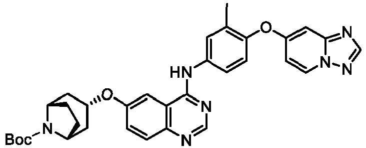 | *tert-*Butyl *exo-3*-((4-((4-([1,2,4]triazolo[1,5-*a*]pyridin-7-yloxy)-3-methylphenyl)amino)quinazolin-6-yl)oxy)-8-azabicyclo[3.2.1]octane-8-carboxylate |
| | 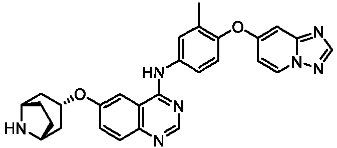 | 6-((*exo*-8-Azabicyclo[3.2.1]octan-3-yl)oxy)-*N*-(4-([1,2,4]triazolo[1,5-*a*]pyridin-7-yloxy)-3-methylphenyl)quinazolin-4-amine |
| 10a | 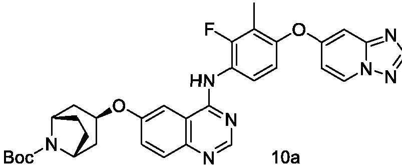 | *tert*-Butyl *endo-*3-((4-((4-([1,2,4]triazolo[1,5-*a*]pyridin-7-yloxy)-2-fluoro-3-methylphenyl)amino)quinazolin-6-yl)oxy)-8-azabicyclo[3.2.1]octane-8-carboxylate **10a** |
| 10b | 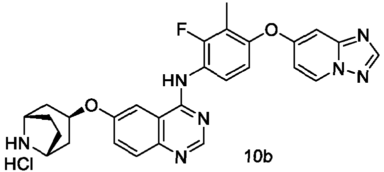 | 6-((*endo*-8-Azabicyclo[3.2.1]octan-3-yl)oxy)-*N*-(4-([1,2,4]triazolo[1,5-*a*]pyridin-7-yloxy)-2-fluoro-3-methylphenyl)quinazolin-4-amine hydrochloride **10b** |
| | 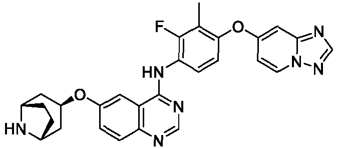 | 6-((*endo*-8-Azabicyclo[3.2.1]octan-3-yl)oxy)-*N*-(4-([1,2,4]triazolo[1,5-*a*]pyridin-7-yloxy)-2-fluoro-3-methylphenyl)quinazolin-4-amine |
| | 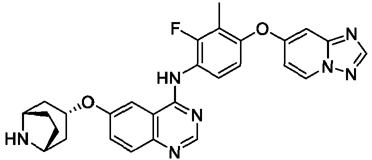 | 6-((*exo*-8-Azabicyclo[3.2.1]octan-3-yl)oxy)-*N*-(4-([1,2,4]triazolo[1,5-*a]*pyridin-7-yloxy)-2-fluoro-3-methylphenyl)quinazolin-4-amine |
| | 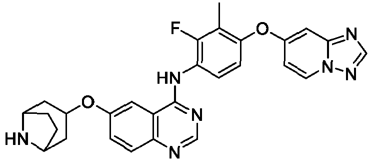 | 6-((8-Azabicyclo[3.2.1]octan-3-yl)oxy)-*N*-(4-([1,2,4]triazolo[1,5-*a*]pyridin-7-yloxy)-2-fluoro-3-methylphenyl)quinazolin-4-amine |
| | 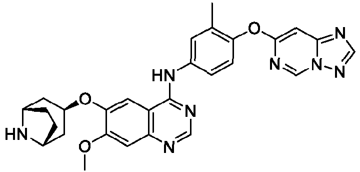 | 6-(*endo*-8-Azabicyclo[3.2.1]octan-3-yl)oxy)-*N*-(4-([1,2,4]triazolo[1,5-*c*]pyrimidin-7-yloxy)-3-methylphenyl)-7-methoxyquinazolin-4-amine |
| 12a | 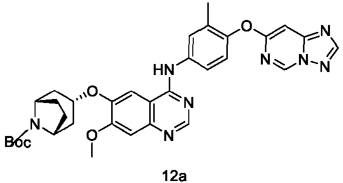 | *tert-*Butyl *exo-*3*-*((4-((4-([1,2,4]triazolo[1,5-*c*]pyrimidin-7-yloxy)-3-methylphenyl)amino)-7-methoxyquinazolin-6-yl)oxy)-8-azabicyclo[3.2.1]octane-8-carboxylate **12a** |
| 12b | 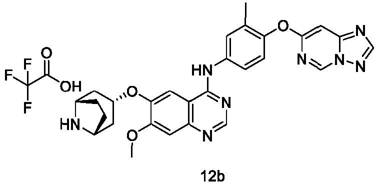 | 6-(*exo*-8-Azabicyclo[3.2.1]octan-3-yl)oxy)-*N*-(4-([1,2,4]triazolo[1,5-*c*]pyrimidin-7-yloxy)-3-methylphenyl)-7-methoxyquinazolin-4-amine 2,2,2-trifluoroacetate **12b** |
| | 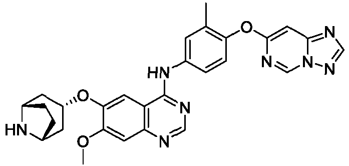 | 6-(*exo*-8-Azabicyclo[3.2.1]octan-3-yl)oxy)-*N*-(4-([1,2,4]triazolo[1,5-c]pyrimidin-7-yloxy)-3-methylphenyl)-7-methoxyquinazolin-4-amine |
| | 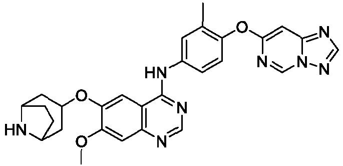 | 6-(8-Azabicyclo[3.2.1]octan-3-yl)oxy)-*N*-(4-([1,2,4]triazolo[1,5-*c*]pyrimidin-7-yloxy)-3-methylphenyl)-7-methoxyquinazolin-4-amine |
| | 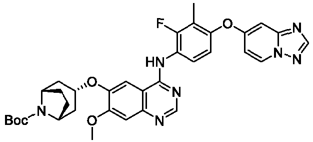 | *tert*-Butyl *exo*-3-((4-((4-([1,2,4]triazolo[1,5-*a*]pyridin-7-yloxy)-2-fluoro-3-methylphenyl)amino)-7-methoxyquinazolin-6-yl)oxy)-8-azabicyclo[3.2.1]octane-8-carboxylate |
| | 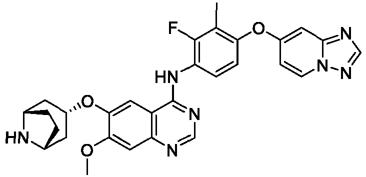 | 6-((*exo*-8-Azabicyclo[3.2.1]octan-3-yl)oxy)-*N*-(4-([1,2,4]triazolo[1,5-*a*]pyridin-7-yloxy)-2-fluoro-3-methylphenyl)-7-methoxyquinazolin-4-amine |
| | 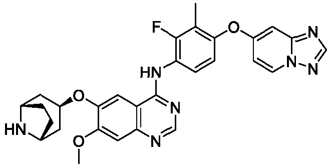 | 6-((*endo*-8-Azabicyclo[3.2.1]octan-3-yl)oxy)-*N*-(4-([1,2,4]triazolo[1,5-*a*]pyridin-7-yloxy)-2-fluoro-3-methylphenyl)-7-methoxyquinazolin-4-amine |
| | 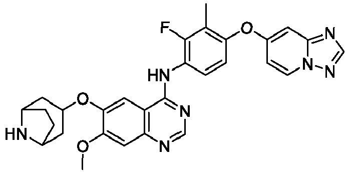 | 6-((8-Azabicyclo[3.2.1]octan-3-yl)oxy)-*N*-(4-([1,2,4]triazolo[1,5-*a*]pyridin-7-yloxy)-2-fluoro-3-methylphenyl)-7-methoxyquinazolin-4-amine |
| | 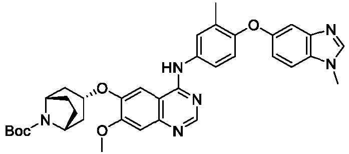 | *tert*-Butyl *exo*-3-((7-methoxy-4-((3-methyl-4-((1-methyl-1*H-*benzo[*d*]imidazol-5-yl)oxy)phenyl)amino)quinazolin-6-yl)oxy)-8-azabicyclo[3.2.1]octane-8-carboxylate |
| | 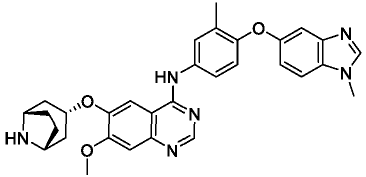 | 6-((*exo*-8-Azabicyclo[3.2.1]octan-3-yl)oxy)-7-methoxy-*N*-(3-methyl-4-((1-methyl-1*H-*benzo[*d*]imidazol-5-yl)oxy)phenyl)quinazolin-4-amine |
| | 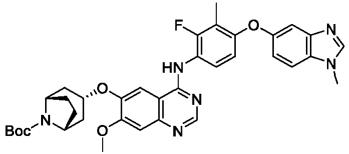 | *tert*-Butyl *exo-*3-((4-((2-fluoro-3-methyl-4-((1-methyl-1*H-*benzo[*d*]imidazol-5-yl)oxy)phenyl)amino)-7-methoxyquinazolin-6-yl)oxy)-8-azabicyclo[3.2.1]octane-8-carboxylate |
| | 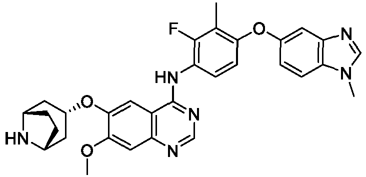 | 6-((*exo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)-7-methoxyquinazolin-4-amine |
| | 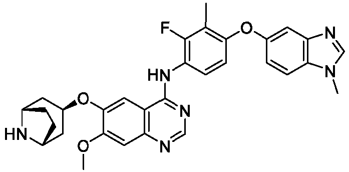 | 6-((*endo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)-7-methoxyquinazolin-4-amine |
| | 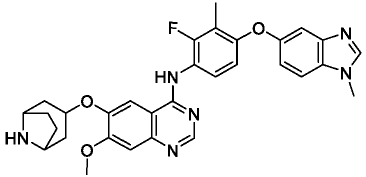 | 6-((8-Azabicyclo[3.2.1]octan-3-yl)oxy)-*N*-(2-fluoro-3-methyl-4-((1-methyl-1*H*-benzo[*d*]imidazol-5-yl)oxy)phenyl)-7-methoxyquinazolin-4-amine |
| 16f | 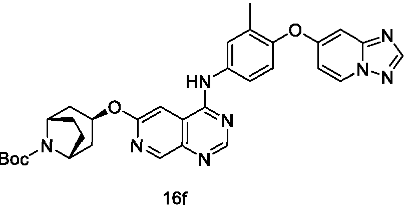 | *tert-*Butyl *endo-*3-((4-((4-([1,2,4]triazolo[1,5-*a*]pyridin-7-yloxy)-3-methylphenyl)amino)pyrido[3,4-*d*]pyrimidin-6-yl)oxy)-8-azabicyclo[3.2.1]octane-8-carboxylate **16f** |
| 16g | 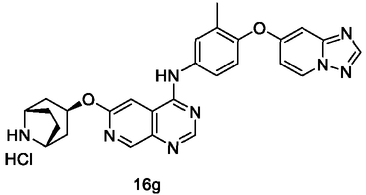 | 6-((*endo*-8-Azabicyclo[3.2.1]octan-3-yloxy)-*N*-(4-([1,2,4]triazolo[1,5-*a*]pyridin-7-yloxy)-3-methylphenyl)pyrido[3,4-*d*]pyrimidin-4-amine hydrochloride **16g** |
| | 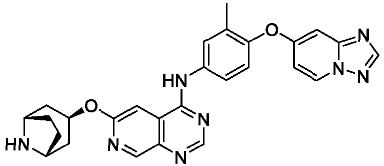 | 6-((*endo*-8-Azabicyclo[3.2.1]octan-3-yl)oxy)-*N*-(4-([1,2,4]triazolo[1,5-*a*]pyridin-7-yloxy)-3-methylphenyl)pyrido[3,4-*d*]pyrimidin-4-amine |
| | 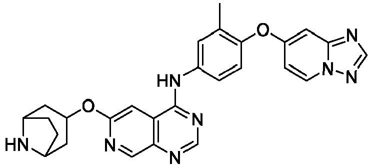 | 6-((8-Azabicyclo[3.2.1]octan-3-yl)oxy)-*N*-(4-([1,2,4]triazolo[1,5-*a*]pyridin-7-yloxy)-3-methylphenyl)pyrido[3,4-*d*]pyrimidin-4-amine |
| | 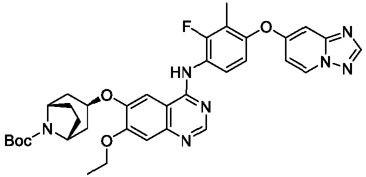 | *tert*-Butyl *endo*-3-((4-((4-([1,2,4]triazolo[1,5-*a*]pyridin-7-yloxy)-2-fluoro-3-methylphenyl)amino)-7-ethoxyquinazolin-6-yl)oxy)-8-azabicyclo[3.2.1]octane-8-carboxylate |
| | 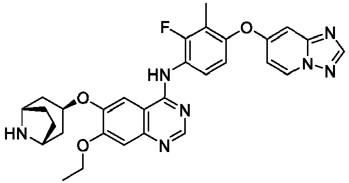 | 6-((*endo*-8-Azabicyclo[3.2.1]octan-3-yloxy)-*N*-(4-([1,2,4]triazolo[1,5-*a*]pyridin-7-yloxy)-2-fluoro-3-methylphenyl)-7-ethoxyquinazolin-4-amine |
| | 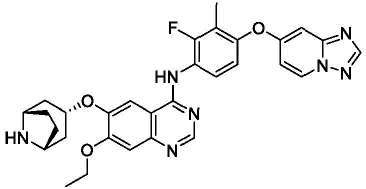 | 6-((*exo*-8-Azabicyclo[3.2.1]octan-3-yl)oxy)-*N*-(4-([1,2,4]triazolo[1,5-*a*]pyridin-7-yloxy)-2-fluoro-3-methylphenyl)-7-ethoxyquinazolin-4-amine |
| | 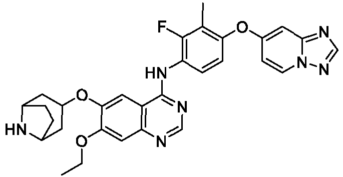 | 6-((8-Azabicyclo[3.2.1]octan-3-yl)oxy)-*N*-(4-([1,2,4]triazolo[1,5-*a*]pyridin-7-yloxy)-2-fluoro-3-methylphenyl)-7-ethoxyquinazolin-4-amine |
| | 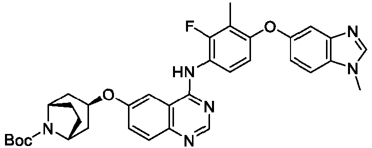 | *tert*-Butyl *endo*-3-((4-((2-fluoro-3-methyl-4-((1-methyl-1*H-*benzo[*d*]imidazol-5-yl)oxy)phenyl)amino)quinazolin-6-yl)oxy)-8-azabicyclo[3.2.1]octane-8-carboxylate |
| | 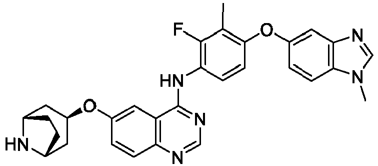 | 6-((*endo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)quinazolin-4-amine |
| 19b | 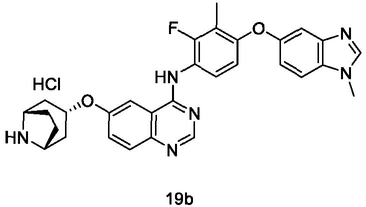 | 6-((*exo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)quinazolin-4-amine hydrochloride **19b** |
| | 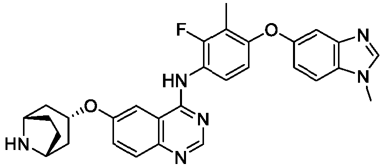 | 6-((*exo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)quinazolin-4-amine |
| | 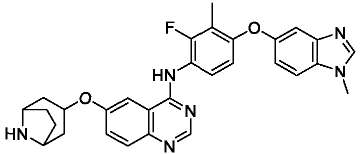 | 6-((8-Azabicyclo[3.2.1]octan-3-yl)oxy)-*N*-(2-fluoro-3-methyl-4-((1-methyl-1*H*-benzo[*d*]imidazol-5-yl)oxy)phenyl)quinazolin-4-amine |
| | 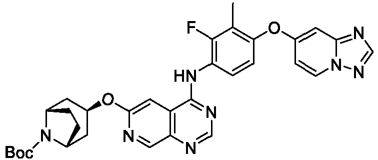 | *tert-*Butyl *endo-*3-((4-((4-([1,2,4]triazolo[1,5-*a*]pyridin-7-yloxy)-2-fluoro-3-methylphenyl)amino)pyrido[3,4-*d*]pyrimidin-6-yl)oxy)-8-azabicyclo[3.2.1]octane-8-carboxylate |
| | 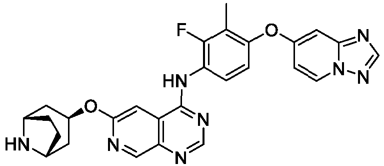 | 6-((*endo*-8-Azabicyclo[3.2.1]octan-3-yl)oxy)-*N*-(4-([1,2,4]triazolo[1,5-*a*]pyridin-7-yloxy)-2-fluoro-3-methylphenyl)pyrido[3,4-*d*]pyrimidin-4-amine |
| 21b | 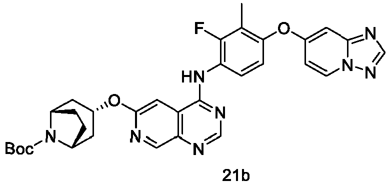 | *tert*-Butyl *exo*-3-((4-((4-([1,2,4]triazolo[1,5-*a*]pyridin-7-yloxy)-2-fluoro-3-methylphenyl)amino)pyrido[3,4-*d*]pyrimidin-6-yl)oxy)-8-azabicyclo[3.2.1]octane-8-carboxylate **21b** |
| 21c | 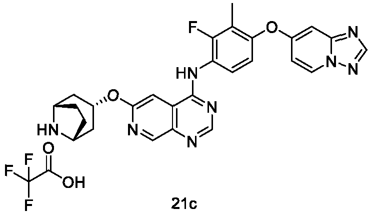 | 6-((*exo*-8-Azabicyclo[3.2.1]octan-3-yl)oxy)-*N*-(4-([1,2,4]triazolo[1,5-*a*]pyridin-7-yloxy)-2-fluoro-3-methylphenyl)pyrido[3,4-*d*]pyrimidin-4-amine 2,2,2-trifluoroacetate **21c** |
| | 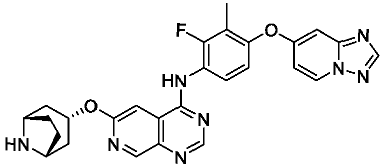 | 6-((*exo*-8-Azabicyclo[3.2.1]octan-3-yl)oxy)-*N*-(4-([1,2,4]triazolo[1,5-*a*]pyridin-7-yloxy)-2-fluoro-3-methylphenyl)pyrido[3,4-*d*]pyrimidin-4-amine |
| | 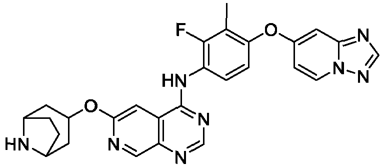 | 6-((8-Azabicyclo[3.2.1]octan-3-yl)oxy)-*N*-(4-([1,2,4]triazolo[1,5-*a*]pyridin-7-yloxy)-2-fluoro-3-methylphenyl)pyrido[3,4-*d*]pyrimidin-4-amine |
| 22b | 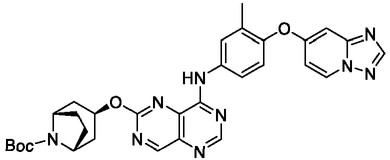 | *tert*-Butyl *endo*-3-((8-((4-([1,2,4]triazolo[1,5-*a*]pyridin-7-yloxy)-3-methylphenyl)amino)pyrimido[5,4-*d*]pyrimidin-2-yl)oxy)-8-azabicyclo[3.2.1]octane-8-carboxylate **22b** |
| | 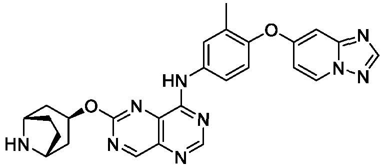 | 6-((*endo*-8-Azabicyclo[3.2.1]octan-3-yl)oxy)-*N*-(4-([1,2,4]triazolo[1,5-*a*]pyridin-7-yloxy)-3-methylphenyl)pyrimido[5,4-*d*]pyrimidin-4-amine |
| | 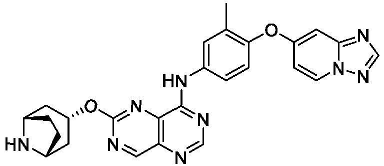 | 6-((*exo*-8-Azabicyclo[3.2.1]octan-3-yl)oxy)-*N*-(4-([1,2,4]triazolo[1,5-*a*]pyridin-7-yloxy)-3-methylphenyl)pyrimido[5,4-*d*]pyrimidin-4-amine |
| | 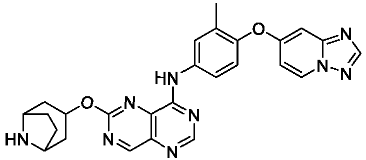 | 6-((8-Azabicyclo[3.2.1]octan-3-yl)oxy)-*N*-(4-([1,2,4]triazolo[1,5-*a*]pyridin-7-yloxy)-3-methylphenyl)pyrimido[5,4-*d*]pyrimidin-4-amine |
| 23g | 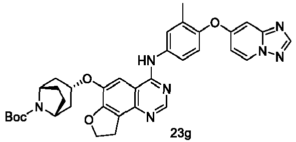 | *tert*-Butyl *exo*-3-((4-((4-([1,2,4]triazolo[1,5-*a*]pyridin-7-yloxy)-3-methylphenyl)amino)-8,9-dihydrofuro[2,3-*h*]quinazolin-6-yl)oxy)-8-azabicyclo[3.2.1]octane-8-carboxylate **23g** |
| | 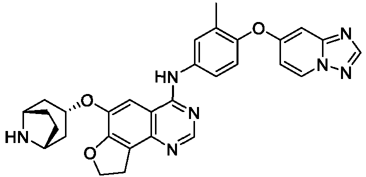 | 6-((*exo-*8-Azabicyclo[3.2.1]octan-3-yl)oxy)-*N*-(4-([1,2,4]triazolo[1,5-*a*]pyridin-7-yloxy)-3-methylphenyl)-8,9-dihydrofuro[2,3-*h*]quinazolin-4-amine |
| | 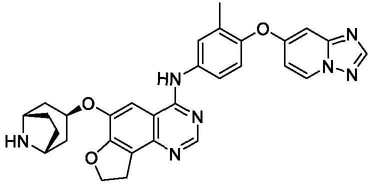 | 6-((*endo-*8-Azabicyclo[3.2.1]octan-3-yl)oxy)-*N*-(4-([1,2,4]triazolo[1,5-*a*]pyridin-7-yloxy)-3-methylphenyl)-8,9-dihydrofuro[2,3-*h*]quinazolin-4-amine |
| | 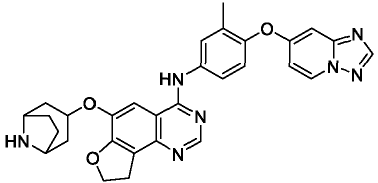 | 6-((8-Azabicyclo[3.2.1]octan-3-yl)oxy)-*N*-(4-([1,2,4]triazolo[1,5-*a*]pyridin-7-yloxy)-3-methylphenyl)-8,9-dihydrofuro[2,3-*h*]quinazolin-4-amine |
| | 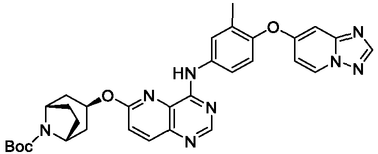 | *tert-*Butyl *endo-*3-((4-((4-([1,2,4]triazolo[1,5-*a*]pyridin-7-yloxy)-3-methylphenyl)amino)pyrido[3,2-*d*]pyrimidin-6-yl)oxy)-8-azabicyclo[3.2.1]octane-8-carboxylate |
| | 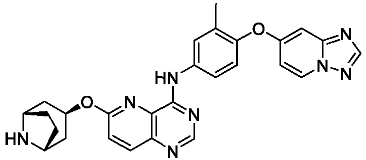 | 6-((*endo*-8-Azabicyclo[3.2.1]octan-3-yl)oxy)-*N*-(4-([1,2,4]triazolo[1,5-*a*]pyridin-7-yloxy)-3-methylphenyl)pyrido[3,2-*d*]pyrimidin-4-amine |
| | 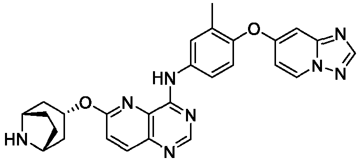 | 6-((*exo*-8-Azabicyclo[3.2.1]octan-3-yl)oxy)-*N*-(4-([1,2,4]triazolo[1,5-*a*]pyridin-7-yloxy)-3-methylphenyl)pyrido[3,2-*d*]pyrimidin-4-amine |
| | 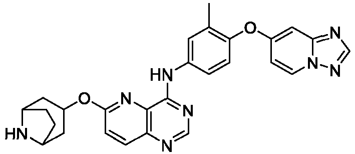 | 6-((8-Azabicyclo[3.2.1]octan-3-yl)oxy)-*N*-(4-([1,2,4]triazolo[1,5-*a*]pyridin-7-yloxy)-3-methylphenyl)pyrido[3,2-*d*]pyrimidin-4-amine |
| | 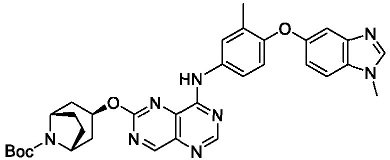 | *tert-*Butyl *endo*-3-((8-((3-methyl-4-((1-methyl-1*H*-benzo[*d*]imidazol-5-yl)oxy)phenyl)amino)pyrimido[5,4 -*d*]pyrimidin-2-yl)oxy)-8-azabicyclo[3.2.1]octane-8-carboxylate |
| | 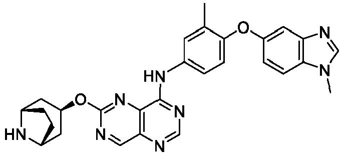 | 6-((*endo*-8-Azabicyclo[3.2.1]octan-3-yl)oxy)-*N*-(3-methyl-4-((1-methyl-1*H*-benzo[*d*])imidazol-5-yl)oxy)phenyl)benzo[5,4-*d*]pyrimidin-4-amine |
| | 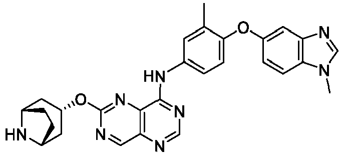 | 6-((exo-8-Azabicyclo[3.2.1]octan-3-yl)oxy)-*N*-(3-methyl-4-((1-methyl-1*H*-benzo[*d*])imidazol-5-yl)oxy)phenyl)benzo[5,4-*d*]pyrimidin-4-amine |
| | 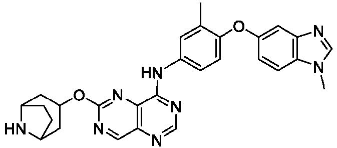 | 6-((8-Azabicyclo[3.2.1]octan-3-yl)oxy)-*N*-(3-methyl-4-((1-methyl-1*H*-benzo[*d*])imidazol-5-yl)oxy)phenyl)benzo[5,4-*d*]pyrimidin-4-amine |
| 26a | 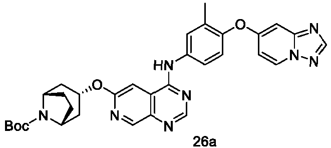 | *tert-*Butyl *exo*-3-((4-((4-([1,2,4]triazolo[1,5-*a*]pyridin-7-yloxy)-3-methylphenyl)amino)pyrido[3,4-*d*]pyrimidin-6-yl)oxy)-8-azabicyclo[3.2.1]octane-8-carboxylate **26a** |
| 26b | 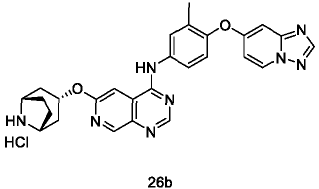 | 6-((*exo*-8-Azabicyclo[3.2.1]octan-3-yl)oxy)-*N*-(4-([1,2,4]triazolo[1,5-*a*]pyridin-7-yloxy)-3-methylphenyl)pyrido[3,4-*d*]pyrimidin-4-amine hydrochloride **26b** |
| | 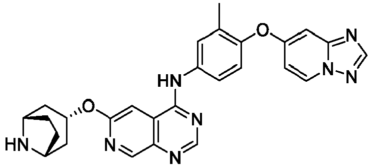 | 6-((*exo-*8-Azabicyclo[3.2.1]octan-3-yl)oxy)-*N*-(4-([1,2,4]triazolo[1,5-*a*]pyridin-7-yloxy)-3-methylphenyl)pyrido[3,4-*d*]pyrimidin-4-amine |
| | 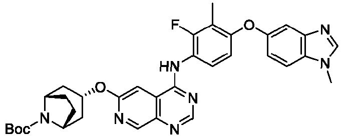 | *tert*-Butyl *exo*-3-((4-((2-fluoro-3-methyl-4-((1-methyl-1*H-*benzo[*d*]imidazol-5-yl)oxy)phenyl)amino)pyrido[3,4-*d*]pyrimidin-6-yl)oxy)-8-azabicyclo[3.2.1]octane-8-carboxylate |
| | 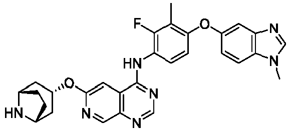 | 6-((*exo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)pyrido[3,4-*d*]pyrimidin-4-amine |
| | 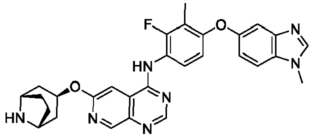 | 6-((*endo*-8-Azabicyclo[3.2.1]octan-3-yl)oxy)-*N*-(2-fluoro-3-methyl-4-((1-methyl-1*H*-benzo[*d*]imidazol-5-yl)oxy)phenyl)pyrido[3,4-*d*]pyrimidin-4-amine |
| | 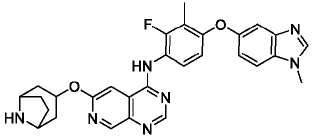 | 6-((8-Azabicyclo[3.2.1]octan-3-yl)oxy)-*N*-(2-fluoro-3-methyl-4-((1-methyl-1*H*-benzo[*d*]imidazol-5-yl)oxy)phenyl)pyrido[3,4-*d*]pyrimidin-4-amine |
| | 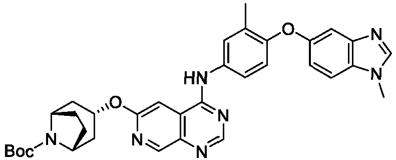 | *tert*-Butyl *exo*-3-((4-((3-methyl-4-((1-methyl-1*H*-benzo[*d*]imidazol-5-yl)oxy)phenyl)amino)pyrido[3,4-*d*]pyrimidin-6-yl)oxy)-8-azabicyclo[3.2.1]octane-8-carboxylate |
| | 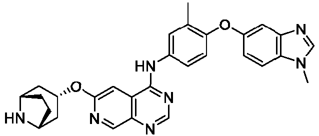 | 6-((*exo*-8-Azabicyclo[3.2.1]octan-3-yl)oxy)-*N*-(3-methyl-4-((1-methyl-1*H*-benzo[*d*]imidazol-5-yl)oxy)phenyl)pyrido[3,4-*d*]pyrimidin-4-amine |
| | 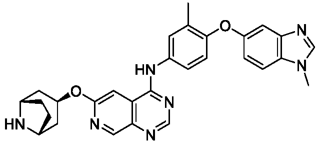 | 6-((*endo*-8-Azabicyclo[3.2.1]octan-3-yl)oxy)-*N*-(3-methyl-4-((1-methyl-1*H*-benzo[*d*]imidazol-5-yl)oxy)phenyl)pyrido[3,4-*d*]pyrimidin-4-amine |
| | 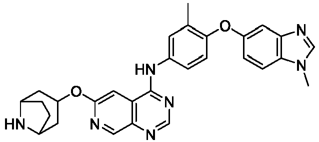 | 6-((8-Azabicyclo[3.2.1]octan-3-yl)oxy)-*N*-(3-methyl-4-((1-methyl-1*H*-benzo[*d*]imidazol-5-yl)oxy)phenyl)pyrido[3,4-*d*]pyrimidin-4-amine |

Another aspect of the present disclosure relates to a method for preparing a compound of general formula (II) or a pharmaceutically acceptable salt thereof, comprising the following step: 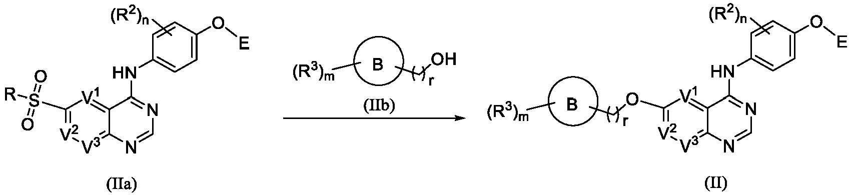 conducting a nucleophilic substitution reaction of a compound of general formula (IIa) or a salt thereof with a compound of general formula (IIb) or a salt thereof to give the compound of general formula (II) or the pharmaceutically acceptable salt thereof;
wherein:
R is C₁₋₆ alkyl; preferably, R is methyl;
E, ring B, R², R³, V¹, V², V³, r, m, and n are as defined in general formula (II).

Another aspect of the present disclosure relates to a method for preparing a compound of general formula (III) or a pharmaceutically acceptable salt thereof, comprising the following step: 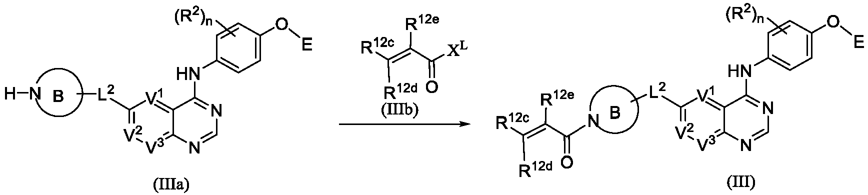 conducting a condensation reaction of a compound of general formula (IIIa) or a salt thereof with a compound of general formula (IIIb) or a salt thereof to give the compound of general formula (III) or the pharmaceutically acceptable salt thereof;
wherein:
X^{L} is halogen; preferably, X^{L} is chlorine;
E, ring B, R², L², V¹, V², V³, R^{12c}, R^{12d}, R^{12e}, and n are as defined in general formula (III). Another aspect of the present disclosure relates to a pharmaceutical composition comprising the compound of general formula (I), general formula (II), or general formula (III) disclosed herein and the compound shown in Table A or the pharmaceutically acceptable salt thereof, and one or more pharmaceutically acceptable carriers, diluents, or excipients.

The present disclosure further relates to use of the compound of general formula (I), general formula (II), or general formula (III) and the compound shown in Table A or the pharmaceutically acceptable salt thereof, or the pharmaceutical composition comprising same, in the preparation of a type I receptor tyrosine kinase inhibitor.

The present disclosure further relates to use of the compound of general formula (I), general formula (II), or general formula (III) and the compound shown in Table A or the pharmaceutically acceptable salt thereof, or the pharmaceutical composition comprising same, in the preparation of a HER2 inhibitor.

The present disclosure further relates to use of the compound of general formula (I), general formula (II), or general formula (III) and the compound shown in Table A or the pharmaceutically acceptable salt thereof, or the pharmaceutical composition comprising same, in the preparation of a drug for inhibiting HER2.

The present disclosure further relates to use of the compound of general formula (I), general formula (II), or general formula (III) and the compound shown in Table A or the pharmaceutically acceptable salt thereof, or the pharmaceutical composition comprising same, in the preparation of a drug for treating and/or preventing a HER2-mediated disease or disorder.

The present disclosure further relates to use of the compound of general formula (I), general formula (II), or general formula (III) and the compound shown in Table A or the pharmaceutically acceptable salt thereof, or the pharmaceutical composition comprising same, in the preparation of a drug for treating and/or preventing a disease or disorder by inhibiting HER2.

The present disclosure further relates to use of the compound of general formula (I), general formula (II), or general formula (III) or the compound shown in Table A or the pharmaceutically acceptable salt thereof, or the pharmaceutical composition comprising same, in the preparation of a drug for treating and/or preventing cancer; the cancer is preferably selected from the group consisting of brain cancer, breast cancer, ovarian cancer, lung cancer, anal cancer, melanoma, neuroblastoma, colorectal cancer, cervical cancer, fallopian tube cancer, endometrial cancer, prostate cancer, gastric cancer, head and neck cancer, nasopharyngeal cancer, oral cancer, bile duct cancer, esophageal cancer, liver cancer, skin cancer, mesothelioma, bladder cancer, renal cell cancer, renal pelvis cancer, ureter cancer, small intestine cancer, pancreatic cancer, thyroid cancer, parathyroid cancer, vaginal cancer, vulva cancer, leukemia, adrenal cancer, cancer of the urinary tract, penile cancer, testicular cancer, bone cancer, osteosarcoma, myeloma, soft tissue sarcoma, pituitary adenoma, brain stem neuroglioma, spinal tumor, and lymphoma; more preferably, the cancer is selected from the group consisting of breast cancer, gastric cancer, lung cancer, colorectal cancer, pancreatic cancer, prostate cancer, bladder cancer, and ovarian cancer; further preferably, the lung cancer is non-small cell lung cancer.

The present disclosure also relates to a method for inhibiting HER2, comprising administering to a patient in need thereof a therapeutically effective amount of the compound of general formula (I), general formula (II), or general formula (III) and the compound shown in Table A or the pharmaceutically acceptable salt thereof, or the pharmaceutical composition comprising same.

The present disclosure also relates to a method for treating and/or preventing a disease or disorder by inhibiting HER2, comprising administering to a patient in need thereof a therapeutically effective amount of the compound of general formula (I), general formula (II), or general formula (III) and the compound shown in Table A or the pharmaceutically acceptable salt thereof, or the pharmaceutical composition comprising same.

The present disclosure also relates to a method for treating and/or preventing a HER2-mediated disease or disorder, comprising administering to a patient in need thereof a therapeutically effective amount of the compound of general formula (I), general formula (II), or general formula (III) and the compound shown in Table A or the pharmaceutically acceptable salt thereof, or the pharmaceutical composition comprising same.

The present disclosure further relates to a method for treating and/or preventing cancer, comprising administering to a patient in need thereof a therapeutically effective amount of the compound of general formula (I), general formula (II), or general formula (III) and the compound shown in Table A or the pharmaceutically acceptable salt thereof, or the pharmaceutical composition comprising same; the cancer is preferably selected from the group consisting of brain cancer, breast cancer, ovarian cancer, lung cancer, anal cancer, melanoma, neuroblastoma, colorectal cancer, cervical cancer, fallopian tube cancer, endometrial cancer, prostate cancer, gastric cancer, head and neck cancer, nasopharyngeal cancer, oral cancer, bile duct cancer, esophageal cancer, liver cancer, skin cancer, mesothelioma, bladder cancer, renal cell cancer, renal pelvis cancer, ureter cancer, small intestine cancer, pancreatic cancer, thyroid cancer, parathyroid cancer, vaginal cancer, vulva cancer, leukemia, adrenal cancer, cancer of the urinary tract, penile cancer, testicular cancer, bone cancer, osteosarcoma, myeloma, soft tissue sarcoma, pituitary adenoma, brain stem neuroglioma, spinal tumor, and lymphoma; more preferably, the cancer is selected from the group consisting of breast cancer, gastric cancer, lung cancer, colorectal cancer, pancreatic cancer, prostate cancer, bladder cancer, and ovarian cancer; further preferably, the lung cancer is non-small cell lung cancer.

The present disclosure further relates to a compound of general formula (I), general formula (II), or general formula (III) and a compound shown in Table A or a pharmaceutically acceptable salt thereof, or a pharmaceutical composition comprising same, for use as a drug.

The present disclosure further relates to a compound of general formula (I), general formula (II), or general formula (III) and a compound shown in Table A or a pharmaceutically acceptable salt thereof, or a pharmaceutical composition comprising same, for use as a HER2 inhibitor.

The present disclosure further relates to a compound of general formula (I), general formula (II), or general formula (III) and a compound shown in Table A or a pharmaceutically acceptable salt thereof, or a pharmaceutical composition comprising same, for use in inhibiting HER2.

The present disclosure further relates to a compound of general formula (I), general formula (II), or general formula (III) and a compound shown in Table A or a pharmaceutically acceptable salt thereof, or a pharmaceutical composition comprising same, treating and/or preventing a disease or disorder by inhibiting HER2.

The present disclosure further relates to a compound of general formula (I), general formula (II), or general formula (III) and a compound shown in Table A or a pharmaceutically acceptable salt thereof, or a pharmaceutical composition comprising same, for use in treating and/or preventing a HER2-mediated disease or disorder.

The present disclosure further relates to a compound of general formula (I), general formula (II), or general formula (III) and a compound shown in Table A or a pharmaceutically acceptable salt thereof, or a pharmaceutical composition comprising same, for use in treating and/or preventing cancer; the cancer is preferably selected from the group consisting of brain cancer, breast cancer, ovarian cancer, lung cancer, anal cancer, melanoma, neuroblastoma, colorectal cancer, cervical cancer, fallopian tube cancer, endometrial cancer, prostate cancer, gastric cancer, head and neck cancer, nasopharyngeal cancer, oral cancer, bile duct cancer, esophageal cancer, liver cancer, skin cancer, mesothelioma, bladder cancer, renal cell cancer, renal pelvis cancer, ureter cancer, small intestine cancer, pancreatic cancer, thyroid cancer, parathyroid cancer, vaginal cancer, vulva cancer, leukemia, adrenal cancer, cancer of the urinary tract, penile cancer, testicular cancer, bone cancer, osteosarcoma, myeloma, soft tissue sarcoma, pituitary adenoma, brain stem neuroglioma, spinal tumor, and lymphoma; more preferably, the cancer is selected from the group consisting of breast cancer, gastric cancer, lung cancer, colorectal cancer, pancreatic cancer, prostate cancer, bladder cancer, and ovarian cancer; further preferably, the lung cancer is non-small cell lung cancer.

The disease or disorder described herein is one that is treated and/or prevented by inhibiting HER2.

In some embodiments of the present disclosure, the disease or disorder is cancer; the cancer is preferably selected from the group consisting of brain cancer, breast cancer, ovarian cancer, lung cancer, anal cancer, melanoma, neuroblastoma, colorectal cancer, cervical cancer, fallopian tube cancer, endometrial cancer, prostate cancer, gastric cancer, head and neck cancer, nasopharyngeal cancer, oral cancer, bile duct cancer, esophageal cancer, liver cancer, skin cancer, mesothelioma, bladder cancer, renal cell cancer, renal pelvis cancer, ureter cancer, small intestine cancer, pancreatic cancer, thyroid cancer, parathyroid cancer, vaginal cancer, vulva cancer, leukemia, adrenal cancer, cancer of the urinary tract, penile cancer, testicular cancer, bone cancer, osteosarcoma, myeloma, soft tissue sarcoma, pituitary adenoma, brain stem neuroglioma, spinal tumor, and lymphoma; more preferably, the cancer is selected from the group consisting of breast cancer, gastric cancer, lung cancer, colorectal cancer, pancreatic cancer, prostate cancer, bladder cancer, and ovarian cancer; further preferably, the lung cancer is non-small cell lung cancer.

In some embodiments of the present disclosure, HER2 is mutant HER2, preferably exon 20-mutated HER2. The exon 20 mutation is preferably an insertion mutation of 4 amino acids YVMA (p.A775_G776insYVMA).

As a general guide, the active compound of the present disclosure is preferably in the form of a unit dose, or in the form of a single dose that can be self-administered by a patient. The unit dose of the compound or composition of the present disclosure may be expressed in the form of a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 to 99 wt.% of the active compound.

The pharmaceutical composition comprising the active ingredient may be in a form suitable for oral administration, for example, in the form of a tablet, dragee, lozenge, aqueous or oil suspension, dispersible powder or granule, emulsion, hard or soft capsule, or syrup or elixir. An oral composition may be prepared by following any method known in the art for preparing pharmaceutical compositions, and such a composition may comprise one or more ingredients selected from the group consisting of a sweetener, a corrigent, a colorant, and a preservative, so as to provide a pharmaceutical formulation that is pleasing to the eye and palatable. The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant. The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using suitable dispersants or wetting agents and suspending agents. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

As is well known to those skilled in the art, the dosage of the drug depends on a variety of factors, including, but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of treatment, the daily dose of the compound, or the type of pharmaceutically acceptable salts, can be verified according to conventional treatment regimens.

### Terminology

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl is preferably an alkyl group having 1 to 12 carbon atoms (i.e., C₁₋₁₂ alkyl), and more preferably an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e., 3- to 12-membered cycloalkyl), further preferably a cycloalkyl group having 3 to 8 ring atoms (i.e., 3- to 8-membered cycloalkyl), more preferably a cycloalkyl group having 3 to 6 ring atoms (i.e., 3- to 6-membered cycloalkyl), and most preferably a cycloalkyl group having 5 or 6 ring atoms (i.e., 5- or 6-membered cycloalkyl).

Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain in the rings one or more double bonds or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one all-carbon ring is contained and the point of attachment is on the all-carbon ring; and which has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6- to 14-membered spirocycloalkyl), and more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include: 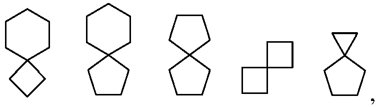 wherein the point of attachment may be at any position; 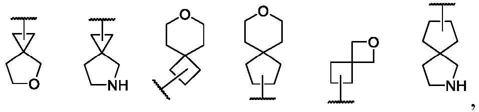 and the like.

The term "fused cycloalkyl" refers to a polycyclic system in which two adjacent carbon atoms are shared between rings, which is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group; and which may contain in the rings one or more double bonds and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), and more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include: 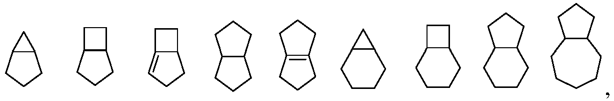 wherein the point of attachment may be at any position; 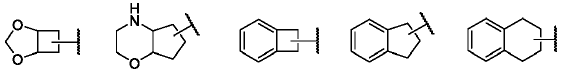 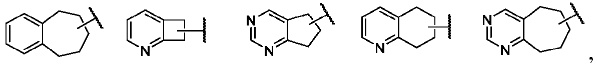 and the like.

The term "bridged cycloalkyl" refers to an all-carbon polycyclic system in which two carbon atoms that are not directly connected are shared between rings, which may contain in the rings one or more double bonds and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl).

The bridged cycloalkyl is preferably a bridged cycloalkyl group having 6 to 14 carbon atoms (i.e., 6- to 14-membered bridged cycloalkyl), and more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl), preferably bicyclic bridged cycloalkyl or tricyclic bridged cycloalkyl. Non-limiting examples include: 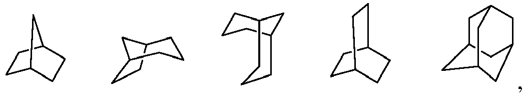 wherein the point of attachment may be at any position.

Cycloalkyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl), e.g., 4- to 12-membered heterocyclyl containing at least one nitrogen atom; further preferably a heterocyclyl group having 3 to 8 ring atoms (i.e., 3- to 8-membered heterocyclyl); more preferably a heterocyclyl group having 3 to 6 ring atoms (i.e., 3- to 6-membered heterocyclyl); and most preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one monocyclic heterocyclyl group is contained and the point of attachment is on the monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl is preferably a spiroheterocyclyl group having 6 to 14 ring atoms (i.e., 6- to 14-membered spiroheterocyclyl), and more preferably a spiroheterocyclyl group having 7 to 11 ring atoms (i.e., 7- to 11-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include: 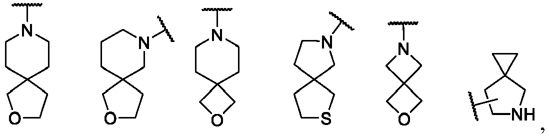 and the like.

The term "fused heterocyclyl" refers to a polycyclic heterocyclic system in which two adjacent atoms are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); which is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6- to 14-membered fused heterocyclyl), more preferably a fused heterocyclyl group having 7 to 10 ring atoms (i.e., 7- to 10-membered fused heterocyclyl), and further preferably a fused heterocyclyl group having 8 ring atoms (i.e., 8-membered fused heterocyclyl). The fused heterocyclyl includes bicyclic and polycyclic fused heterocyclyl (e.g., tricyclic fused heterocyclyl and tetracyclic fused heterocyclyl), preferably bicyclic fused heterocyclyl or tricyclic fused heterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include: 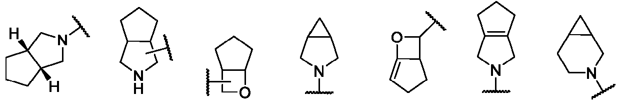 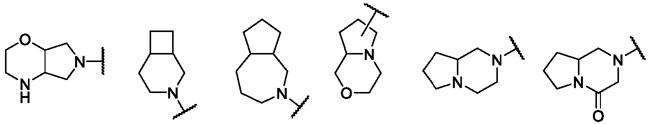 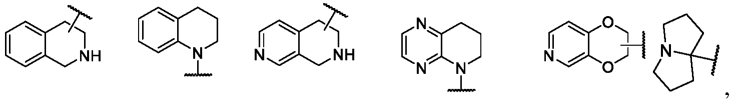 and the like.

The term "bridged heterocyclyl" refers to a polycyclic heterocyclic system in which two atoms that are not directly connected are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); and which has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), more preferably a bridged heterocyclyl group having 7 to 10 ring atoms (i.e., 7- to 10-membered bridged heterocyclyl), and further preferably a bridged heterocyclyl group having 7 to 8 ring atoms (i.e., 7- to 8-membered bridged heterocyclyl). According to the number of constituent rings, bridged heterocyclyl can be divided into bicyclic bridged heterocyclyl and polycyclic bridged heterocyclyl (e.g., tricyclic bridged heterocyclyl and tetracyclic bridged heterocyclyl), preferably bicyclic bridged heterocyclyl or tricyclic bridged heterocyclyl. Non-limiting examples include: 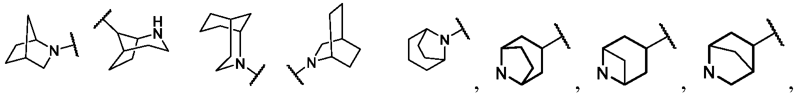 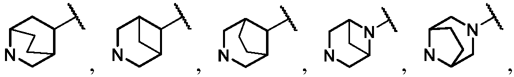 and the like.

Heterocyclyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). An example of the monocyclic aryl is phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include: 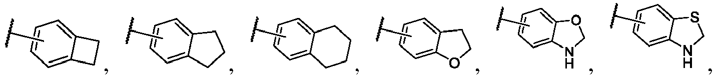 and the like.

Aryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl is preferably a heteroaryl group having 5 to 10 ring atoms (i.e., 5- to 10-membered heteroaryl), more preferably a monocyclic heteroaryl group having 5 or 6 ring atoms (i.e., 5- or 6-membered monocyclic heteroaryl) or a bicyclic heteroaryl group having 9 to 10 ring atoms (i.e., 9- to 10-membered bicyclic heteroaryl), and most preferably a 5- or 6-membered monocyclic heteroaryl group containing in the ring 1, 2, or 3 heteroatoms selected from the group consisting of nitrogen, oxygen, and sulfur or a 9- to 10-membered bicyclic heteroaryl group containing in the ring 1, 2, 3, or 4 heteroatoms selected from the group consisting of nitrogen, oxygen, and sulfur.

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridonyl, N-alkylpyridinone (e.g., 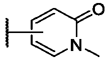 ), pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances the number of ring atoms continues to represent the number of ring atoms in the polycyclic heteroaromatic ring system. Non-limiting examples include: 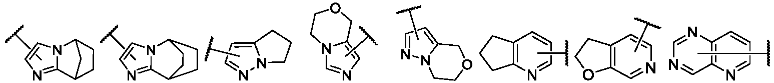 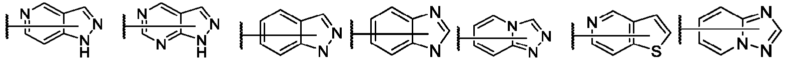 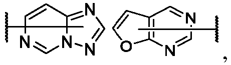 and the like.

Heteroaryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when other parts of the molecule are involved in reactions. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group so that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "aminoalkyl" refers to an alkyl group substituted with one or more amino groups, wherein the alkyl group is as defined above.

The term "haloalkoxy" refers to an alkoxy group substituted with one or more halogens, wherein the alkoxy group is as defined above.

The term "hydroxyalkyl" refers to an alkyl group substituted with one or more hydroxy groups, wherein the alkyl is as defined above.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)*-* and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, separation of enantiomers and diastereomers is generally accomplished by chromatography.

In the chemical structures of the compounds of the present disclosure, a bond " 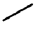 " represents an unspecified configuration; that is, if chiral isomers exist in the chemical structures, the bond " 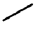 " may be " 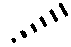 " or " 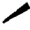 ", or both the configurations of " 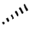 " and " 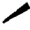 " are included simultaneously. For all carbon-carbon double bonds, both *Z*- and *E-*forms are included, even if only one configuration is named.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

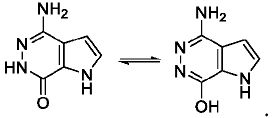

For example, reference to pyrazolyl is understood to include any one of the following two structures or a mixture of the two tautomers:

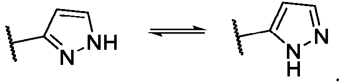

All tautomeric forms fall within the scope of the present disclosure, and the nomenclature of the compounds does not exclude any tautomer.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (deuterium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴1, ¹²⁵I, ¹²⁹I, and ¹³¹I; deuterium is preferred.

Compared to non-deuterated drugs, deuterated drugs have the advantages of reduced toxic and side effects, increased drug stability, enhanced efficacy, prolonged biological half-lives, and the like. All isotopic variations of the compounds of the present disclosure, whether radioactive or not, are intended to be included within the scope of the present disclosure. Each available hydrogen atom connected to a carbon atom may be independently replaced with a deuterium atom, wherein replacement of deuterium may be partial or complete, and replacement of partial deuterium refers to replacement of at least one hydrogen atom with at least one deuterium atom.

In the compounds of the present disclosure, when a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that halogen or cyano may, but does not necessarily, exist, and the description includes the instance where alkyl is substituted with halogen or cyano and the instance where alkyl is not substituted with halogen and cyano. "Substitution" or "substituted" means that one or more, preferably 1 to 6, and more preferably 1 to 3 hydrogen atoms in the group are independently substituted with a corresponding number of substituents. Those skilled in the art can determine (experimentally or theoretically) possible or impossible substitutions without undue effort. For example, it may be unstable when an amino or hydroxy having free hydrogen is bound to a carbon atom having an unsaturated (e.g., olefinic) bond.

"Pharmaceutical composition" refers to a mixture containing one or more of the compounds or the pharmaceutically acceptable salts thereof described herein, and other chemical components, and other components, for example, pharmaceutically acceptable carriers and excipients. The pharmaceutical composition is intended to promote administration to an organism and facilitate the absorption of the active ingredient so that it can exert its biological activity.

"Pharmaceutically acceptable salt" refers to a salt of the compound of the present disclosure, which may be selected from the group consisting of inorganic or organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the specific active substance used. The appropriate therapeutically effective amount in a case may be determined by those skilled in the art in the light of routine tests.

As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthesis Methods for Compounds of the Present Disclosure

To achieve the purposes of the present disclosure, the following technical solutions are adopted in the present disclosure:

### Scheme 1

A method for preparing the compound of general formula (II) or the pharmaceutically acceptable salt thereof of the present disclosure, comprising the following step: 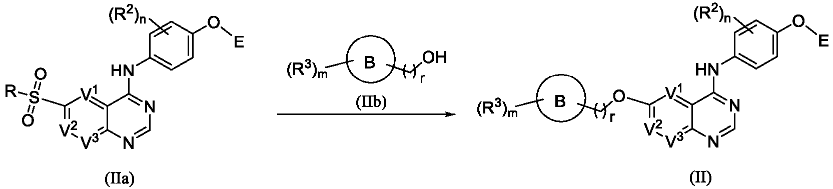 conducting a nucleophilic substitution reaction of a compound of general formula (IIa) or a salt thereof with a compound of general formula (IIb) or a salt thereof under alkaline conditions to give the compound of general formula (II) or the pharmaceutically acceptable salt thereof;
wherein:
R is C₁₋₆ alkyl; preferably, R is methyl;
E, ring B, R², R³, V¹, V², V³, r, m, and n are as defined in general formula (II).

### Scheme 2

A method for preparing the compound of general formula (III) or the pharmaceutically acceptable salt thereof of the present disclosure, comprising the following step: 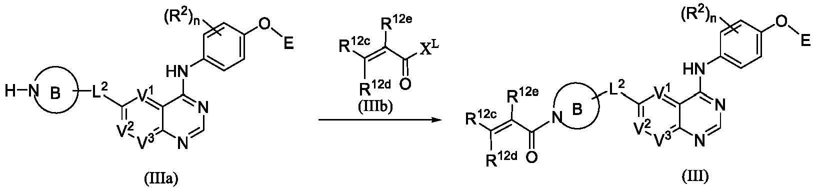 conducting a condensation reaction of a compound of general formula (IIIa) or a salt thereof with a compound of general formula (IIIb) or a salt thereof under alkaline conditions to give the compound of general formula (III) or the pharmaceutically acceptable salt thereof;
wherein:
X^{L} is halogen; preferably, X^{L} is chlorine;
E, ring B, R², L², V¹, V², V³, R^{12c}, R^{12d}, R^{12e}, and n are as defined in general formula (III). In the above synthesis schemes, the base includes organic bases and inorganic bases; the organic bases include, but are not limited to, triethylamine, N,N-diisopropylethylamine, n-butyllithium, lithium diisopropylamide, potassium acetate, sodium acetate, sodium ethoxide, sodium *tert*-butoxide, or potassium *tert*-butoxide; the inorganic bases include, but are not limited to, sodium hydride, potassium phosphate, sodium carbonate, potassium carbonate, anhydrous potassium carbonate, cesium carbonate, sodium hydroxide, lithium hydroxide monohydrate, lithium hydroxide, and potassium hydroxide; preferably, the base in Scheme 1 is potassium *tert*-butoxide; the base in Scheme 2 is *N,N-*diisopropylethylamine.

The above synthesis schemes are preferably carried out in solvents, including but not limited to: ethylene glycol dimethyl ether, acetic acid, methanol, ethanol, acetonitrile, n-butanol, toluene, tetrahydrofuran, dichloromethane, petroleum ether, ethyl acetate, *n-*hexane, dimethyl sulfoxide, 1,4-dioxane, water, *N,N*-dimethylformamide, *N,N-*dimethylacetamide, glacial acetic acid, and mixtures thereof.

### DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument or Bruker AVANCE NEO 500M, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS); Waters ACQuity UPLC-QD/SQD (manufacturer: Waters; MS model: Waters ACQuity Qda Detector/Waters SQ Detector); and THERMO Ultimate 3000-Q Exactive (manufacturer: THERMO; MS model: THERMO Q Exactive). The high performance liquid chromatography (HPLC) analyses were performed on Agilent HPLC 1200DAD, Agilent HPLC 1200VWD, and Waters HPLC e2695-2489 high performance liquid chromatographs.

The chiral HPLC analyses were performed on an Agilent 1260 DAD high performance liquid chromatograph.

The preparative high performance liquid chromatography steps were performed on Waters 2545-2767, Waters 2767-SQ Detecor2, Shimadzu LC-20AP, and Gilson GX-281 preparative chromatographs.

The preparative chiral chromatography steps were performed on a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm-0.2 mm, and those used in the thin-layer chromatography separation and purification had a layer thickness of 0.4 mm-0.5 mm.

In the silica gel column chromatography steps, a 200-300 mesh silica gel (Huanghai, Yantai) was generally used as the carrier.

The kinase mean inhibition rates and IC₅₀ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, Accela ChemBio Co., Ltd., Chembee Chemicals, and WuXi AppTec Co. Ltd.

In the examples, the reactions can all be performed under an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 rounds of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples was conducted by thin-layer chromatography (TLC). The developing solvent for reactions, the eluent system for column chromatography purification and the developing solvent system for thin-layer chromatography included: A: dichloromethane/methanol system, B: *n*-hexane/ethyl acetate system, and C: petroleum ether/ethyl acetate system. The volume ratio of the solvents was adjusted according to the polarity of the compound, or by adding a small amount of basic or acidic reagents such as triethylamine and acetic acid.

### Example 1

### 1-(endo-3-((4-((4-([1,2,4]Triazolo[1,5-a]pyridin-7-yloxy)-3-methylphenyl)amino)quinazolin-6-yl)oxy)-8-azabicyclo[3.2.1]octan-8-yl)prop-2-en-1-one 1

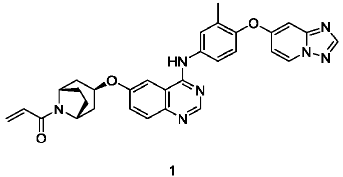

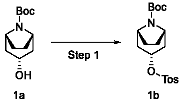

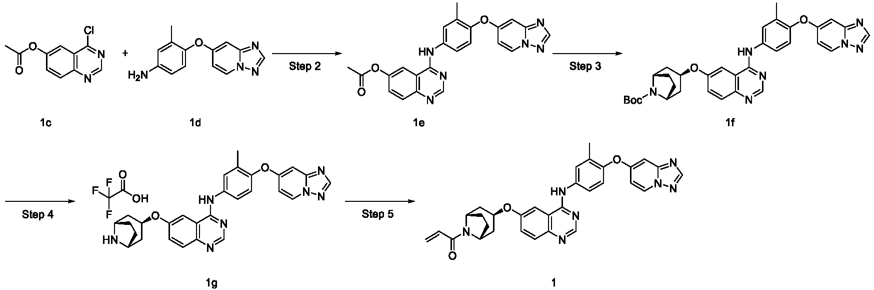

### Step 1

### tert-Butyl exo-3-(p-toluenesulfonyloxy)-8-azabicyclo[3.2.1]octane-8-carboxylate 1b

*tert*-Butyl *exo*-3-hydroxy-8-azabicyclo[3.2.1]octane-8-carboxylate **1a** (1 g, 4.4 mmol, Shanghai Accela ChemBio Co., Ltd.), *p*-toluenesulfonyl chloride (1 g, 5.24 mmol), and 4-dimethylaminopyridine (542 mg, 4.4 mmol) were dissolved in dichloromethane (20 mL), and *N,N*-diisopropylethylamine (853 mg, 6.6 mmol) was added. The mixture was stirred for 16 h. The reaction solution was concentrated under reduced pressure, and the residue was purified by silica gel column chromatography with eluent system B to give the title compound **1b** (1 g, yield: 59.5%).

### Step 2

### 4-((4-([1,2,4]Triazolo[1,5-a]pyridin-7-yloxy)-3-methylphenyl)amino)quinazolin-6-yl acetate 1e

4-Chloroquinazolin-6-yl acetate **1c** (200 mg, 898 µmol, prepared by the method disclosed in the document *"*Bioorganic & Medicinal Chemistry Letters, 2009, 19(19): 5531-5538") was dissolved in isopropanol (5 mL), and 4-([1,2,4]triazolo[1,5-*a*]pyridin-7-yloxy)-3-methylaniline **1d** (216 mg, 898 µmol, prepared by the method disclosed in method 1 on page 33 of the specification in the patent application "WO2021156180A1") was added. The mixture was reacted at 80 °C for 2 h. The reaction solution was concentrated under reduced pressure, and the residue was purified by silica gel column chromatography with eluent system A to give the title compound **1e** (200 mg, yield: 52.2%). MS m/z (ESI): 427.2 [M+1].

### Step 3

### tert-Butyl endo-3-((4-((4-([1,2,4]triazolo[1,5-a]pyridin-7-yloxy)-3-methylphenyl)amino)quinazolin-6-yl)oxy)-8-azabicyclo[3.2.1]octane-8-carboxylate 1f

Compound **1e** (200 mg, 469 µmol) was dissolved in *N,N*-dimethylformamide (15 mL), and compound **1b** (197 mg, 516 µmol) and cesium carbonate (229 mg, 703 µmol) were added. The mixture was reacted at 80 °C for 14 h. The reaction solution was concentrated under reduced pressure, and the residue was purified by silica gel column chromatography with eluent system A to give the title compound **1f** (150 mg, yield: 53.8%). MS m/z (ESI): 594.2 [M+1].

### Step 4

### 6-((endo-8-Azabicyclo[3.2.1]octan-3-yl)oxy)-N-(4-([1,2,4]triazolo[1,5-a]pyridin-7-yloxy)-3-methylphenyl)quinazolin-4-amine 2,2,2-trifluoroacetate 1g

Compound **1f** (100 mg, 168 µmol) was dissolved in dichloromethane (2 mL), and trifluoroacetic acid (1 mL) was added at 0 °C. The mixture was stirred for 2 h. The reaction solution was concentrated under reduced pressure to give a crude product of the title compound **1g** (100 mg). The product was directly used in the next step without purification.

MS m/z (ESI): 494.2 [M+1].

### Step 5

### 1-(endo-3-((4-((4-([1,2,4]Triazolo[1,5-a]pyridin-7-yloxy)-3-methylphenyl)amino)quinazolin-6-yl)oxy)-8-azabicyclo[3.2.1]octan-8-yl)prop-2-en-1-one 1

The crude product of compound **1g** (100 mg, 168 µmol) was dissolved in dichloromethane (5 mL), *N,N-*diisopropylethylamine (42 mg, 329 µmol) was added, and acryloyl chloride (18 mg, 197 µmol) was added under an ice bath. The mixture was stirred for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **1** (15 mg, yield: 16.6%).

MS m/z (ESI): 548.2 [M+1].

¹H NMR (500 MHz, CDCl₃): δ 8.72 (s, 1H), 8.61 (s, 1H), 8.52 (d, 1H), 8.24 (s, 1H), 7.90 (d, 1H), 7.70 (s, 1H), 7.67 (d, 1H), 7.63 (d, 1H), 7.44 (d, 1H), 7.08 (d, 1H), 6.93 (d, 1H), 6.83 (s, 1H), 6.58-6.53 (m, 1H), 6.41-6.37 (m, 1H), 5.74 (d, 1H), 4.90-4.88 (m, 1H), 4.83-4.81 (m, 1H), 4.44-4.42 (m, 1H), 2.42-2.40 (m, 1H), 2.37-2.31 (m, 3H), 2.29 (s, 3H), 2.28-2.26 (m, 2H), 2.09-2.02 (m, 2H).

### Example 2

### 1-(endo-3-((4-((3-Methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 2

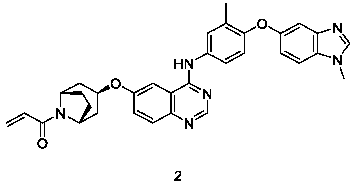

The title compound **2** (10 mg, 11%) was obtained by following the synthetic route in Example 1 with the starting material compound **1d** in step 2 replaced by 3-methyl-4-((1-methyl-1H-benzo[d]imidazol-5-yl)oxy)aniline (prepared by the method disclosed in method 1 on page 34 of the specification in the patent application "WO2021156178A1"). MS m/z (ESI): 561.2 [M+1].

¹H NMR (500 MHz, CDCl₃): δ 8.68 (s, 1H), 8.00 (s, 1H), 7.90-7.87 (m, 2H), 7.54 (s, 1H), 7.42-7.35 (m, 5H), 7.11 (d, 1H), 6.89 (d, 1H), 6.56-6.51 (m, 1H), 6.42-6.39 (m, 1H), 5.74 (d, 1H), 4.83-4.78 (m, 2H), 4.39 (s, 1H), 3.87 (s, 3H), 2.38-2.36 (m, 1H), 2.34 (s, 3H), 2.30-2.27 (m, 3H), 2.24-2.08 (m, 4H).

### Example 3

### 6-(((2S,7aR)-2-Fluorotetrahydro-1H-pyrrolizin-7a(5H)-yl)methoxy)-N-(3-methyl-4-((1-methyl-1H-benzo[d]imidazol-5-yl)oxy)phenyl)pyrimido[5,4-d]pyrimidin-4-amine 3

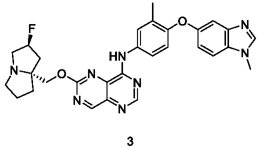

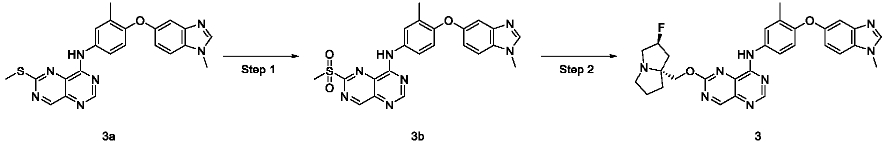

### Step 1

### N-(3-Methyl-4-((1-methyl-1H-benzo[d]imidazol-5-yl)oxy)phenyl)-6-(methanesulfonyl)pyrimido[5,4-d]pyrimidin-4-amine 3b

*N*-(3-Methyl-4-((1-methyl-1*H*-benzo[d]imidazol-5-yl)oxy)phenyl)-6-(methylthio)pyrimido[5,4-*d*]pyrimidin-4-amine **3a** (1 g, 3.32 mmol, prepared by the method disclosed in Compound J on page 40 of the specification in the patent application "WO2021156178A1") was dissolved in dichloromethane (25 mL), and m-chloroperoxybenzoic acid (482 mg, 2.8 mmol) was added. The mixture was stirred for 0.5 h. The reaction solution was quenched by adding a saturated aqueous sodium bicarbonate solution and extracted with dichloromethane (20 mL × 3). The organic phases were combined, dried over anhydrous sodium sulfate, and filtered to remove the drying agent, and the filtrate was concentrated under reduced pressure to give a crude product of the title compound **3b** (650 mg). The product was directly used in the next step without purification.

MS m/z (ESI): 462.1 [M+1].

### Step 2

### 6-(((2S,7aR)-2-Fluorotetrahydro-1H-pyrrolizin-7a(5H)-yl)methoxy)-N-(3-methyl-4-((1-methyl-1H-benzo[d]imidazol-5-yl)oxy)phenyl)pyrimido[5,4-d]pyrimidin-4-amine 3

The crude product of compound **3b** (35 mg, 75.8 µmol) and ((2*R*,7*aS*)-2-fluorotetrahydro-1*H*-pyrrolizin-7*a*(5*H*)-yl)methanol (15 mg, 94 µmol, WuXi AppTec Co. Ltd.) were dissolved in dichloromethane (2 mL), and potassium *tert*-butoxide (16 mg, 142 µmol) was added. The mixture was stirred at 60 °C for 1 h. Water was added to the reaction solution, and the mixture was extracted with dichloromethane (5 mL × 3). The organic phases were combined, dried over anhydrous sodium sulfate, and filtered to remove the drying agent, and the filtrate was concentrated under reduced pressure. The residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **3** (5.3 mg, yield: 10.4%).

MS m/z (ESI): 541.1 [M+1].

¹H NMR (500 MHz, CDCl₃): δ 9.28 (s, 1H), 8.73 (s, 1H), 8.59 (s, 1H), 7.88 (s, 1H), 7.77 (s, 1H), 7.69 (d, 1H), 7.38-7.34 (m, 2H), 7.10 (d, 1H), 6.95 (d, 1H), 5.42-5.35 (m, 1H), 4.37 (d, 1H), 4.27 (d, 1H), 3.88 (s, 3H), 3.37-3.22 (m, 3H), 3.05 (s, 1H), 2.39 (s, 3H), 2.26-2.19 (m, 2H), 2.08-2.00 (m, 3H), 1.95 (s, 1H).

### Example 4

### 1-(endo-3-((7-Methoxy-4-((3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 4

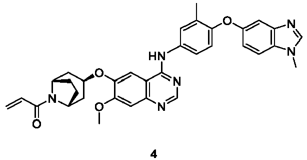

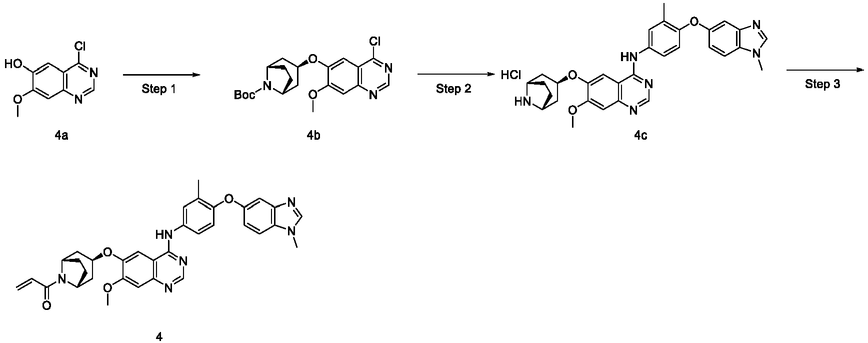

### Step 1

### tert-Butyl endo-3-((4-chloro-7-methoxyquinazolin-6-yl)oxy)-8-azabicyclo[3.2.1]octane-8-carboxylate 4b

4-Chloro-7-methoxyquinazolin-6-ol **4a** (140 mg, 664 µmol, Jiangsu Aikon Biopharmaceutical R&D Co., Ltd.), compound **1a** (181 mg, 797 µmol), and triphenylphosphine (262 mg, 997 µmol) were dissolved in tetrahydrofuran (5 mL), and diisopropyl azodicarboxylate (269 mg, 1.33 mmol, Shanghai Accela ChemBio Co., Ltd.) was added dropwise under an ice bath. The mixture was naturally warmed to room temperature and reacted for 16 h. The reaction solution was concentrated under reduced pressure, and the residue was purified by silica gel column chromatography with elution system C to give the title compound **4b** (270 mg, yield: 96%).

MS m/z (ESI): 420.2 [M+1].

### Step 2

### 6-((endo-8-Azabicyclo[3.2.1]octan-3-yl)oxy)-7-methoxy-N-(3-methyl-4-((1-methyl-1H-benzo[d]imidazol-5-yl)oxy)phenyl)quinazolin-4-amine hydrochloride 4c

Compound **4b** (60 mg, 142 µmol) and 3-methyl-4-((1-methyl-1*H*-benzo[d]imidazol-5-yl)oxy)aniline (36.2 mg, 142 µmol) were dissolved in isopropanol (5 mL), and 2 drops of a 4 M solution of hydrogen chloride in isopropanol were added. The mixture was reacted at 80 °C for 1 h. The reaction solution was concentrated under reduced pressure to give a crude product of the title compound **4c** (90 mg), which was directly used in the next step without purification.

MS m/z (ESI): 537.2 [M+1].

### Step 3

### 1-(endo-3-((7-Methoxy-4-((3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 4

The crude product of compound **4c** (76 mg, 140 µmol) was dissolved in dichloromethane (5 mL), *N,N-*diisopropylethylamine (55 mg, 424 µmol) was added, and acryloyl chloride (15 mg, 169 µmol) was added under an ice bath. The mixture was reacted for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **4** (10 mg, yield: 11.9%).

MS m/z (ESI): 591.2 [M+1].

¹H NMR (500 MHz, CDCl₃): δ 8.64 (s, 1H), 7.96 (s, 1H), 7.86 (s, 1H), 7.50 (s, 1H), 7.41-7.31 (m, 4H), 7.09 (dd, 1H), 6.87 (d, 1H), 6.53 (dd, 1H), 6.39 (d, 1H), 5.74-5.68 (m, 1H), 4.82 (d, 1H), 4.78 (d, 1H), 4.38 (d, 1H),4.01 (s, 3H), 3.87 (s, 3H), 2.45 (d, 1H), 2.41-2.32 (m, 1H), 2.29 (s, 3H), 2.27-2.20 (m, 2H), 2.16 (d, 1H), 2.12 (d, 1H), 2.07-2.01 (m, 3H).

### Example 5

### 1-(endo-3-((4-((4-([1,2,4]Triazolo[1,5-a]pyridin-7-yloxy)-3-methylphenyl)amino)-7-methoxyquinazolin-6-yl)oxy)-8-azabicyclo[3.2.1]octan-8-yl)prop-2-en-1-one 5

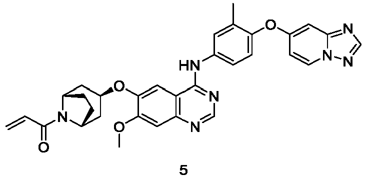

The title compound **5** (20 mg, 18.3%) was obtained by following the synthetic route in Example 4 with the starting material compound 3-methyl-4-((1-methyl-1*H*-benzo[*d*] imidazol-5-yl)oxy)aniline in step 2 replaced by compound **1d**.

MS m/z (ESI): 578.2 [M+1].

¹H NMR (500 MHz, CDCl₃): δ 8.69 (s, 1H), 8.51 (d, 1H), 8.24 (s, 1H), 8.16 (s, 1H), 7.68 (s, 1H), 7.65 (d, 1H), 7.44 (s, 1H), 7.30 (s, 1H), 7.08 (d, 1H), 6.95-6.89 (m, 1H), 6.86 (s, 1H), 6.56 (dd, 1H), 6.39 (d, 1H), 5.72 (d, 1H), 4.89 (s, 1H), 4.80 (d, 1H), 4.42 (s, 1H), 4.03 (s, 3H), 2.49 (d, 1H), 2.39 (t, 1H), 2.30 (d, 1H), 2.25 (d, 1H), 2.23 (s, 3H), 2.21-2.11 (m, 1H), 2.06 (d, 3H).

### Example 6

### 1-(exo-3-((4-((4-([1,2,4]Triazolo[1,5-a]pyridin-7-yloxy)-3-methylphenyl)amino)-7-methoxyquinazolin-6-yl)oxy)-8-azabicyclo[3.2.1]octan-8-yl)prop-2-en-1-one 6

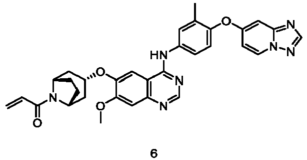

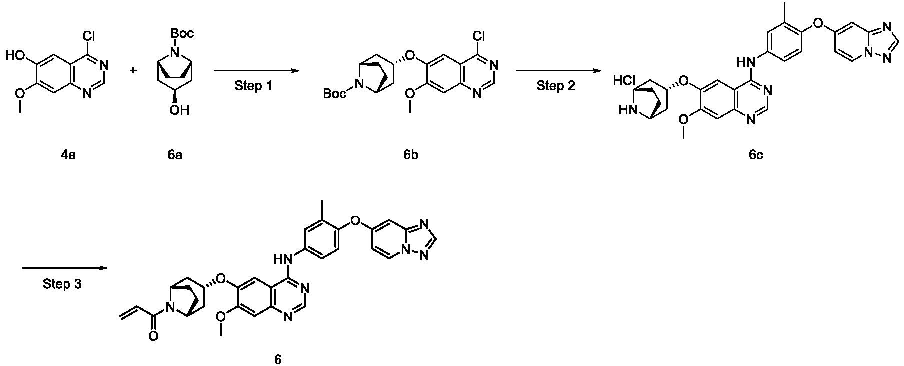

### Step 1

### tert-Butyl exo-3-((4-chloro-7-methoxyquinazolin-6-yl)oxy)-8-azabicyclo[3.2.1]octane-8-carboxylate 6b

Compound **4a** (100 mg, 474 µmol, Jiangsu Aikon Biopharmaceutical R&D Co., Ltd.), *tert*-butyl *endo*-3-hydroxy-8-azabicyclo[3.2.1]octane-8-carboxylate **6a** (130 mg, 570 µmol, Shanghai Bide Pharmatech Co., Ltd.), and triphenylphosphine (186 mg, 712 µmol) were dissolved in tetrahydrofuran (5 mL), and diisopropyl azodicarboxylate (192 mg, 949 µmol) was added dropwise under an ice bath. The mixture was naturally warmed to room temperature and reacted for 16 h. The reaction solution was concentrated under reduced pressure, and the residue was purified by silica gel column chromatography with elution system C to give the title compound **6b** (180 mg, yield: 90.3%).

MS m/z (ESI): 420.2 [M+1].

### Step 2

### 6-((exo-8-Azabicyclo[3.2.1]octan-3-yl)oxy)-N-(4-([1,2,4]triazolo[1,5-a]pyridin-7-yloxy)-3-methylphenyl)-7-methoxyquinazolin-4-amine hydrochloride 6c

Compound **6b** (180 mg, 428 µmol) and compound **1d** (103 mg, 428 µmol) were dissolved in isopropanol (3 mL), and a 4 M solution of hydrogen chloride in isopropanol (0.1 mL) was added. The mixture was reacted at 80 °C for 1 h. The reaction solution was concentrated under reduced pressure to give a crude product of the title compound **6c** (220 mg), which was directly used in the next step without purification.

MS m/z (ESI): 524.2 [M+1].

### Step 3

### 1-(exo-3-((4-((4-([1,2,4]Triazolo[1,5-a]pyridin-7-yloxy)-3-methylphenyl)amino)-7-methoxyquinazolin-6-yl)oxy)-8-azabicyclo[3.2.1]octan-8-yl)prop-2-en-1-one 6

The crude product of compound **6c** (220 mg, 393 µmol) was dissolved in dichloromethane (5 mL), *N,N-*diisopropylethylamine (152 mg, 1.2 mmol) was added, and acryloyl chloride (45.6 mg, 504 µmol) was added under an ice bath. The mixture was reacted for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **6** (100 mg, yield: 41.2%).

MS m/z (ESI): 578.2 [M+1].

¹H NMR (500 MHz, CDCl₃): δ 8.70 (s, 1H), 8.52 (d, 1H), 8.25 (s, 1H), 7.85 (s, 1H), 7.67 (d, 1H), 7.64-7.58 (m, 1H), 7.51 (s, 1H), 7.29 (s, 1H), 7.09 (d, 1H), 6.93 (dd, 1H), 6.82 (d, 1H), 6.55 (dd, 1H), 6.47-6.40 (m, 1H), 5.79-5.73 (m, 1H), 4.86 (d, 1H), 4.80 (dt, 1H), 4.47 (d, 1H), 4.00 (s, 3H), 2.31 (dd, 1H), 2.23 (s, 4H), 2.12 (dd, 1H), 2.02 (td, 2H), 1.91-1.78 (m, 2H), 1.72 (td, 1H).

### Example 7

### 1-(endo-3-((4-((4-([1,2,4]Triazolo[1,5-c]pyrimidin-7-yloxy)-3-methylphenyl)amino)quinazolin-6-yl)oxy)-8-azabicyclo[3.2.1]octan-8-yl)prop-2-en-1-one 7

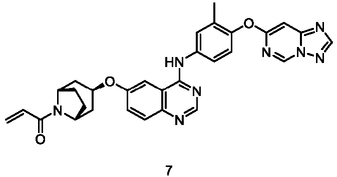

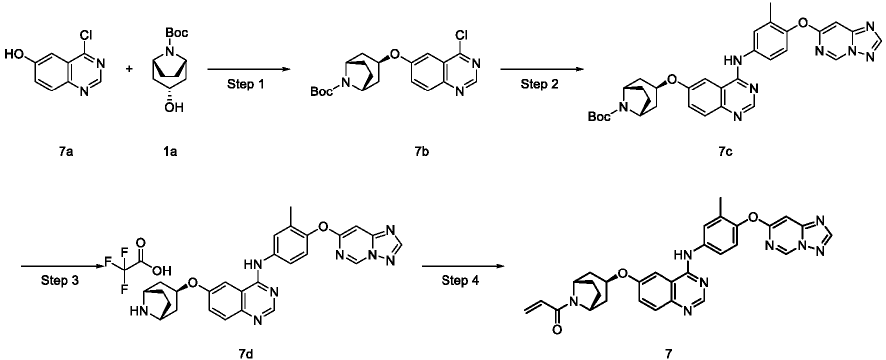

### Step 1

### tert-Butyl endo-3-((4-chloroquinazolin-6-yl)oxy)-8-azabicyclo[3.2.1]octane-8-carboxylate 7b

4-Chloroquinazolin-6-ol **7a** (20 mg, 110 µmol, prepared by the method disclosed in the document *"*European Journal of Medicinal Chemistry, 2018, vol. 147, p. 130-149"), compound **1a** (30 mg, 133 µmol), and triphenylphosphine (58 mg, 221 µmol) were dissolved in tetrahydrofuran (5 mL), and diisopropyl azodicarboxylate (34 mg, 166 µmol) was added dropwise under an ice bath. The mixture was naturally warmed to room temperature and reacted for 16 h. The reaction solution was concentrated under reduced pressure, and the residue was purified by silica gel column chromatography with elution system C to give the title compound **7b** (40 mg, yield: 92.6%).

MS m/z (ESI: 390.2 [M+1].

### Step 2

### tert-Butyl endo-3-((4-((4-([1,2,4]triazolo[1,5-c]pyrimidin-7-yloxy)-3-methylphenyl)amino)quinazolin-6-yl)oxy)-8-azabicyclo[3.2.1]octane-8-carboxylate 7c

Compound **7b** (40 mg, 102 µmol) was dissolved in isopropanol (2 mL), and 4-([1,2,4]triazolo[1,5-*c*]pyrimidin-7-yloxy)-3-methylaniline (25 mg, 102 µmol, prepared by the method disclosed in example 1 on page 95 of the specification in the patent application "WO2020057511A1") was added. The mixture was reacted at 80 °C for 1 h. The reaction solution was concentrated under reduced pressure to give a crude product of the title compound **7c** (60 mg). The product was directly used in the next step without purification.

MS m/z (ESI): 595.2 [M+1].

### Step 3

### 6-((endo-8-Azabicyclo[3.2.1]octan-3-yl)oxy)-N-(4-([1,2,4]triazolo[1,5-c]pyrimidin-7-yloxy)-3-methylphenyl)quinazolin-4-amine 2,2,2-trifluoroacetate 7d

The crude product of compound **7c** (60 mg, 101 µmol) was dissolved in dichloromethane (1 mL), and trifluoroacetic acid (0.5 mL) was added at 0 °C. The mixture was stirred for 2 h. The reaction solution was concentrated under reduced pressure to give a crude product of the title compound **7d** (62 mg). The product was directly used in the next step without purification.

MS m/z (ESI): 495.2 [M+1].

### Step 4

### 1-(endo-3-((4-((4-([1,2,4]Triazolo[1,5-c]pyrimidin-7-yloxy)-3-methylphenyl)amino)quinazolin-6-yl)oxy)-8-azabicyclo[3.2.1]octan-8-yl)prop-2-en-1-one 7

The crude product of compound **7d** (62 mg, 101 µmol) was dissolved in dichloromethane (2 mL), *N,N-*diisopropylethylamine (39 mg, 303 µmol) was added, and acryloyl chloride (14 mg, 152 µmol) was added under an ice bath. The mixture was stirred for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **7.**

MS m/z (ESI): 549.2 [M+1].

### Example 8

### 1-(exo-3-((4-((4-([1,2,4]Triazolo[1,5-c]pyrimidin-7-yloxy)-3-methylphenyl)amino)quinazolin-6-yl)oxy)-8-azabicyclo[3.2.1]octan-8-yl)prop-2-en-1-one 8

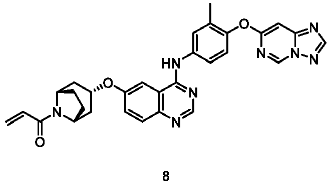

The title compound **8** (30 mg, yield: 27%) was obtained by following the synthetic route in Example 7 with the starting material compound **1a** in step 1 replaced by compound **6a.**

MS m/z (ESI): 549.2 [M+1].

¹H NMR (500 MHz, CDCl₃): δ 9.22 (s, 1H), 8.73 (s, 1H), 8.35 (s, 1H), 7.91 (d, 1H), 7.74 (s, 1H), 7.69 (d, 1H), 7.62 (s, 1H), 7.49 (d, 1H), 7.37 (s, 1H), 7.16 (d, 1H), 6.92 (s, 1H), 6.56 (dd, 1H), 6.46 (d, 1H), 5.78 (d, 1H), 4.93-4.86 (m, 2H), 4.50 (d, 1H), 2.35-2.31 (m, 1H), 2.28 (s, 3H), 2.26-2.23 (m, 1H), 2.19 (m, 1H), 2.09-2.04 (m, 1H), 2.01-1.90 (m, 2H), 1.85-1.77 (m, 2H).

### Example 9

### 1-(exo-3-((4-((4-([1,2,4]Triazolo[1,5-a]pyridin-7-yloxy)-3-methylphenyl)amino)quinazolin-6-yl)oxy)-8-azabicyclo[3.2.1]octan-8-yl)prop-2-en-1-one 9

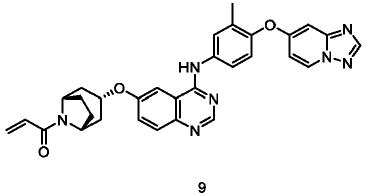

The title compound **9** (100 mg, yield: 44%) was obtained by following the synthetic route in Example 1 with the starting material compound **1a** in step 1 replaced by compound **6a.**

MS m/z (ESI): 548.2 [M+1].

¹H NMR (500 MHz, CDCl₃): δ 8.72 (s, 1H), 8.52 (d, 1H), 8.25 (s, 1H), 8.21 (s, 1H), 7.89 (d, 1H), 7.65 (d, 1H), 7.59 (dd, 2H), 7.46 (dd, 1H), 7.07 (d, 1H), 6.93 (dd, 1H), 6.78 (d, 1H), 6.55 (dd, 1H), 6.47-6.40 (m, 1H), 5.79-5.73 (m, 1H), 4.89 (dt, 2H), 4.47 (d, 1H), 2.36-2.28 (m, 1H), 2.21 (s, 4H), 2.14 (q, 1H), 2.05-1.92 (m, 2H), 1.91-1.83 (m, 1H), 1.82-1.75 (m, 2H).

### Example 10

### 1-(endo-3-((4-((4-([1,2,4]Triazolo[1,5-a]pyridin-7-yloxy)-2-fluoro-3-methylphenyl)amino)quinazolin-6-yl)oxy)-8-azabicyclo[3.2.1]octan-8-yl)prop-2-en-1-one 10

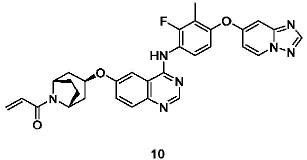

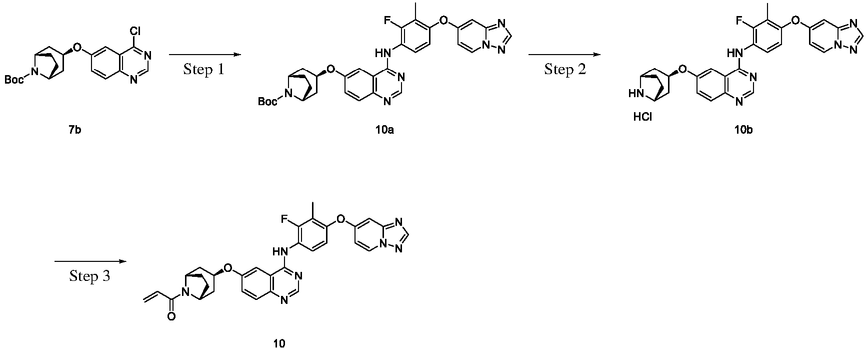

### Step 1

### tert-Butyl endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]octane-8-carboxylate 10a

Compound **7b** (1.2 g, 3 mmol) was dissolved in isopropanol (5 mL), and 4-([1,2,4]triazolo[1,5-*a*]pyridin-7-yloxy)-2-fluoro-3-methylaniline (845 mg, 3.27 mmol, prepared by the method disclosed in Example 95 on page 143 of the specification in the patent application "WO2022003575A1") was added. The mixture was reacted at 80 °C for 2 h. The reaction solution was concentrated under reduced pressure, and the residue was purified by silica gel column chromatography with eluent system A to give the title compound **10a** (1.05 g, yield: 55.7%).

MS m/z (ESI): 612.2 [M+1].

### Step 2

### 6-((endo-8-Azabicyclo[3.2.1]octan-3-yl)oxy)-N-(4-([1,2,4]triazolo[1,5-a]pyridin-7-yloxy)-2-fluoro-3-methylphenyl)quinazolin-4-amine hydrochloride 10b

Compound **10a** (1.05 g, 1.7 mmol) was dissolved in methanol (10 mL), and a 4 M solution of hydrogen chloride in 1,4-dioxane (0.1 mL) was added. The mixture was stirred for 1 h. The reaction solution was concentrated under reduced pressure to give a crude product of the title compound **10b** (940 mg). The crude product was directly used in the next step without purification.

MS m/z (ESI): 512.2 [M+1].

### Step 3

### 1-(endo-3-((4-((4-([1,2,4]Triazolo[1,5-a]pyridin-7-yloxy)-2-fluoro-3-methylphenyl)amino)quinazolin-6-yl)oxy)-8-azabicyclo[3.2.1]octan-8-yl)prop-2-en-1-one 10

The crude product of compound **10b** (250 mg, 488.7 µmol) was dissolved in dichloromethane (5 mL), *N,N*-diisopropylethylamine (126 mg, 974 µmol) was added, and acryloyl chloride (50 mg, 552.4 µmol) was added under an ice bath. The mixture was stirred for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **10** (110 mg, yield: 39.7%).

MS m/z (ESI): 566.2 [M+1].

¹H NMR (500 MHz, MeOD): δ 8.80-8.78 (d, 1H), 8.41 (s, 1H), 8.33 (s, 1H), 7.84-7.82 (m, 1H), 7.77 (s, 1H), 7.60-7.56 (m, 2H), 7.14-7.10 (m, 2H), 7.02-7.01 (m, 1H), 6.79-6.74 (m, 1H), 6.35 (dd, 1H), 5.81 (dd, 1H), 4.97-4.93 (m, 1H), 4.76-4.73 (m, 1H), 4.61-4.59 (m, 1H), 2.44-2.17 (m, 10H), 2.06-2.02 (m, 1H).

### Example 11

### 1-(endo-3-((4-((4-([1,2,4]Triazolo[1,5-c]pyrimidin-7-yloxy)-3-methylphenyl)amino)-7-methoxyquinazolin-6-yl)oxy)-8-azabicyclo[3.2.1]octan-8-yl)prop-2-en-1-one 11

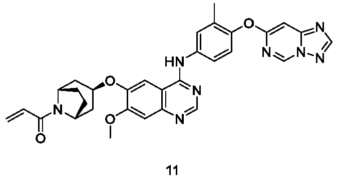

The title compound **11** (6 mg, yield: 15.5%) was obtained by following the synthetic route in Example 4 with the starting material 3-methyl-4-((1-methyl-1*H*-benzo[*d*]imidazol-5-yl)oxy)aniline in step 2 replaced by 4-([1,2,4]triazolo[1,5-c]pyrimidin-7-yloxy)-3-methylaniline.

MS m/z (ESI): 579.2 [M+1].

¹H NMR (500 MHz, CDCl₃): δ 9.22 (s, 1H), 8.69 (s, 1H), 8.35 (s, 1H), 8.10 (s, 1H), 7.70 (s, 1H), 7.68 (d, 1H), 7.39 (s, 1H), 7.31 (s, 1H), 7.12 (d, 1H), 6.89 (s, 1H), 6.56 (dd, 1H), 6.46-6.34 (m, 1H), 5.73 (d, 1H), 4.86 (s, 1H), 4.84-4.77 (m, 1H), 4.42 (s, 2H), 4.03 (s, 3H), 2.52-2.47 (m, 1H), 2.40-2.35 (m, 1H), 2.31-2.26 (m, 1H), 2.25 (s, 3H), 2.23-2.12 (m, 2H), 2.08-2.03 (m, 2H).

### Example 12

### 1-(exo-3-((4-((4-([1,2,4]Triazolo[1,5-c]pyrimidin-7-yloxy)-3-methylphenyl)amino)-7-methoxyquinazolin-6-yl)oxy)-8-azabicyclo[3.2.1]octan-8-yl)prop-2-en-1-one 12

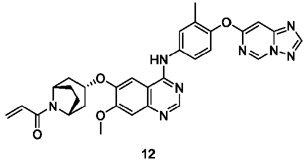

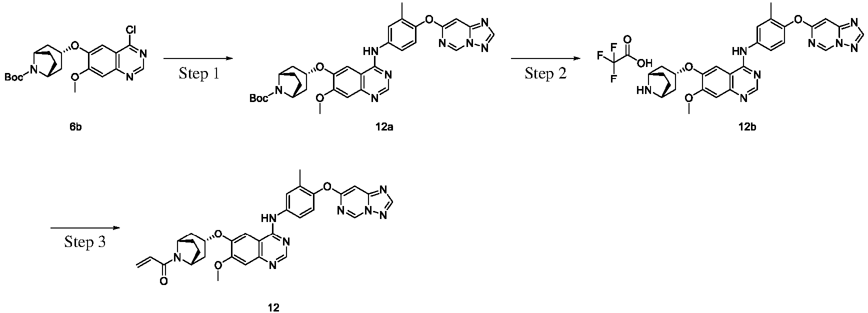

### Step 1

### tert-Butyl exo-3-((4-((4-([1,2,4]triazolo[1,5-c]pyrimidin-7-yloxy)-3-methylphenyl)amino)-7-methoxyquinazolin-6-yl)oxy)-8-azabicyclo[3.2.1]octane-8-carboxylate 12a

Compound **6b** (30 mg, 71 µmol) and 4-([1,2,4]triazolo[1,5-c]pyrimidin-7-yloxy)-3-methylaniline (20 mg, 83 µmol) were dissolved in isopropanol (4 mL). The mixture was reacted at 80 °C for 14 h. The reaction solution was concentrated under reduced pressure to give a crude product of the title compound **12a** (44 mg). The crude product was directly used in the next step without purification.

MS m/z (ESI): 625.2 [M+1].

### Step 2

### 6-(exo-8-Azabicyclo[3.2.1]octan-3-yl)oxy)-N-(4-([1,2,4]triazolo[1,5-c]pyrimidin-7-yloxy)-3-methylphenyl)-7-methoxyquinazolin-4-amine 2,2,2-trifluoroacetate 12b

The crude product of compound **12a** (40 mg, 64 µmol) was dissolved in dichloromethane (1 mL), and trifluoroacetic acid (0.5 mL) was added under an ice bath. The mixture was naturally warmed to room temperature and stirred for 2 h. The reaction solution was concentrated under reduced pressure to give a crude product of the title compound **12b** (40 mg). The product was directly used in the next step without purification.

MS m/z (ESI): 525.2 [M+1].

### Step 3

### 1-(exo-3-((4-((4-([1,2,4]Triazolo[1,5-c]pyrimidin-7-yloxy)-3-methylphenyl)amino)-7-methoxyquinazolin-6-yl)oxy)-8-azabicyclo[3.2.1]octan-8-yl)prop-2-en-1-one 12

The crude product of compound **12b** (40 mg, 62 µmol) was dissolved in dichloromethane (2 mL), *N,N-*diisopropylethylamine (22 mg, 170 µmol) was added, and acryloyl chloride (5.6 mg, 62 µmol) was added under an ice bath. The mixture was stirred for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **12** (10 mg, yield: 30%).

MS m/z (ESI): 579.2 [M+1].

¹H NMR (500 MHz, CDCl₃): δ 9.22 (d, 1H), 8.70 (s, 1H), 8.35 (s, 1H), 7.71 (d, 1H), 7.66 (dd, 1H), 7.49 (d, 1H), 7.37 (s, 1H), 7.16 (d, 1H), 6.92 (d, 1H), 6.56 (dd, 1H), 6.44 (dd, 1H), 5.77 (dd, 1H), 4.88 (d, 1H), 4.80 (tt, 1H), 4.56-4.42 (m, 1H), 4.02 (s, 3H), 2.32-2.29 (m, 1H), 2.28 (s, 3H), 2.27-2.10 (m, 2H), 2.04 (t, 3H), 1.86 (q, 3H).

### Example 13

### 1-(exo-3-((4-((4-([1,2,4]Triazolo[1,5-a]pyridin-7-yloxy)-2-fluoro-3-methylphenyl)amino)-7-methoxyquinazolin-6-yl)oxy)-8-azabicyclo[3.2.1]octan-8-yl)prop-2-en-1-one 13

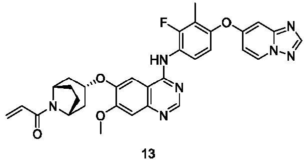

The title compound **13** (15 mg, 36%) was obtained by following the synthetic route in Example 12 with the starting material 4-([1,2,4]triazolo[1,5-c]pyrimidin-7-yloxy)-3-methylaniline in step 1 replaced by 4-([1,2,4]triazolo[1,5-*a*]pyridin-7-yloxy)-2-fluoro-3-methylaniline.

MS m/z (ESI): 596.2 [M+1].

¹H NMR (500 MHz, CDCl₃): δ 8.73 (d, 1H), 8.54 (dd, 1H), 8.49 (d, 1H), 8.26 (d, 1H), 7.45 (s, 1H), 7.32 (d, 1H), 7.01 (d, 1H), 6.93 (dt, 1H), 6.88 (d, 1H), 6.62-6.34 (m, 2H), 5.77 (dt, 1H), 4.98-4.79 (m, 2H), 4.50 (s, 1H), 4.03 (d, 3H), 2.36 (d, 1H), 2.25 (s, 1H), 2.17 (s, 3H), 2.05 (d, 3H), 1.91 (s, 2H), 1.79 (t, 2H).

### Example 14

### 1-(exo-3-((7-Methoxy-4-((3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 14

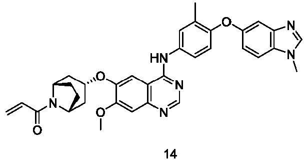

The title compound **14** (12 mg, yield: 29%) was obtained by following the synthetic route in Example 12 with the starting material 4-([1,2,4]triazolo[1,5-*c*]pyrimidin-7-yloxy)-3-methylaniline in step 1 replaced by 3-methyl-4-((1-methyl-1*H*-benzo[d]imidazol-5-yl)oxy)aniline.

MS m/z (ESI): 591.2 [M+1].

¹H NMR (500 MHz, CDCl₃): δ 8.63 (d, 1H), 8.37 (s, 1H), 7.83 (d, 1H), 7.69-7.64 (m, 1H), 7.45 (s, 1H), 7.37-7.33 (m, 1H), 7.31 (s, 1H), 7.24 (d, 1H), 7.18 (s, 1H), 7.13-7.07 (m, 1H), 6.84 (dt, 1H), 6.50 (dq, 1H), 6.40 (d, 1H), 5.76-5.70 (m, 1H), 4.77 (dd, 2H), 4.40 (s, 1H), 3.96 (d, 3H), 3.85 (d, 3H), 2.25 (d, 4H), 2.17 (dd, 2H), 2.07-1.97 (m, 2H), 1.80 (s, 1H), 1.72 (s, 1H), 1.61 (s, 1H).

### Example 15

### 1-(exo-3-((4-((2-Fluoro-3-methyl-4-((1-methyl-1H-benzo[d]imidazol-5-yl)oxy)phenyl)amino)-7-methoxyquinazolin-6-yl)oxy)-8-azabicyclo[3.2.1]octan-8-yl)prop-2-en-1-one 15

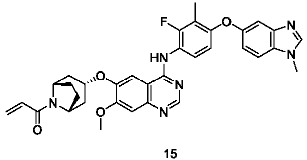

The title compound **15** (20 mg, yield: 45%) was obtained by following the synthetic route in Example 12 with the starting material 4-([1,2,4]triazolo[1,5-*c*]pyrimidin-7-yloxy)-3-methylaniline in step 1 replaced by 2-fluoro-3-methyl-4-((1-methyl-1*H-*benzo[*d*]imidazol-5-yl)oxy)aniline (prepared by the method disclosed in Intermediate Example G on page 75 of the specification in the patent application "WO2022003575A1").

MS m/z (ESI): 609.2 [M+1].

¹H NMR (500 MHz, MeOD): δ 8.33 (t, 1H), 8.16 (d, 1H), 7.80 (d, 1H), 7.60 (t, 1H), 7.33 (s, 1H), 7.27 (d, 1H), 7.20 (d, 1H), 7.15 (d, 1H), 6.82-6.70 (m, 2H), 6.36 (d, 1H), 5.81 (d, 1H), 5.08 (s, 2H), 4.65 (s, 1H), 4.07-3.90 (m, 6H), 2.42 (s, 1H), 2.34 (s, 1H), 2.30 (s, 3H), 2.06 (d, 4H), 1.89 (d, 1H), 1.76 (s, 1H).

### Example 16

### 1-(endo-3-((4-((4-([1,2,4]Triazolo[1,5-a]pyridin-7-yloxy)-3-methylphenyl)amino)pyrido[3,4-d]pyrimidin-6-yl)oxy)-8-azabicyclo[3.2.1]octan-8-yl)prop-2-en-1-one 16

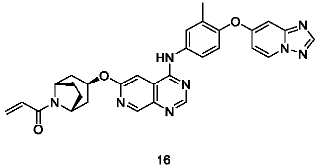

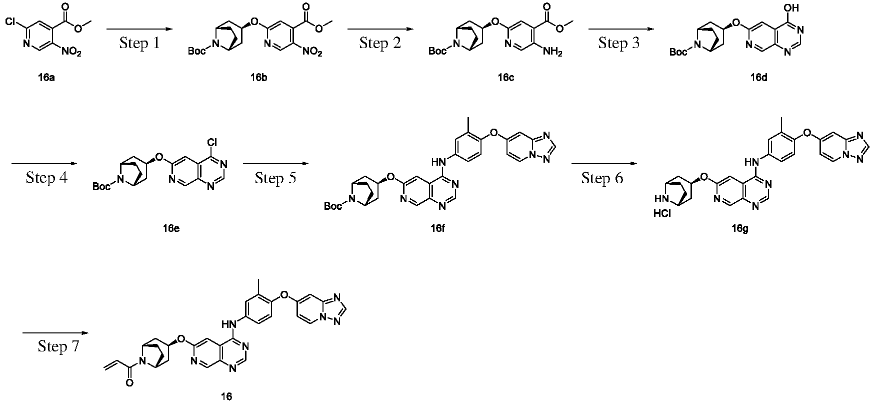

### Step 1

### tert-Butyl endo-3-((4-(methoxycarbonyl)-5-nitropyridin-2-yl)oxy)-8-azabicyclo[3.2.1]octane-8-carboxylate 16b

Compound **6a** (1 g, 4.6 mmol) was dissolved in *N,N*-dimethylformamide (20 mL), and sodium hydride (212 mg, 5.54 mmol, 60% purity) was added under an ice bath. The mixture was reacted for 20 min with the temperature maintained, and then methyl 2-chloro-5-nitroisonicotinate **16a** (1.26 g, 5.54 mmol, Shanghai Bide Pharmatech Co., Ltd.) was added. The mixture was reacted at 35 °C for 30 min and then stirred at room temperature for another 16 h. Water was added to the reaction solution, and the mixture was extracted with dichloromethane (30 mL × 3). The organic phases were combined, dried over anhydrous sodium sulfate, and filtered to remove the drying agent, and the filtrate was concentrated under reduced pressure. The residue was purified by silica gel column chromatography with elution system C to give the title compound **16b** (600 mg, yield: 31.8%).

MS m/z (ESI):408.2 [M+1].

### Step 2

### tert-Butyl endo-3-((5-amino-4-(methoxycarbonyl)pyridin-2-yl)oxy)-8-azabicyclo[3.2.1]octane-8-carboxylate 16c

Compound **16b** (500 mg, 1.2 mmol) was dissolved in methanol (20 mL), and 10% palladium on carbon catalyst (wet) (50 mg) was added. The mixture was stirred for 14 h under hydrogen atmosphere. The reaction solution was filtered, and the filtrate was concentrated under reduced pressure to give a crude product of the title compound **16c** (360 mg). The product was directly used in the next step without purification.

MS m/z (ESI): 378.2 [M+1].

### Step 3

### tert-Butyl endo-3-((4-hydroxypyrido[3,4-d]pyrimidin-6-yl)oxy)-8-azabicyclo[3.2.1]octane-8-carboxylate 16d

The crude product of compound **16c** (200 mg, 529.9 µmol) was dissolved in ethanol (20 mL), and formamidine acetate (276 mg, 2.6 mmol, Shanghai Accela ChemBio Co., Ltd.) was added. The mixture was stirred at 80 °C for 14 h. The reaction solution was cooled to room temperature and then concentrated under reduced pressure. Water was added to the residue, and the mixture was extracted with dichloromethane (10 mL × 3). The organic phases were combined, dried over anhydrous sodium sulfate, and filtered to remove the drying agent, and the filtrate was concentrated under reduced pressure to give a crude product of the title compound **16d** (160 mg). The product was directly used in the next step without purification.

MS m/z (ESI): 373.2 [M+1].

### Step 4

### tert-Butyl endo-3-((4-chloropyrido[3,4-d]pyrimidin-6-yl)oxy)-8-azabicyclo[3.2.1]octane-8-carboxylate 16e

The crude product of compound **16d** (160 mg, 429.66 µmol) was dissolved in toluene (10 mL), and phosphorus oxychloride (329.4 mg, 2.15 mmol) and N,N-diisopropylethylamine (55.42 mg, 429.6 µmol) were added. The mixture was stirred at 80 °C for 2 h. The reaction solution was concentrated under reduced pressure, and the residue was dissolved in ethyl acetate and poured into ice water. The mixture was extracted with ethyl acetate (10 mL × 3). The organic phases were combined, dried over anhydrous sodium sulfate, and filtered to remove the drying agent, and the filtrate was concentrated under reduced pressure to give a crude product of the title compound **16e** (100 mg). The product was directly used in the next step without purification.

MS m/z (ESI): 391.1 [M+1].

### Step 5

### tert-Butyl endo-3-((4-((4-([1,2,4]triazolo[1,5-a]pyridin-7-yloxy)-3-methylphenyl)amino)pyrido[3,4-d]pyrimidin-6-yl)oxy)-8-azabicyclo[3.2.1]octane-8-carboxylate 16f

The crude product of compound **16e** (100 mg, 255.8 µmol) was dissolved in isopropanol (10 mL), and compound **1d** (61.5 mg, 255.8 µmol) was added. The mixture was stirred at 80 °C for 1 h. The reaction solution was concentrated under reduced pressure to give a crude product of the title compound **16f** (150 mg). The product was directly used in the next step without purification.

MS m/z (ESI): 595.2 [M+1].

### Step 6

### 6-((endo-8-Azabicyclo[3.2.1]octan-3-yl)oxy)-N-(4-([1,2,4]triazolo[1,5-a]pyridin-7-yloxy)-3-methylphenyl)pyrido[3,4-d]pyrimidin-4-amine hydrochloride 16g

The crude product of compound **16f** (150 mg, 252.2 µmol) was dissolved in dichloromethane (3 mL), and a 4 M solution of hydrogen chloride in 1,4-dioxane (1 mL) was added. The mixture was stirred for 1 h. The reaction solution was concentrated under reduced pressure to give a crude product of the title compound **16g** (130 mg). The crude product was directly used in the next step without purification.

MS m/z (ESI): 495.2 [M+1].

### Step 7

### 1-(endo-3-((4-((4-([1,2,4]Triazolo[1,5-a]pyridin-7-yloxy)-3-methylphenyl)amino)pyrido[3,4-d]pyrimidin-6-yl)oxy)-8-azabicyclo[3.2.1]octan-8-yl)prop-2-en-1-one 16

The crude product of compound **16g** (130 mg, 245 µmol) was dissolved in dichloromethane (5 mL), *N,N*-diisopropylethylamine (63 mg, 490 mmol) was added, and acryloyl chloride (24.4 mg, 269 µmol) was added under an ice bath. The mixture was reacted for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **16** (110 mg, yield: 39.7%).

MS m/z (ESI): 549.2 [M+1].

¹H NMR (500 MHz, DMSO-*d*₆): δ 9.97 (s, 1H), 8.97-8.91 (m, 2H), 8.58 (s, 1H), 8.39 (s, 1H), 7.92 (s, 2H), 7.90 (d, 1H), 7.25 (d, 1H), 7.04 (dd, 1H), 6.82 (d, 1H), 6.75 (dd, 2H), 6.20 (dd, 1H), 5.71 (dd, 1H), 5.45 (s, 1H), 4.60-4.55 (m, 2H), 2.32-2.24 (m, 2H), 2.22 (s, 3H), 2.18 (d, 2H), 2.04-1.91 (m, 3H).

### Example 17

### 1-(endo-3-((4-((4-([1,2,4]Triazolo[1,5-a]pyridin-7-yloxy)-2-fluoro-3-methylphenyl)amino)-7-ethoxyquinazolin-6-yl)oxy)-8-azabicyclo[3.2.1]octan-8-yl)prop-2-en-1-one 17

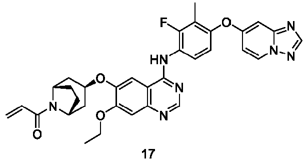

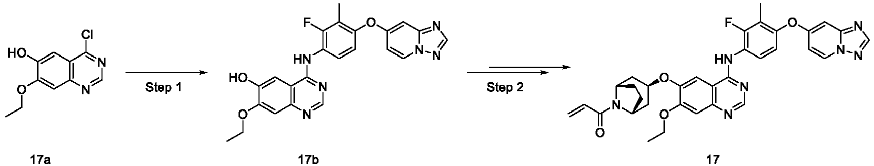

### Step 1

### 4-((4-([1,2,4]Triazolo[1,5-a]pyridin-7-yloxy)-2-fluoro-3-methylphenyl)amino)-7-ethoxyquinazolin-6-ol 17b

4-Chloro-7-ethoxyquinazolin-6-ol **17a** (140 mg, 623 µmol, prepared by the method disclosed in Example 3 on page 54 of the specification in the patent application "WO2021231400A1") was dissolved in isopropanol (2 mL), and 4-([1,2,4]triazolo[1,5-*a*]pyridin-7-yloxy)-2-fluoro-3-methylaniline (160 mg, 623 µmol) was added. The mixture was stirred at 110 °C for 14 h. The reaction solution was concentrated under reduced pressure, and the residue was purified by silica gel column chromatography with eluent system A to give the title compound **17b** (92 mg, yield: 33%).

MS m/z (ESI): 447.2 [M+1].

### Step 2

### 1-(endo-3-((4-((4-([1,2,4]Triazolo[1,5-a]pyridin-7-yloxy)-2-fluoro-3-methylphenyl)amino)-7-ethoxyquinazolin-6-yl)oxy)-8-azabicyclo[3.2.1]octan-8-yl)prop-2-en-1-one 17

The title compound **17** (1.6 mg, yield: 1.4%) was obtained by following the synthetic route from step 3 to step 5 in Example 1 with the starting material compound **1e** in step 3 replaced by compound **17b.**

MS m/z (ESI): 610.2 [M+1].

¹H NMR (500 MHz, DMSO-*d₆*): δ 9.43 (s, 1H), 8.98 (d, 1H), 8.42 (s, 1H), 8.38 (s, 1H), 7.81 (s, 1H), 7.49 (t, 1H), 7.21 (s, 1H), 7.12-7.08 (m, 1H), 6.95 (d, 1H), 6.79-6.74 (m, 1H), 6.66 (s, 1H), 6.19 (dd, 1H), 5.70 (dd, 1H), 4.89 (t, 1H), 4.55 (dd, 2H), 4.21 (q, 2H), 2.16 (d, 3H), 2.14-1.96 (m, 7H), 1.88 (t, 1H), 1.43 (t, 3H).

### Example 18

### 1-(endo-3-((4-((2-Fluoro-3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 18

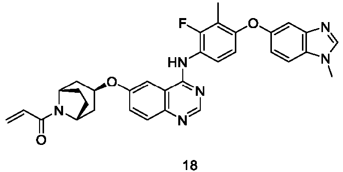

The title compound **18** (15 mg, yield: 25%) was obtained by following the synthetic route in Example 1 with the starting material compound **1d** in step 2 replaced by 2-fluoro-3-methyl-4-((1-methyl-1*H*-benzo[*d*]imidazol-5-yl)oxy)aniline.

MS m/z (ESI): 579.2 [M+1].

¹H NMR (500 MHz, CDCl₃): δ 8.70 (s, 1H), 8.09 (t, 1H), 7.96-7.79 (m, 2H), 7.49 (s, 1H), 7.44 (dd, 1H), 7.40-7.33 (m, 2H), 7.16 (d, 1H), 7.10 (dd, 1H), 6.73 (dd, 1H), 6.55 (dd, 1H), 6.44 (dd, 1H), 5.75 (dd, 1H), 4.86-4.81 (m, 2H), 4.41 (d, 1H), 3.88 (s, 3H), 2.39-2.34 (m, 2H), 2.30 (s, 3H), 2.29-2.21 (m, 2H), 2.19-2.14 (m, 2H), 2.10-1.99 (m, 2H).

### Example 19

### 1-(exo-3-((4-((2-Fluoro-3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 19

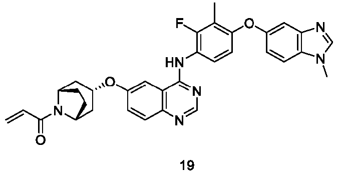

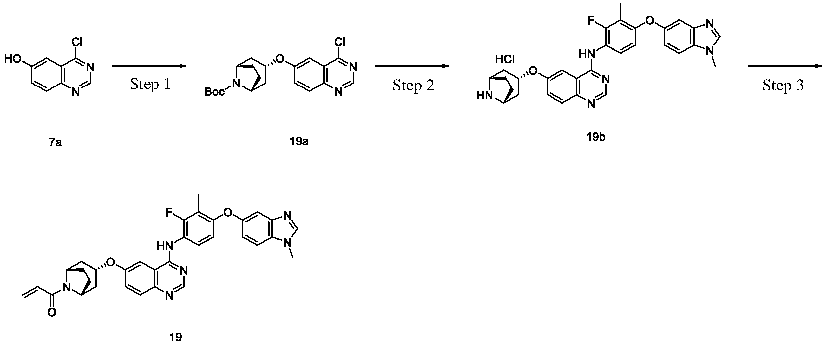

### Step 1

### 1-(exo-3-((4-Chloroquinazolin-6-yl)oxy)-8-azabicyclo[3.2.1]octan-8-yl)prop-2-en-1-one 19a

Compound **7a** (27 mg, 149 µmol), compound **6a** (34 mg, 149 µmol), and triphenylphosphine (80 mg, 305 µmol) were dissolved in tetrahydrofuran (5 mL), and diisopropyl azodicarboxylate (60 mg, 296 µmol) was added dropwise under an ice bath. The mixture was naturally warmed to room temperature and reacted for 16 h. The reaction solution was concentrated under reduced pressure, and the residue was purified by silica gel column chromatography with elution system C to give the title compound **19a** (34 mg, yield: 58.3%).

MS m/z (ESI): 390.2 [M+1].

### Step 2

### 6-((exo-8-Azabicyclo[3.2.1]octan-3-yl)oxy)-N-(2-fluoro-3-methyl-4-((1-methyl-1H-benzo[d]imidazol-5-yl)oxy)phenyl)quinazolin-4-amine hydrochloride 19b

Compound **19a** (34 mg, 87.2 µmol) and 2-fluoro-3-methyl-4-((1-methyl-1*H-*benzo[*d*]imidazol-5-yl)oxy)aniline (25 mg, 92.1 µmol) were dissolved in isopropanol (3 mL), and a 4 M solution of hydrochloric acid in isopropanol (0.1 mL) was added. The mixture was reacted at 80 °C for 1 h. The reaction solution was concentrated under reduced pressure to give a crude product of the title compound **19b** (45 mg). The crude product was directly used in the next step without purification.

MS m/z (ESI): 525.2 [M+1].

### Step 3

### 1-(exo-3-((4-((2-Fluoro-3-methyl-4-((1-methyl-1H-benzo[d]imidazol-5-yl)oxy)phenyl)amino)quinazolin-6-yl)oxy)-8-azabicyclo[3.2.1]octan-8-yl)prop-2-en-1-one 19

The crude product of compound **19b** (45 mg, 80.3 µmol) was dissolved in dichloromethane (3 mL), *N,N*-diisopropylethylamine (33 mg, 255.3 mmol) was added, and acryloyl chloride (10 mg, 110 µmol) was added under an ice bath. The mixture was reacted for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **19** (15 mg, yield: 30%).

MS m/z (ESI): 579.2 [M+1].

¹H NMR (500 MHz, CDCl₃): δ 8.70 (s, 1H), 8.17 (t, 1H), 7.96-7.77 (m, 2H), 7.54-7.44 (m, 2H), 7.41-7.33 (m, 2H), 7.10 (dd, 1H), 6.74 (dd, 1H), 6.55 (dd, 1H), 6.45 (dd, 1H), 5.77 (dd, 1H), 4.92 (tq, 2H), 4.65-4.36 (m, 1H), 3.88 (s, 3H), 2.42-2.32 (m, 1H), 2.31 (s, 3H), 2.30-2.14 (m, 3H), 2.13-2.06 (m, 1H), 2.05-1.88 (m, 2H), 1.88-1.76 (m, 2H).

### Example 20

### 1-(endo-3-((4-((4-([1,2,4]Triazolo[1,5-a]pyridin-7-yloxy)-2-fluoro-3-methylphenyl)amino)pyrido[3,4-d]pyrimidin-6-yl)oxy)-8-azabicyclo[3.2.1]octan-8-yl)prop-2-en-1-one 20

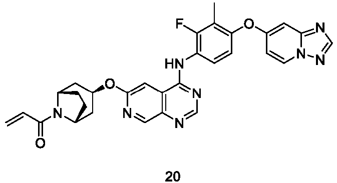

The title compound **20** (10 mg, yield: 6.4%) was obtained by following the synthetic route in Example 16 with the starting material compound **1d** in step 5 replaced by 4-([1,2,4]triazolo[1,5-*a*]pyridin-7-yloxy)-2-fluoro-3-methylaniline.

MS m/z (ESI): 567.2 [M+1].

¹H NMR (500 MHz, CDCl₃): δ 8.70 (s, 1H), 8.09 (t, 1H), 7.96-7.79 (m, 2H), 7.49 (s, 1H), 7.44 (dd, 1H), 7.40-7.33 (m, 2H), 7.16 (d, 1H), 7.10 (dd, 1H), 6.73 (dd, 1H), 6.55 (dd, 1H), 6.44 (dd, 1H), 5.75 (dd, 1H), 4.83 (dt, 2H), 4.41 (d, 1H), 2.39-2.34 (td, 2H), 2.30 (s, 3H), 2.28-2.23 (m, 1H), 2.19-2.14 (m, 2H), 2.08-2.02 (m, 2H).

### Example 21

### 1-(exo-3-((4-((4-([1,2,4]Triazolo[1,5-a]pyridin-7-yloxy)-2-fluoro-3-methylphenyl)amino)pyrido[3,4-d]pyrimidin-6-yl)oxy)-8-azabicyclo[3.2.1]octan-8-yl)prop-2-en-1-one 21

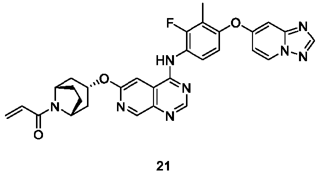

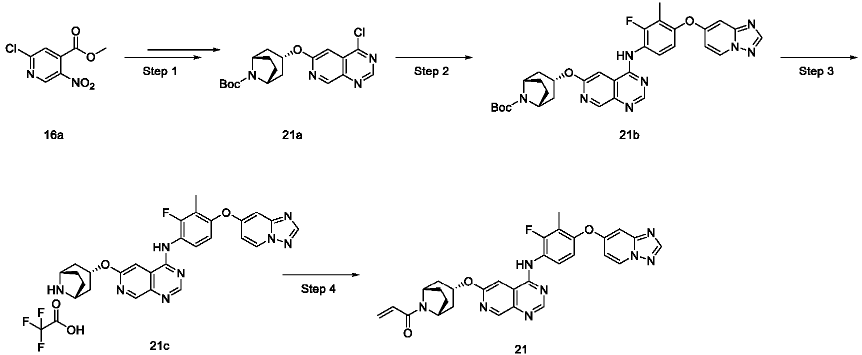

### Step 1

### tert-Butyl exo-3-((4-chloropyrido[3,4-d]pyrimidin-6-yl)oxy)-8-azabicyclo[3.2.1]octane-8-carboxylate 21a

The title compound **21a** (200 mg, yield: 21.2%) was obtained by following the synthetic route from step 1 to step 4 in Example 16 with the starting material compound **6a** in step 1 replaced by compound **1a.**

MS m/z (ESI): 391.2 [M+1].

### Step 2

### tert-Butyl exo-3-((4-((4-([1,2,4]triazolo[1,5-a]pyridin-7-yloxy)-2-fluoro-3-methylphenyl)amino)pyrido[3,4-d]pyrimidin-6-yl)oxy)-8-azabicyclo[3.2.1]octane-8-carboxylate 21b

Compound **21a** (15 mg, 38.3 µmol) was dissolved in isopropanol (2 mL), and 4-([1,2,4]triazolo[1,5-*a*]pyridin-7-yloxy)-2-fluoro-3-methylaniline (10 mg, 38.7 µmol) was added. The mixture was reacted at 80 °C for 1 h. The reaction solution was concentrated under reduced pressure to give a crude product of the title compound **21b** (23 mg). The product was directly used in the next step without purification.

MS m/z (ESI): 613.2 [M+1].

### Step 3

### 6-((exo-8-Azabicyclo[3.2.1]octan-3-yl)oxy)-N-(4-([1,2,4]triazolo[1,5-a]pyridin-7-yloxy)-2-fluoro-3-methylphenyl)pyrido[3,4-d]pyrimidin-4-amine 2,2,2-trifluoroacetate 21c

Compound **21b** (23 mg, 37.5 µmol) was dissolved in dichloromethane (3 mL), and 0.5 mL of trifluoroacetic acid (1 mL) was added. The mixture was stirred for 1 h. The reaction solution was concentrated under reduced pressure to give a crude product of the title compound **21c** (24 mg). The crude product was directly used in the next step without purification.

MS m/z (ESI): 513.2 [M+1].

### Step 4

### 1-(exo-3-((4-((4-([1,2,4]Triazolo[1,5-a]pyridin-7-yloxy)-2-fluoro-3-methylphenyl)amino)pyrido[3,4-d]pyrimidin-6-yl)oxy)-8-azabicyclo[3.2.1]octan-8-yl)prop-2-en-1-one 21

The crude product of compound **21c** (24 mg, 37 µmol) was dissolved in dichloromethane (2 mL), *N,N*-diisopropylethylamine (48 mg, 371 µmol) was added, and acryloyl chloride (3.5 mg, 38 µmol) was added under an ice bath. The mixture was reacted for 1 h. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **21** (15 mg, yield: 71%).

MS m/z (ESI): 567.2 [M+1].

¹H NMR (500 MHz, CDCl₃): δ 9.04 (s, 1H), 8.74 (s, 1H), 8.55 (dd, 2H), 8.26 (s, 1H), 7.69 (s, 1H), 7.06 (s, 1H), 7.02 (dd, 1H), 6.95-6.89 (m, 2H), 6.57 (dd, 1H), 6.44 (dd, 1H), 5.79-5.71 (m, 2H), 4.91 (d, 1H), 4.49 (s, 1H), 2.42 (s, 1H), 2.27 (s, 1H), 2.22 (d, 3H), 2.21-2.14 (m, 1H), 1.99 (h, 4H), 1.77 (t, 1H).

### Example 22

### 1-(endo-3-((8-((4-([1,2,4]Triazolo[1,5-a]pyridin-7-yloxy)-3-methylphenyl)amino)pyrimido[5,4-d]pyrimidin-2-yl)oxy)-8-azabicyclo[3.2.1]octan-8-yl)prop-2-en-1-one 22

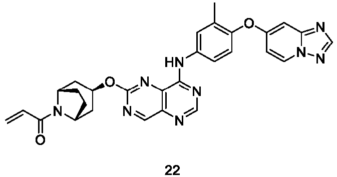

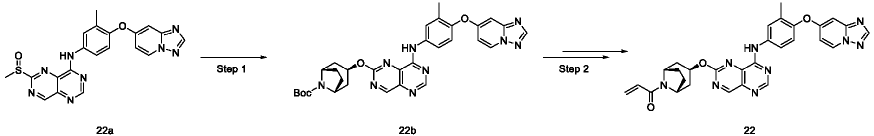

### Step 1

### tert-Butyl endo-3-((8-((4-([1,2,4]triazolo[1,5-a]pyridin-7-yloxy)-3-methylphenyl)amino)pyrimido[5,4-d]pyrimidin-2-yl)oxy)-8-azabicyclo[3.2.1]octane-8-carboxylate 22b

Compound **6a** (78.8 mg, 346 µmol) was dissolved in *N,N*-dimethylformamide (20 mL), and sodium hydride (18.5 mg, 462 µmol, 60% purity) was added under an ice bath. The mixture was reacted for 20 min with the temperature maintained, and then *N*-(4-([1,2,4]triazolo[1,5-*a*]pyridin-7-yloxy)-3-methylphenyl)-6-(methylsulfinyl)pyrimido[5,4-d]pyrimidin-4-amine 22a (100 mg, 231 µmol, prepared by the method disclosed in "Synthesis of Compound K" on page 38 of the specification in the patent application "WO2021156180A1") was added. The mixture was reacted at 60 °C for 1 h and then cooled to room temperature. The reaction solution was quenched by adding water and extracted with ethyl acetate (10 mL × 2). The organic phases were combined, dried over anhydrous sodium sulfate, and filtered to remove the drying agent, and the filtrate was concentrated under reduced pressure. The residue was purified by silica gel column chromatography with elution system A to give the title compound 22b (80 mg, yield: 58%).

MS m/z (ESI): 596.2 [M+1].

### Step 2

### 1-(endo-3-((8-((4-([1,2,4]Triazolo[1,5-a]pyridin-7-yloxy)-3-methylphenyl)amino)pyrimido[5,4-d]pyrimidin-2-yl)oxy)-8-azabicyclo[3.2.1]octan-8-yl)prop-2-en-1-one 22

The title compound **22** (15 mg, yield: 20%) was obtained by following the synthetic route from step 4 to step 5 in Example 1 with the starting material compound **1f** in step 4 replaced by compound **22b.**

MS m/z (ESI): 550.2 [M+1].

¹H NMR (500 MHz, DMSO-*d*₆): δ 9.81 (s, 1H), 9.36 (s, 1H), 8.95 (d, 1H), 8.65 (s, 1H), 8.39 (s, 1H), 7.98-7.91 (m, 2H), 7.26 (d, 1H), 7.04 (dd, 1H), 6.82-6.73 (m, 2H), 6.21 (dd, 1H), 5.80 (d, 1H), 5.72 (dd, 1H), 4.57 (d, 2H), 2.22 (s, 5H), 2.13-1.96 (m, 6H).

### Example 23

### 1-(exo-3-((4-((4-([1,2,4]Triazolo[1,5-a]pyridin-7-yloxy)-3-methylphenyl)amino)-8,9-dihydrofuro[2,3-h]quinazolin-6-yl)oxy)-8-azabicyclo[3.2.1]octan-8-yl)prop-2-en-1-one 23

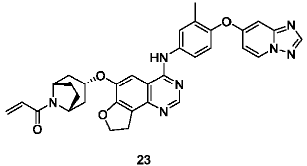

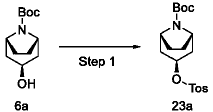

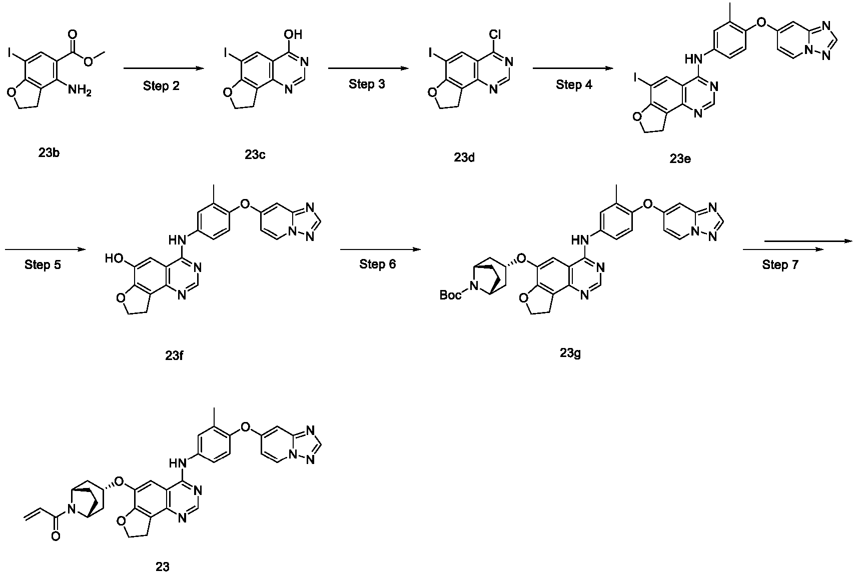

### Step 1

### tert-Butyl endo-3-(p-toluenesulfonyloxy)-8-azabicyclo[3.2.1]octane-8-carboxylate 23a

Compound **6a** (4 g, 17.6 mmol), *p*-toluenesulfonyl chloride (4 g, 21.1 mmol), and 4-dimethylaminopyridine (2.17 g, 17.6 mmol) were dissolved in dichloromethane (50 mL), and *N,N*-diisopropylethylamine (2.27 mg, 17.6 mmol) was added. The mixture was stirred for 16 h. The reaction solution was concentrated under reduced pressure, and the residue was purified by silica gel column chromatography with eluent system B to give the title compound **23a** (4.5 g, yield: 67%).

### Step 2

### 6-Iodo-8,9-dihydrofuro[2,3-h]quinazolin-4-ol 23c

Methyl 4-amino-7-iodo-2,3-dihydrobenzofuran-5-carboxylate **23b** (1 g, 3.13 mmol, prepared by the method disclosed in Example 11 on page 93 of the specification in the patent application "WO2021249475A1") was dissolved in ethanol (50 mL), and formamidine acetate (1.6 g, 15.3 mmol, Shanghai Accela ChemBio Co., Ltd.) was added. The mixture was stirred at reflux for 14 h. The reaction solution was cooled to room temperature and then concentrated under reduced pressure to remove most of the solvent. Water was added to the residue, and the mixture was stirred for 0.5 h and filtered. The filter cake was washed with water and dried to give a crude product of the title compound **23c** (1 g). The product was directly used in the next step without purification.

MS m/z (ESI): 315.2 [M+1].

### Step 3

### 4-Chloro-6-iodo-8,9-dihydrofuro[2,3-h]quinazoline 23d

The crude product of compound **23c** (500 mg, 1.59 mmol) was dissolved in phosphorus oxychloride (20 mL), and *N,N*-diisopropylethylamine (41 mg, 317 µmol) was added. The mixture was stirred at 100 °C for 2 h. The reaction solution was concentrated under reduced pressure, and the residue was dissolved in ethyl acetate and poured into ice water. The mixture was extracted with ethyl acetate (15 mL × 3). The organic phases were combined, dried over anhydrous sodium sulfate, and filtered to remove the drying agent, and the filtrate was concentrated under reduced pressure to give a crude product of the title compound **23d** (529 mg). The product was directly used in the next step without purification.

MS m/z (ESI): 333.1 [M+1].

### Step 4

### N-(4-([1,2,4]Triazolo[1,5-a]pyridin-7-yloxy)-3-methylphenyl)-6-iodo-8,9-dihydrofuro[2,3-h]quinazolin-4-amine 23e

The crude product of compound **23d** (300 mg, 902 µmol) was dissolved in isopropanol (20 mL), and compound **1d** (216 mg, 898 µmol) was added. The mixture was reacted at 80 °C for 2 h. The reaction solution was concentrated under reduced pressure, and the residue was purified by silica gel column chromatography with eluent system A to give the title compound **23e** (380 mg, yield: 78%).

MS m/z (ESI): 537.2 [M+1].

### Step 5

### 4-((4-([1,2,4]Triazolo[1,5-a]pyridin-7-yloxy)-3-methylphenyl)amino)-8,9-dihydrofuro[2,3-h]quinazolin-6-ol 23f

Compound **23e** (300 mg, 559.3 µmol) was dissolved in dimethyl sulfoxide (3 mL) and water (1 mL), and potassium hydroxide (94 mg, 1.67 mmol), copper acetylacetonate (15 mg, 57.3 µmol, J&K), and N1,N2-bis(4-hydroxy-2,6-dimethylphenyl)oxamide (18.4 mg, 56 µmol, Shanghai Bide Pharmatech Co., Ltd.) were added. The mixture was purged with nitrogen and reacted at 95 °C for 14 h. The reaction solution was concentrated under reduced pressure, and the residue was purified by silica gel column chromatography with eluent system A to give the title compound **23f** (60 mg, yield: 25%).

MS m/z (ESI): 427.2 [M+1].

### Step 6

### tert-Butyl exo-3-((4-((4-([1,2,4]triazolo[1,5-a]pyridin-7-yloxy)-3-methylphenyl)amino)-8,9-dihydrofuro[2,3-h]quinazolin-6-yl)oxy)-8-azabicyclo[3.2.1]octane-8-carboxylate 23g

Compound **23f** (60 mg, 140 µmol) was dissolved in *N,N*-dimethylformamide (10 mL), and compound **23b** (86 mg, 281 µmol) and cesium carbonate (82 mg, 281 µmol) were added. The mixture was reacted at 80 °C for 14 h. The reaction solution was concentrated under reduced pressure, and the residue was purified by silica gel column chromatography with eluent system A to give the title compound **23g** (75 mg, yield: 83%).

MS m/z (ESI): 636.2 [M+1].

### Step 7

### 1-(exo-3-((4-((4-([1,2,4]Triazolo[1,5-a]pyridin-7-yloxy)-3-methylphenyl)amino)-8,9-dihydrofuro[2,3-h]quinazolin-6-yl)oxy)-8-azabicyclo[3.2.1]octan-8-yl)prop-2-en-1-one 23

The title compound **23** (40 mg, yield: 60%) was obtained by following the synthetic route from step 2 to step 3 in Example 12 with the starting material compound **12a** in step 2 replaced by compound **23g.**

MS m/z (ESI): 590.2 [M+1].

¹H NMR (500 MHz, MeOD): δ 8.76 (t, 1H), 8.43 (s, 1H), 8.30 (s, 1H), 7.80 (d, 1H), 7.71 (s, 1H), 7.68-7.62 (m, 1H), 7.21 (d, 1H), 7.10 (dd, 1H), 6.86 (s, 1H), 6.76 (dq, 1H), 6.36 (d, 1H), 5.82 (d, 1H), 5.15 (s, 1H), 4.90 (s, 2H), 4.78 (s, 1H), 4.65 (d, 1H), 3.56 (m, 2H), 2.39 (s, 1H), 2.33 (d, 1H), 2.26 (d, 3H), 2.17 (s, 1H), 2.09-1.96 (m, 3H), 1.85 (t, 1H), 1.74 (t, 1H).

### Example 24

### 1-(endo-3-((4-((4-([1,2,4]Triazolo[1,5-a]pyridin-7-yloxy)-3-methylphenyl)amino)pyrido[3,2-d]pyrimidin-6-yl)oxy)-8-azabicyclo[3.2.1]octan-8-yl)prop-2-en-1-one 24

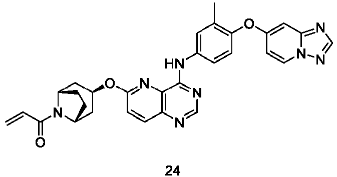

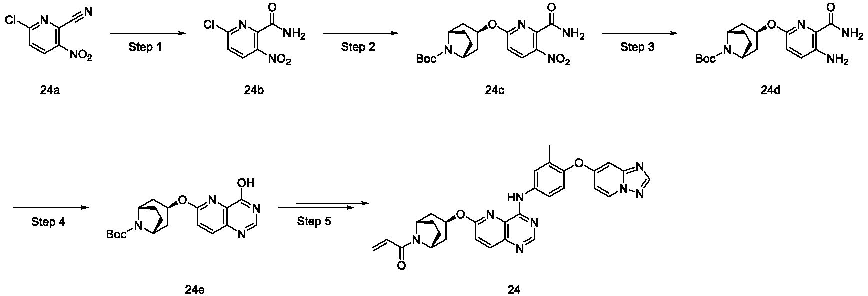

### Step 1

### 6-Chloro-3-nitropicolinamide 24b

6-Chloro-2-cyano-3-nitropyridine **24a** (5 g, 27.2 mmol, Shanghai Roye Electric Co., Ltd.) was added in batches to 90% sulfuric acid (50 mL). The mixture was stirred at 70 °C for 3 h. The reaction solution was cooled to room temperature and poured into ice water. A solid was precipitated, and the mixture was filtered. The filter cake was washed with water and dried to give a crude product of the title compound **24b** (3 g). The product was directly used in the next step without purification.

MS m/z (ESI): 202.2 [M+1].

### Step 2

### tert-Butyl endo-3-((6-carbamoyl-5-nitropyridin-2-yl)oxy)-8-azabicyclo[3.2.1]octane-8-carboxylate 24c

Compound **6a** (676.6 mg, 3 mmol) was dissolved in dimethyl sulfoxide (10 mL), and potassium *tert*-butoxide (835 mg, 7.4 mmol) was added under an ice bath. The mixture was naturally warmed to room temperature and stirred for 30 min, and then a solution of compound **24b** (500 mg, 2.5 mmol) in dimethyl sulfoxide (5 mL) was added. The mixture was stirred for 2 h. The reaction solution was quenched by adding water and extracted with ethyl acetate (10 mL × 3). The organic phases were combined, dried over anhydrous sodium sulfate, and filtered to remove the drying agent, and the filtrate was concentrated under reduced pressure. The residue was purified by silica gel column chromatography with elution system A to give the title compound **24c** (500 mg, yield: 51.3%).

MS m/z (ESI): 393.2 [M+1].

### Step 3

### tert-Butyl endo-3-((5-amino-6-carbamoylpyridin-2-yl)oxy)-8-azabicyclo[3.2.1]octane-8-carboxylate 24d

Compound **24c** (500 mg, 1.27 mmol) was dissolved in methanol (10 mL), and Raney nickel (150 mg) was added. The mixture was stirred for 12 h under hydrogen atmosphere. The reaction solution was filtered, and the filtrate was concentrated under reduced pressure to give a crude product of the title compound **24d** (320 mg). The product was directly used in the next step without purification.

MS m/z (ESI): 363.2 [M+1].

### Step 4

### tert-Butyl endo-3-((4-hydroxypyrido[3,2-d]pyrimidin-6-yl)oxy)-8-azabicyclo[3.2.1]octane-8-carboxylate 24e

The crude product of compound **24d** (320 mg, 882.9 µmol) was mixed with diethoxymethyl acetate (5 mL, J&K), and the mixture was stirred at 100 °C for 16 h. The reaction solution was cooled to room temperature and then concentrated under reduced pressure, and the residue was purified by silica gel column chromatography with elution system C to give the title compound **24e** (110 mg, yield: 33%).

MS m/z (ESI): 373.2 [M+1].

### Step 5

### 1-(endo-3-((4-((4-([1,2,4]Triazolo[1,5-a]pyridin-7-yloxy)-3-methylphenyl)amino)pyrido[3,2-d]pyrimidin-6-yl)oxy)-8-azabicyclo[3.2.1]octan-8-yl)prop-2-en-1-one 24

The title compound **24** (5 mg, yield: 10.8%) was obtained by following the synthetic route from step 4 to step 7 in Example 16 with the starting material compound **16d** in step 4 replaced by compound **24e.**

MS m/z (ESI): 549.2 [M+1].

¹H NMR (500 MHz, MeOD): δ 8.76 (d, 1H), 8.57 (s, 1H), 8.30 (s, 1H), 8.11 (d, 1H), 7.95-7.88 (m, 2H), 7.40 (d, 1H), 7.21 (d, 1H), 7.09 (dd, 1H), 6.83-6.73 (m, 2H), 6.35 (dd, 1H), 5.91 (t, 1H), 5.81 (dd, 1H), 5.36 (dd, 1H), 4.79-4.73 (m, 1H), 4.63-4.58 (m, 1H), 2.46-2.33 (m, 3H), 2.27 (s, 3H), 2.26-2.15 (m, 3H), 2.05 (q, 2H).

### Example 25

### 1-(endo-3-((8-((3-Methyl-4-((1-methyl-1H-benzo[d]imidazol-5-yl)oxy)phenyl)amino)pyrimido[5,4-d]pyrimidin-2-yl)oxy)-8-azabicyclo[3.2.1]octan-8-yl)prop-2-en-1-one 25

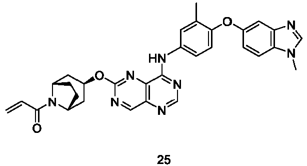

The title compound **25** (15 mg, yield: 20%) was obtained by following the synthetic route in Example 22 with the starting material compound **22a** in step 1 replaced by *N*-(3-methyl-4-((1-methyl-1*H*-benzo[*d*]imidazol-5-yl)oxy)phenyl)-6-(methylsulfinyl)pyrimido[5,4-d]pyrimidin-4-amine (prepared by the method disclosed in Compound K on page 41 of the specification in the patent application "WO2021156178A1").

MS m/z (ESI): 563.2 [M+1].

¹H NMR (500 MHz, MeOD): δ 9.25 (t, 1H), 8.58 (d, 1H), 8.14 (d, 1H), 7.83 (s, 1H), 7.73 (d, 1H), 7.60-7.54 (m, 1H), 7.15 (s, 1H), 7.12-7.08 (m, 1H), 6.92 (t, 1H), 6.77 (t, 1H), 6.35 (d, 1H), 5.84-5.75 (m, 2H), 4.75 (m, 1H), 4.60 (m, 1H), 3.93 (t, 3H), 2.44 (t, 3H), 2.33 (m, 3H), 2.28 (s, 1H), 2.24-2.16 (m, 3H), 2.05 (m, 1H).

### Example 26

### 1-(exo-3-((4-((4-([1,2,4]Triazolo[1,5-a]pyridin-7-yloxy)-3-methylphenyl)amino)pyrido[3,4-d]pyrimidin-6-yl)oxy)-8-azabicyclo[3.2.1]octan-8-yl)prop-2-en-1-one 26

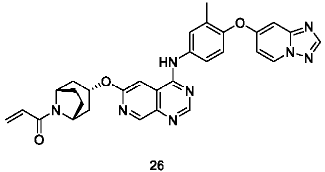

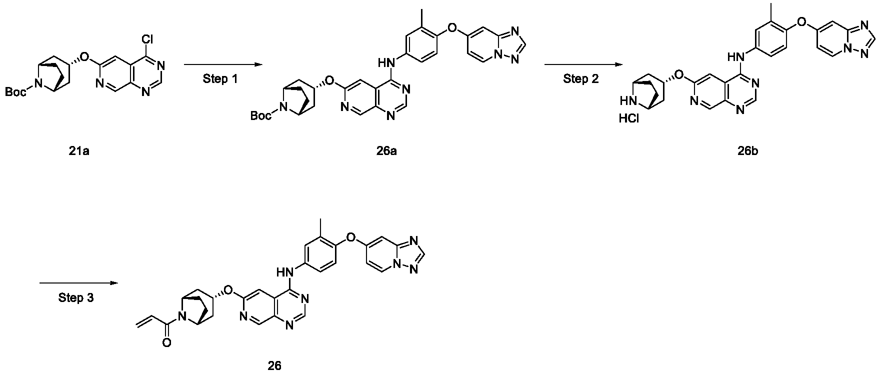

### Step 1

### tert-Butyl exo-3-((4-((4-([1,2,4]triazolo[1,5-a]pyridin-7-yloxy)-3-methylphenyl)amino)pyrido[3,4-d]pyrimidin-6-yl)oxy)-8-azabicyclo[3.2.1]octane-8-carboxylate 26a

The crude product of compound **21a** (1.7 g, 4.86 mmol) was dissolved in isopropanol (50 mL), and compound **1d** (1.17 g, 4.87 mmol) was added. The mixture was stirred at 80 °C for 5 h. The reaction solution was concentrated under reduced pressure to give a crude product of the title compound **26a** (2.8 g). The product was directly used in the next step without purification.

MS m/z (ESI): 595.2 [M+1].

### Step 2

### 6-((exo-8-Azabicyclo[3.2.1]octan-3-yl)oxy)-N-(4-([1,2,4]triazolo[1,5-a]pyridin-7-yloxy)-3-methylphenyl)pyrido[3,4-d]pyrimidin-4-amine hydrochloride 26b

The crude product of compound **26a** (2.8 g, 4.7 mmol) was dissolved in dichloromethane (20 mL), and 20 mL of a 4 M solution of hydrogen chloride in 1,4-dioxane (20 mL) was added. The mixture was stirred for 1 h. The reaction solution was concentrated under reduced pressure to give a crude product of the title compound **26b** (2.5 g). The crude product was directly used in the next step without purification.

MS m/z (ESI): 495.2 [M+1].

### Step 3

### 1-(exo-3-((4-((4-([1,2,4]Triazolo[1,5-a]pyridin-7-yloxy)-3-methylphenyl)amino)pyrido[3,4-d]pyrimidin-6-yl)oxy)-8-azabicyclo[3.2.1]octan-8-yl)prop-2-en-1-one 26

The crude product of compound **26b** (2.5 g, 4.7 mmol) was dissolved in dichloromethane (20 mL), *N,N*-diisopropylethylamine (1.8 g, 14 mmol) was added, and acryloyl chloride (469 mg, 5.18 mmol) was added under an ice bath. The mixture was reacted for 10 min. The reaction solution was concentrated under reduced pressure, and the residue was purified by preparative high performance liquid chromatography (Waters-2545, column: YMC Triart-Exrs C18, 30 × 150 mm, 5 µm; mobile phase: aqueous phase (10 mmol/L ammonium bicarbonate) and acetonitrile, gradient ratio: acetonitrile 30%-45%, flow rate: 30 mL/min) to give the title compound **26** (1.5 g, yield: 58%).

MS m/z (ESI): 549.2 [M+1].

¹H NMR (500 MHz, DMSO-*d*₆): δ 9.86 (s, 1H), 8.94 (d, 2H), 8.58 (s, 1H), 8.39 (s, 1H), 7.95 (d, 1H), 7.93-7.85 (m, 2H), 7.23 (d, 1H), 7.03 (dd, 1H), 6.83-6.79 (m, 1H), 6.78-6.73 (m, 1H), 6.22 (dd, 1H), 5.72 (dd, 1H), 5.61 (tt, 1H), 4.64 (dt, 2H), 2.38-2.35 (m, 1H), 2.27 (dd, 1H), 2.20 (s, 3H), 2.09-2.01 (m, 1H), 1.97-1.84 (m, 3H), 1.69 (t, 1H), 1.55 (t, 1H).

### Example 27

### 1-(exo-3-((4-((2-Fluoro-3-methyl-4-((1-methyl-1H-benzo[d]imidazol-5-yl)oxy)phenyl)amino)pyrido[3,4-d]pyrimidin-6-yl)oxy)-8-azabicyclo[3.2.1]octan-8-yl)prop-2-en-1-one 27

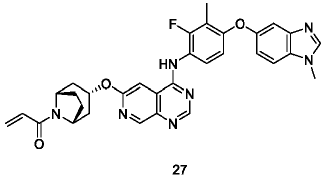

The title compound **27** (50 mg, yield: 45.3%) was obtained by following the synthetic route in Example 26 with the starting material compound **1d** in step 1 replaced by 2-fluoro-3-methyl-4-((1-methyl-1*H*-benzo[*d*]imidazol-5-yl)oxy)aniline.

MS m/z (ESI): 580.2 [M+1].

¹H NMR (500 MHz, CDCl₃): δ 9.00 (s, 1H), 8.68 (s, 1H), 8.20 (t, 1H), 7.89 (s, 1H), 7.57 (d, 1H), 7.40-7.35 (m, 2H), 7.09 (dd, 1H), 7.04 (s, 1H), 6.73 (dd, 1H), 6.57 (dd, 1H), 6.44 (dd, 1H), 5.78-5.68 (m, 2H), 4.93-4.88 (m, 1H), 4.48 (d, 1H), 3.88 (s, 3H), 2.41 (dd, 1H), 2.31 (d, 4H), 2.17 (dt, 1H), 2.05 (q, 2H), 1.96-1.91 (m, 1H), 1.76 (t, 1H).

### Example 28

### 1-(exo-3-((4-((3-Methyl-4-((1-methyl-1H-benzo[d]imidazol-5-yl)oxy)phenyl)amino)pyrido[3,4-d]pyrimidin-6-yl)oxy)-8-azabicyclo[3.2.1]octan-8-yl)prop-2-en-1-one 28

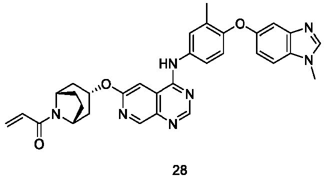

The title compound **28** (50 mg, yield: 47%) was obtained by following the synthetic route in Example 26 with the starting material compound **1d** in step 1 replaced by 3-methyl-4-((1-methyl-1*H*-benzo[*d*]imidazol-5-yl)oxy)aniline.

MS m/z (ESI): 562.2 [M+1].

¹H NMR (500 MHz, CDCl₃): δ 8.96 (s, 1H), 8.67 (s, 1H), 8.12 (s, 1H), 7.87 (s, 1H), 7.67 (d, 1H), 7.51 (dd, 1H), 7.38-7.31 (m, 2H), 7.09 (dd, 1H), 6.89 (d, 1H), 6.58 (dd, 1H), 6.43 (dd, 1H), 5.79-5.70 (m, 2H), 4.89 (d, 1H), 4.48 (d, 1H), 3.87 (s, 3H), 2.45 (dd, 1H), 2.35 (s, 3H), 2.25-2.15 (m, 2H), 2.05 (q, 3H), 1.93 (s, 1H), 1.72 (t, 1H).

### Biological Evaluations

The present disclosure is further described and explained below with reference to test examples. However, these test examples are not intended to limit the scope of the present disclosure.

### Test example 1: Ba/F3 Cell Proliferation Assay

EGFR wild-type Ba/F3 cells (Cobioer, Cat. No. CBP73110) in the logarithmic growth phase were seeded into a 96-well plate at 2.5 × 10³ cells/100 µL in a growth medium, and HER2 wild-type Ba/F3 cells (Cobioer, Cat. No. CBP73110) or HER2 A775_G776insYVMA mutant Ba/F3 (Cobioer, Cat. No. CBP73184) in the logarithmic growth phase were seeded into a 96-well plate at 5 × 10³ cells/100 µL in a growth medium. The plates were placed in a cell incubator at 37 °C overnight. The compound diluted in 3-fold gradient with a medium was added to the plates at 100 µL/well the next day. All treatments were performed in triplicate. The plates were cultured in the cell incubator at 37 °C for another 72 h. Celltiter-Glo Luminescent cell viability assay was performed. The cell proliferation rate corresponding to each well was calculated according to the following formula: proliferation% = (test compound well G3 - G0 average value)/(DMSO control well G3 average value - G0 average value) × 100. According to the corresponding proliferation rate and concentration of each gradient concentration well, a gradient curve of cell proliferation was fitted using Prism Graphpad software, and GI₅₀ (GI₅₀ is defined as the concentration of a compound corresponding to the cell proliferation inhibition rate of 50%) of the compound was calculated.

The HER2-selective inhibitor, tucatinib (synthesized with reference to Example 11 in the patent WO2007059257A2), has the following structure:

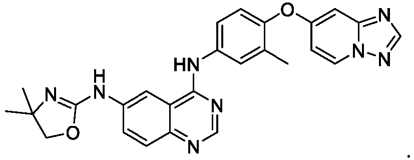

**Table 1. GI₅₀ values for inhibitory activities of compounds of the present disclosure on Ba/F3 cell proliferation**

| Example No. | HER2 WT/ BaF3 GI₅₀ (nM) | HER2 WT/ BaF3 Imax % | HER2 A775_G776 ins YVMA/ BaF3 GI₅₀ (nM) | HER2 A775_G776 ins YVMA/ BaF3 Imax % | EGFR WT/ BaF3 GI₅₀ (nM) | EGFR WT/ BaF3 Imax % |
|---|---|---|---|---|---|---|
| 1 | 4.1 | 100 | 3.4 | 100 | 3742 | 97 |
| 2 | 18.9 | 100 | 24.0 | 100 | 2302 | 99 |
| 6 | 25.2 | 100 | 21.4 | 100 | - | - |
| 8 | 6.3 | 100 | 15.7 | 100 | - | - |
| 9 | 13.5 | 100 | 13.8 | 100 | - | - |
| 10 | 9.3 | 100 | 10.3 | 100 | 2840 | 100 |
| 13 | 4.8 | 100 | 8.2 | 100 | - | - |
| 15 | 14.9 | 100 | 19.4 | 100 | - | - |
| 16 | 9.0 | 100 | 15.7 | 100 | 7350 | 73 |
| 17 | 5.1 | 100 | 13.9 | 100 | - | - |
| 18 | 17.7 | 100 | 23.6 | 100 | 2129 | 100 |
| 19 | 14.9 | 100 | 27.0 | 100 | - | - |
| 20 | 13.2 | 100 | 15.5 | 100 | 3506 | 100 |
| 21 | 5.3 | 100 | 10.4 | 100 | 913 | 100 |
| 26 | 9.2 | 100 | 23.4 | 100 | 1629 | 100 |
| 27 | 12.5 | 100 | - | - | - | - |
| 28 | 18.1 | 100 | - | - | > 10000 | -1.76 |
| Tucatinib | - | - | 309.23 | 100 | - | - |

Conclusion: the compounds of the present disclosure have strong proliferation inhibitory effects on Ba/F3 cells with the HER2 exon 20 YVMA insertion mutation and HER2 wild-type-dependent Ba/F3 cells, and have relatively strong selectivity relative to EGFR wild-type-dependent Ba/F3 cells; in addition, the compounds of the present disclosure have proliferation inhibitory activities on Ba/F3 cells with the HER2 exon 20 YVMA insertion mutation, which are significantly better than that of tucatinib.

### Test Example 2: Pharmacokinetic Evaluation

TPGS: D-α-vitamin E polyethylene glycol succinate
HPMC: hydroxypropyl methylcellulose
PEG400: polyethylene glycol 400

### I. SD rat testing

### 1. Abstract

SD rats were used as test animals. After intragastric (i.g.) administration of example compounds to SD rats, the plasma concentrations at different time points were measured by the LC/MS/MS method. The pharmacokinetic behavior of the compound of the present disclosure in SD rats was studied, and its pharmacokinetic profiles were evaluated.

### 2. Method

### 2.1. Test compound

Example 10 compound.

### 2.2. Test animals

Four SD rats, of which half were male and half female, were provided by Vital River Laboratory Animal Technology Co., Ltd. The animals were fasted overnight and then intragastrically dosed.

### 2.3. Compound solution preparation

A certain amount of example compound was weighed out and dissolved in 5% DMSO + 20% PEG400 + 70% (10% TPGS) + 5% (1% HPMC K100LV) to prepare a 5 mg/mL colorless clear solution.

### 2.4. Administration

The dose administered was 50 mg/kg, and the volume was 10.0 mL/kg.

### 3. Procedures

0.2-mL blood samples were collected from the orbit before administration and 0.25 h, 0.5 h, 1.0 h, 2.0 h, 4.0 h, 6.0 h, 8.0 h, 11.0 h, and 24.0 h after administration. The blood samples were placed into EDTA-K2 anticoagulation test tubes and centrifuged at 10,000 rpm for 1 min (4 °C), and plasma was separated within 1 h and stored in dry ice before analysis. The procedure starting from the blood collection to the centrifugation was performed under ice-bath conditions. Access to food was given 2 hours after administration.

After administration of different concentrations of the test compounds, the plasma concentration of the test compound in SD rats was determined: plasma samples (50 µL) were collected from the SD rats at various time points after administration, and 25 µL of camptothecin (1 µg/mL) and 450 µL of acetonitrile were added to each of the samples. The mixtures were vortexed and centrifuged at 3700 rpm for 10 min. 0.1 µL of the supernatant was taken for LC/MS/MS analysis.

### 4. Pharmacokinetic parameters

**Table 2. Pharmacokinetic parameters of compound of the present disclosure in SD rats**

| No. | Plasma concentration Cmax (ng/mL) | Area under curve AUC₀₋ₜ (h*ng/mL) | Half-life T1/2 (h) | Clearance rate CL/F (mL/min/kg) | Apparent distribution volume Vz/F (mL/kg) |
|---|---|---|---|---|---|
| Example 10 compound | 19280 | 70780 | 2.3 | 14.7 | 2952 |

Conclusion: the compound of the present disclosure has high exposure in SD rats and has significant pharmacokinetic advantages.

### II. Testing in C57 mice

### 1. Abstract

C57 mice were used as test animals. After intragastric (i.g.) administration of example compounds to C57 mice, the plasma concentrations at different time points were measured by the LC/MS/MS method. The pharmacokinetic behavior of the compounds of the present disclosure in C57 mice was studied and their pharmacokinetic profiles were evaluated.

### 2. Method

### 2.1. Test compounds

Example 10 compound, Example 18 compound, and Example 26 compound.

### 2.2. Test animals

Twenty-seven C57 mice, female, were evenly divided into 3 groups, provided by Beijing HFK Bioscience Co., Ltd., and intragastrically administered.

### 2.3. Compound solution preparation

A certain amount of example compound was weighed out and dissolved in 5% DMSO + 20% PEG400 + 70% (10% TPGS) + 5% (1% HPMC K100LV) to prepare a 2.5 mg/mL colorless clear solution.

### 2.4. Administration

The dose administered was 50 mg/kg, and the volume was 20.0 mL/kg.

### 3. Procedures

0.1-mL blood samples were collected from the orbit before administration and 0.25 h, 0.5 h, 1.0 h, 2.0 h, 4.0 h, 6.0 h, 8.0 h, 11.0 h, and 24.0 h after administration. The blood samples were placed into EDTA-K2 anticoagulation test tubes and centrifuged at 10,000 rpm for 2 min (4 °C), and plasma was separated within 1 h and stored at -20 °C before analysis. The procedure starting from the blood collection to the centrifugation was performed under ice-bath conditions. Access to food was given 2 hours after administration.

After administration of different concentrations of the test compounds, the plasma concentrations of the test compounds in C57 mice were determined: plasma samples (50 µL) were collected from the C57 mice at various time points after administration, and 25 µL of labetalol (1 µg/mL) and 450 µL of acetonitrile were added to each of the samples. The mixtures were vortexed and centrifuged at 3700 rpm for 10 min. 0.1 µL of the supernatant was taken for LC/MS/MS analysis.

### 4. Pharmacokinetic parameters

**Table 3. Pharmacokinetic parameters of compounds of the present disclosure in C57 mice**

| No. | Plasma concentration Cmax (ng/mL) | Area under curve AUC₀₋ₜ (h*ng/mL) | Half-life T1/2 (h) | Clearance rate CL/F (mL/min/kg) | Apparent distribution volume Vz/F (mL/kg) |
|---|---|---|---|---|---|
| Example 10 compound | 14000 | 31189 | 1.6 | 26.5 | 3755 |
| Example 18 compound | 12726 | 44909 | 2.5 | 18.5 | 3931 |
| Example 26 compound | 11267 | 32058 | 1.3 | 26.0 | 2882 |

Conclusion: the compounds of the present disclosure have high exposure in C57 mice and have significant pharmacokinetic advantages.

## Claims

1. A compound of general formula (I) or a pharmaceutically acceptable salt thereof: 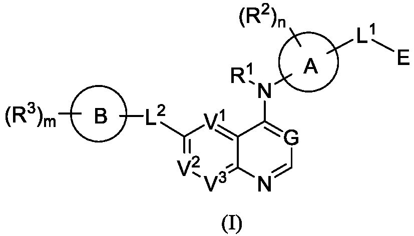 wherein:
ring A is aryl or heteroaryl;
ring B is 7- to 10-membered fused heterocyclyl or 7- to 10-membered bridged heterocyclyl;
G is N or C(R^{A});
R^{A} is selected from the group consisting of a hydrogen atom, halogen, alkyl, haloalkyl, hydroxyalkyl, alkoxy, haloalkoxy, cyano, hydroxy, and amino;
V¹ is C(R^{a}) or N;
V² and V³ are identical or different and are each independently C(R^{a}) or N; or V² is C(R^{bb}), V³ is C(R^{cc}), and R^{bb} and R^{cc}, together with the carbon atom to which they are each attached, form cycloalkyl, heterocyclyl, aryl, or heteroaryl, wherein the cycloalkyl, heterocyclyl, aryl, or heteroaryl is optionally substituted with one or more substituents selected from the group consisting of halogen, alkyl, haloalkyl, hydroxyalkyl, alkoxy, haloalkoxy, cyano, hydroxy, amino, nitro, cycloalkyl, heterocyclyl, aryl, and heteroaryl;
L¹ is selected from the group consisting of O, NR^{b1}, C(O), S, S(O), and S(O)₂;
L² is selected from the group consisting of O, NR^{b2}, C(O), (CR^{c}R^{d})ᵤ, (CR^{c}R^{d})ᵤO, O(CR^{c}R^{d})ᵤ, (CR^{c}R^{d})ᵤNR^{b2}, NR^{b2}(CR^{c}R^{d})ᵤ, C(O)NR^{b2}, and NR^{b2}C(O);
E is 9- to 10-membered heteroaryl, wherein the 9- to 10-membered heteroaryl is optionally substituted with one or more R¹⁶;
R¹⁶ is selected from the group consisting of halogen, alkyl, alkenyl, alkynyl, cyano, nitro, -OR⁴, -NR⁵R⁶, -C(O)R⁴, -C(O)OR⁴, -OC(O)R⁴, -C(O)NR⁵R⁶, -S(O)ₚR⁴, -S(O)ₚNR⁵R⁶, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, aminoalkyl, cyano, -OR^{4a}, -NR^{5a}R^{6a}, cycloalkyl, heterocyclyl, aryl, and heteroaryl;
R^{a} is selected from the group consisting of a hydrogen atom, halogen, alkyl, alkenyl, alkynyl, cyano, nitro, haloalkyl, hydroxyalkyl, -OR^{e}, -(CH₂)ₛ-NR^{f}R^{g}, cycloalkyl, heterocyclyl, aryl, and heteroaryl;
R^{b1} and R^{b2} are identical or different and are each independently selected from the group consisting of a hydrogen atom, alkyl, haloalkyl, hydroxyalkyl, cycloalkyl, and heterocyclyl;
R^{c} and R^{d} are identical or different and are each independently selected from the group consisting of a hydrogen atom, halogen, alkyl, hydroxy, and hydroxyalkyl;
R¹ is selected from the group consisting of a hydrogen atom, alkyl, and cycloalkyl;
each R² is identical or different and is independently selected from the group consisting of halogen, alkyl, alkenyl, alkynyl, cyano, nitro, haloalkyl, hydroxyalkyl, -OR⁷, -(CH₂)ᵥ-NR⁸R⁹, cycloalkyl, heterocyclyl, aryl, and heteroaryl;
each R³ is identical or different and is independently selected from the group consisting of oxo, halogen, alkyl, alkenyl, alkynyl, cyano, nitro, -OR¹⁰, -NR¹¹R¹², -C(O)R¹⁰, - C(O)OR¹⁰, -OC(O)R¹⁰, -C(O)NR¹¹R¹², -NR¹³C(O)R¹⁰, -NR¹³C(O)OR¹⁰, - NR¹³C(O)NR¹¹R¹², -S(O)ₚR¹⁰, -S(O)ₚNR¹¹R¹², -NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl;
R¹⁰ are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more R^{B};
R^{B} is selected from the group consisting of oxo, halogen, alkyl, alkenyl, alkynyl, cyano, -OR^{10b}, -NR^{11b}R^{12b}, -C(O)R^{10b}, -C(O)NR^{11b}R^{12b}, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, alkoxy, hydroxy, cyano, and amino;
R^{e}, R⁴, R^{4a}, R⁷, R^{10a}, and R^{10b} are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, amino, cycloalkyl, heterocyclyl, aryl, and heteroaryl;
R^{f}, R^{g}, R⁵, R⁶, R⁸, R⁹, R¹¹, and R¹² are identical or different at each occurrence and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
or R^{f} and R^{g}, together with the nitrogen atom to which they are attached, form heterocyclyl, or R⁵ and R⁶, together with the nitrogen atom to which they are attached, form heterocyclyl, or R⁸ and R⁹, together with the nitrogen atom to which they are attached, form heterocyclyl, or R¹¹ and R¹², together with the nitrogen atom to which they are attached, form heterocyclyl, wherein the heterocyclyl is optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
R¹³ are identical or different at each occurrence and are each independently selected from the group consisting of a hydrogen atom, alkyl, and cycloalkyl;
R^{5a}, R^{6a}, R¹¹, R^{12a}, R^{11b}, and R^{12b} are identical or different at each occurrence and are each independently selected from the group consisting of a hydrogen atom, alkyl, haloalkyl, hydroxyalkyl, aminoalkyl, cycloalkyl, heterocyclyl, aryl, heteroaryl, cycloalkylalkyl, heterocyclylalkyl, arylalkyl, and heteroarylalkyl;
s is 0, 1, or 2;
v is 0, 1, or 2;
u is 1, 2, 3, or 4;
p is 0, 1, or 2;
n is 0, 1, 2, 3, or 4; and
m is an integer from 0 to 10.

2. The compound of general formula (I) or the pharmaceutically acceptable salt thereof according to claim 1,
wherein:
ring A is aryl or heteroaryl;
ring B is 7- to 10-membered fused heterocyclyl or 7- to 10-membered bridged heterocyclyl;
G is N or C(R^{A});
R^{A} is selected from the group consisting of a hydrogen atom, halogen, alkyl, haloalkyl, hydroxyalkyl, alkoxy, haloalkoxy, cyano, hydroxy, and amino;
V¹, V², and V³ are identical or different and are each independently C(R^{a}) or N;
L¹ is selected from the group consisting of O, NR^{b1}, C(O), S, S(O), and S(O)₂;
L² is selected from the group consisting of O, NR^{b2}, C(O), (CR^{c}R^{d})ᵤ, (CR^{c}R^{d})ᵤO, O(CR^{c}R^{d})ᵤ, (CR^{c}R^{d})ᵤNR^{b2}, NR^{b2}(CR^{c}R^{d})ᵤ, C(O)NR^{b2}, and NR^{b2}C(O);
E is 9- to 10-membered heteroaryl, wherein the 9- to 10-membered heteroaryl is optionally substituted with one or more R¹⁶;
R¹⁶ is selected from the group consisting of halogen, alkyl, alkenyl, alkynyl, cyano, nitro, -OR⁴, -NR⁵R⁶, -C(O)R⁴, -C(O)OR⁴, -OC(O)R⁴, -C(O)NR⁵R⁶, -S(O)ₚR⁴, -S(O)ₚNR⁵R⁶, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, aminoalkyl, cyano, -OR^{4a}, -NR^{5a}R^{6a}, cycloalkyl, heterocyclyl, aryl, and heteroaryl;
R^{a} is selected from the group consisting of a hydrogen atom, halogen, alkyl, alkenyl, alkynyl, cyano, nitro, haloalkyl, hydroxyalkyl, -OR^{e}, -(CH₂)ₛ-NR^{f}R^{g}, cycloalkyl, heterocyclyl, aryl, and heteroaryl;
R^{b1} and R^{b2} are identical or different and are each independently selected from the group consisting of a hydrogen atom, alkyl, haloalkyl, hydroxyalkyl, cycloalkyl, and heterocyclyl;
R^{c} and R^{d} are identical or different and are each independently selected from the group consisting of a hydrogen atom, halogen, alkyl, hydroxy, and hydroxyalkyl;
R¹ is selected from the group consisting of a hydrogen atom, alkyl, and cycloalkyl;
each R² is identical or different and is independently selected from the group consisting of halogen, alkyl, alkenyl, alkynyl, cyano, nitro, haloalkyl, hydroxyalkyl, -OR⁷, -(CH₂)ᵥ-NR⁸R⁹, cycloalkyl, heterocyclyl, aryl, and heteroaryl;
each R³ is identical or different and is independently selected from the group consisting of oxo, halogen, alkyl, alkenyl, alkynyl, cyano, nitro, -OR¹⁰, -NR¹¹R¹², -C(O)R¹⁰, - C(O)OR¹⁰, -OC(O)R¹⁰, -C(O)NR¹¹R¹², -NR¹³C(O)R¹⁰, -NR¹³C(O)OR¹⁰, - NR¹³C(O)NR¹¹R¹², -S(O)ₚR¹⁰, -S(O)ₚNR¹¹R¹², -NR¹³S(O)ₚR¹⁰, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, -OR^{10a}, -NR^{11a}R^{12a}, cycloalkyl, heterocyclyl, aryl, and heteroaryl;
R¹⁰ are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more R^{B};
R^{B} is selected from the group consisting of oxo, halogen, alkyl, alkenyl, alkynyl, cyano, -OR^{10b}, -NR^{11b}R^{12b}, -C(O)R^{10b}, -C(O)NR^{11b}R^{12b}, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, haloalkyl, hydroxyalkyl, alkoxy, hydroxy, cyano, and amino;
R^{e}, R⁴, R^{4a}, R⁷, R^{10a}, and R^{10b} are identical or different at each occurrence and are each independently selected from the group consisting of a hydrogen atom, alkyl, alkenyl, alkynyl, cycloalkyl, heterocyclyl, aryl, and heteroaryl, wherein the alkyl, alkenyl, alkynyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, amino, cycloalkyl, heterocyclyl, aryl, and heteroaryl;
R^{f}, R^{g}, R⁵, R⁶, R⁸, R⁹, R¹¹, and R¹² are identical or different at each occurrence and are each independently selected from the group consisting of a hydrogen atom, alkyl, cycloalkyl, heterocyclyl, aryl, and heteroaryl, wherein the alkyl, cycloalkyl, heterocyclyl, aryl, and heteroaryl are each independently optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
or R^{f} and R^{g}, together with the nitrogen atom to which they are attached, form heterocyclyl, or R⁵ and R⁶, together with the nitrogen atom to which they are attached, form heterocyclyl, or R⁸ and R⁹, together with the nitrogen atom to which they are attached, form heterocyclyl, or R¹¹ and R¹², together with the nitrogen atom to which they are attached, form heterocyclyl, wherein the heterocyclyl is optionally substituted with one or more substituents selected from the group consisting of oxo, halogen, alkyl, alkenyl, alkynyl, haloalkyl, hydroxyalkyl, aminoalkyl, cyano, hydroxy, alkoxy, haloalkoxy, amino, cycloalkyl, heterocyclyl, aryl, and heteroaryl;
R¹³ are identical or different at each occurrence and are each independently selected from the group consisting of a hydrogen atom, alkyl, and cycloalkyl;
R^{5a}, R^{6a}, R^{11a}, R^{12a}, R^{11b}, and R^{12b} are identical or different at each occurrence and are each independently selected from the group consisting of a hydrogen atom, alkyl, haloalkyl, hydroxyalkyl, aminoalkyl, cycloalkyl, heterocyclyl, aryl, heteroaryl, cycloalkylalkyl, heterocyclylalkyl, arylalkyl, and heteroarylalkyl;
s is 0, 1, or 2;
v is 0, 1, or 2;
u is 1, 2, 3, or 4;
p is 0, 1, or 2;
n is 0, 1, 2, 3, or 4; and
m is an integer from 0 to 10.

3. The compound of general formula (I) or the pharmaceutically acceptable salt thereof according to claim 1 or 2, wherein L¹ is O.

4. The compound of general formula (I) or the pharmaceutically acceptable salt thereof according to any one of claims 1 to 3, wherein ring A is 6- to 10-membered aryl or 5- to 10-membered heteroaryl; preferably, ring A is phenyl.

5. The compound of general formula (I) or the pharmaceutically acceptable salt thereof according to any one of claims 1 to 4, wherein G is N.

6. The compound of general formula (I) or the pharmaceutically acceptable salt thereof according to any one of claims 1 to 5, wherein R¹ is a hydrogen atom.

7. The compound of general formula (I) or the pharmaceutically acceptable salt thereof according to any one of claims 1 to 6, wherein L² is O or (CR^{c}R^{d})ᵤO, and R^{c}, R^{d}, and u are as defined in claim 1; preferably, L² is O or CH₂O.

8. The compound of general formula (I) or the pharmaceutically acceptable salt thereof according to any one of claims 1 to 7, being a compound of general formula (II) or a pharmaceutically acceptable salt thereof: 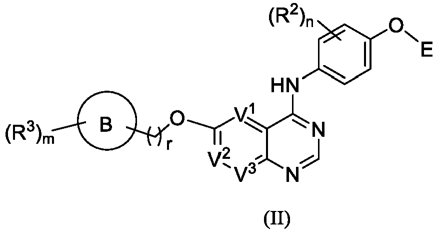 wherein:
r is 0 or 1;
ring B, E, R², R³, V¹, V², V³, m, and n are as defined in claim 1.

9. The compound of general formula (I) or the pharmaceutically acceptable salt thereof according to any one of claims 1 to 8, wherein ring B is 8-membered fused heterocyclyl or 7- to 8-membered bridged heterocyclyl; preferably, ring B is 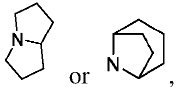 and ring B can be substituted with R³ at any substitutable position.

10. The compound of general formula (I) or the pharmaceutically acceptable salt thereof according to any one of claims 1 to 9, wherein each R³ is identical or different and is independently selected from the group consisting of halogen, C₁₋₆ alkyl, -OR¹⁰, and - C(O)R¹⁰, wherein the C₁₋₆ alkyl is optionally substituted with one or more substituents selected from the group consisting of halogen, cyano, -OR^{10a}, -NR^{11a}R^{12a}, 3- to 8-membered cycloalkyl, 3- to 8-membered heterocyclyl, 6- to 10-membered aryl, and 5- to 10-membered heteroaryl; and R¹⁰, R^{10a}, R^{11a}, and R^{12a} are as defined in claim 1; preferably, R³ is halogen or -C(O)R¹⁰; and R¹⁰ is as defined in claim 1.

11. The compound of general formula (I) or the pharmaceutically acceptable salt thereof according to any one of claims 1 to 7, 9, and 10, being a compound of general formula (III) or a pharmaceutically acceptable salt thereof: 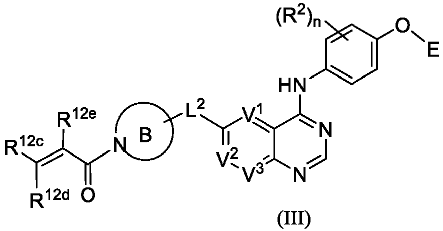
wherein ring B is 7- to 10-membered nitrogen-containing fused heterocyclyl or 7- to 10-membered nitrogen-containing bridged heterocyclyl;
R^{12c}, R^{12d}, and R^{12e} are identical or different and are each independently selected from the group consisting of a hydrogen atom, halogen, C₁₋₆ alkyl, 3- to 8-membered cycloalkyl, and 3- to 8-membered heterocyclyl, wherein the C₁₋₆ alkyl, 3- to 8-membered cycloalkyl, and 3- to 8-membered heterocyclyl are each independently optionally substituted with one or more substituents selected from the group consisting of oxo, halogen, C₁₋₆ alkyl, C₁₋₆ haloalkyl, C₁₋₆ alkoxy, hydroxy, cyano, and amino; preferably, R^{12c}, R^{12d}, and R^{12e} are identical or different and are each independently selected from the group consisting of a hydrogen atom, halogen, and C₁₋₆ alkyl; more preferably, R^{12c}, R^{12d}, and R^{12e} are all hydrogen atoms; and
E, R², L², V¹, V², V³, and n are as defined in claim 1.

12. The compound of general formula (I) or the pharmaceutically acceptable salt thereof according to any one of claims 1 to 11, wherein E is selected from the group consisting 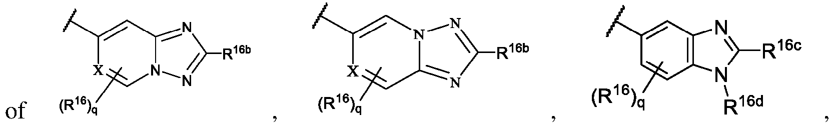 and 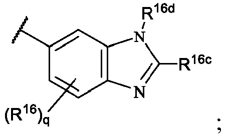 X is N or CR^{16a}; R^{16a}, R^{16b}, and R^{16c} are identical or different and are each independently selected from the group consisting of a hydrogen atom, halogen, and C₁₋₆ alkyl; R^{16d} is selected from the group consisting of a hydrogen atom, C₁₋₆ alkyl, and 3- to 8-membered cycloalkyl; R¹⁶ is halogen or C₁₋₆ alkyl; q is 0, 1, 2, or 3.

13. The compound of general formula (I) or the pharmaceutically acceptable salt thereof according to any one of claims 1 to 12, wherein each R² is identical or different and is independently C₁₋₆ alkyl or halogen.

14. The compound of general formula (I) or the pharmaceutically acceptable salt thereof according to any one of claims 1 and 3 to 13, wherein V¹ is C(R^{a}) or N; V² and V³ are identical or different and are each independently C(R^{a}) or N, or V² is C(R^{bb}), V³ is C(R^{cc}), and R^{bb} and R^{cc}, together with the carbon atom to which they are each attached, form 5- or 6-membered cycloalkyl or 5- or 6-membered heterocyclyl; R^{a} is a hydrogen atom or C₁₋₆ alkoxy.

15. The compound of general formula (I) or the pharmaceutically acceptable salt thereof according to any one of claims 1 to 14, being selected from the group consisting of the following compounds:

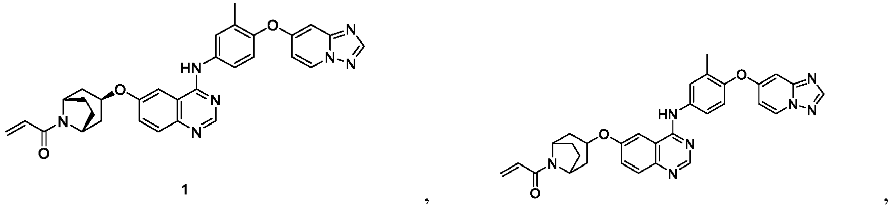

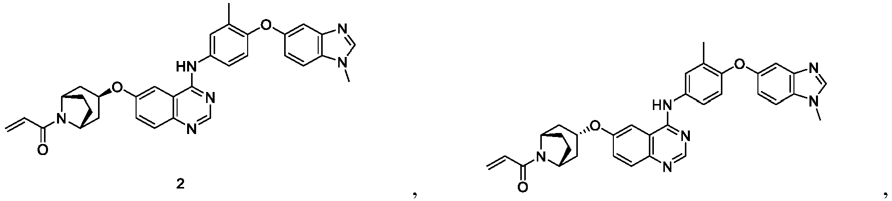

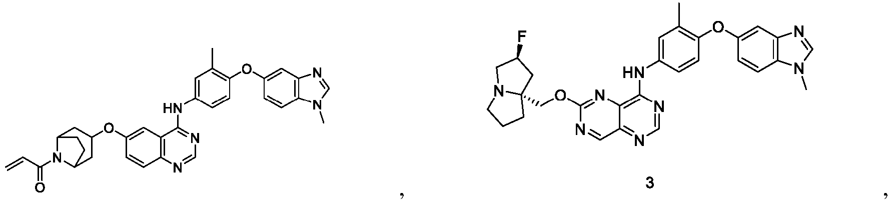

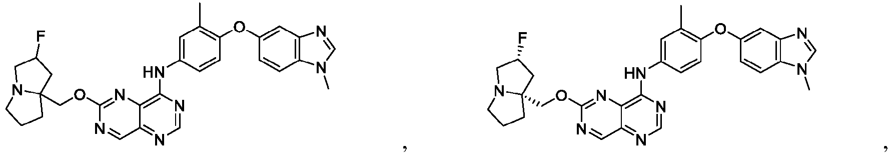

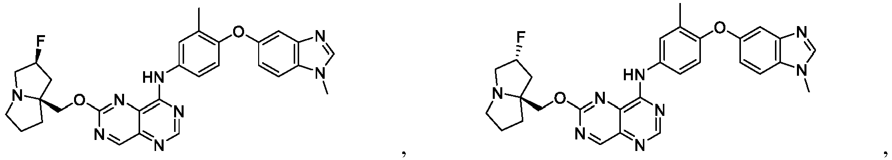

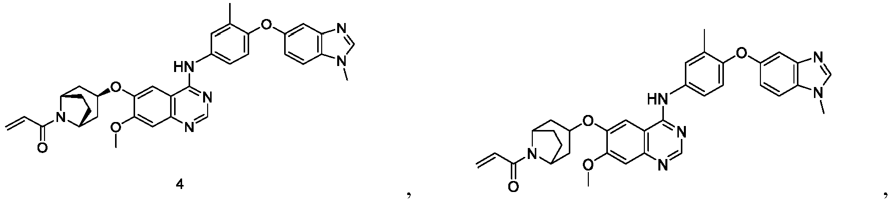

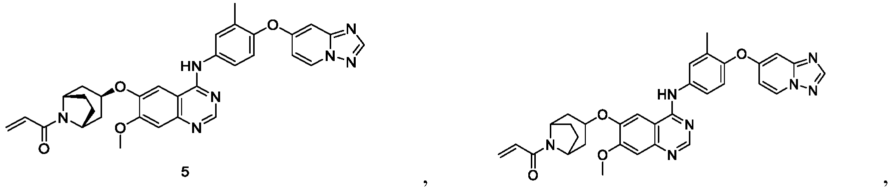

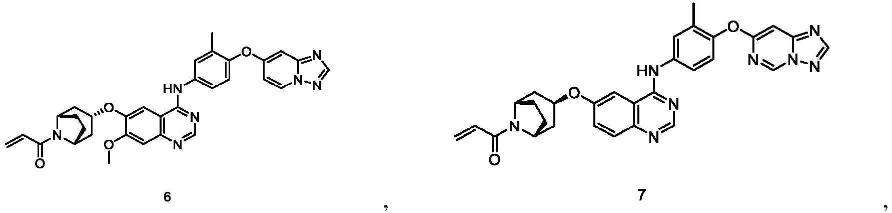

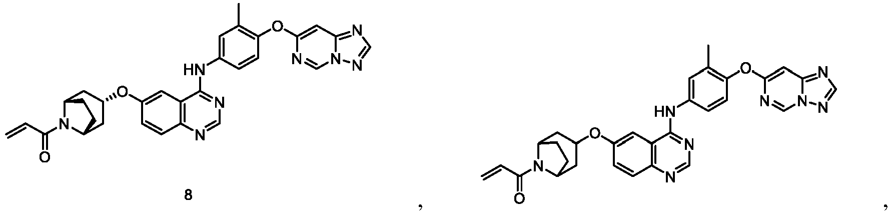

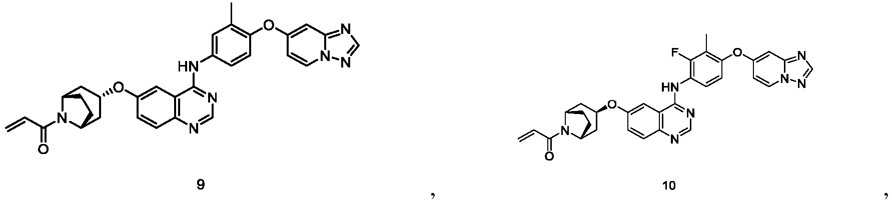

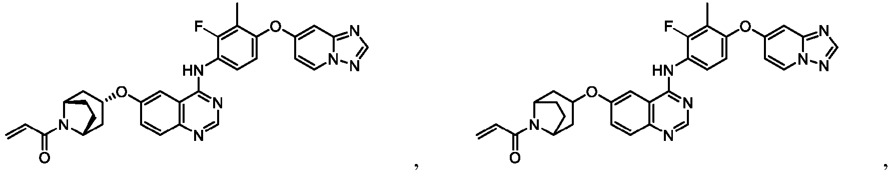

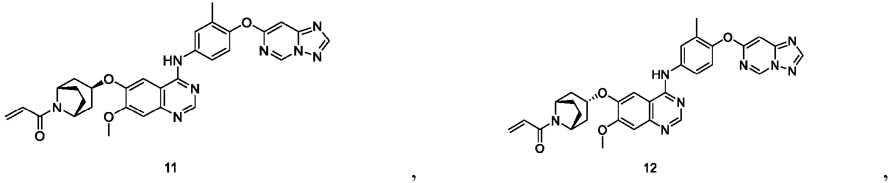

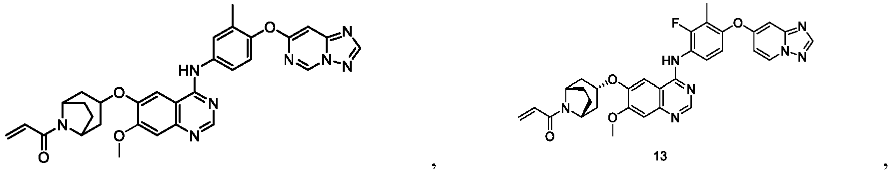

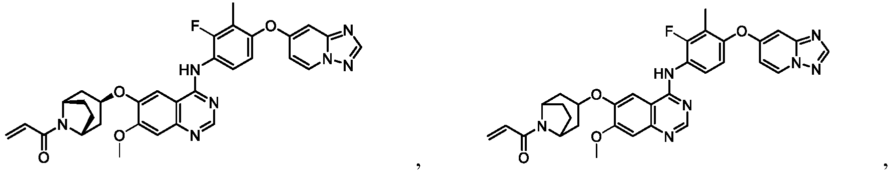

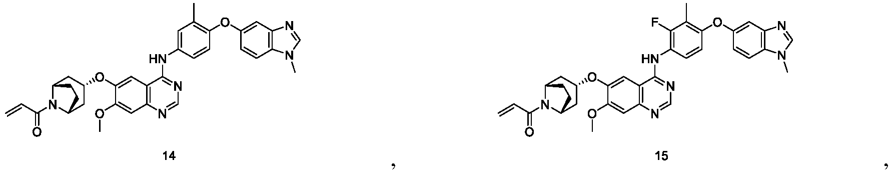

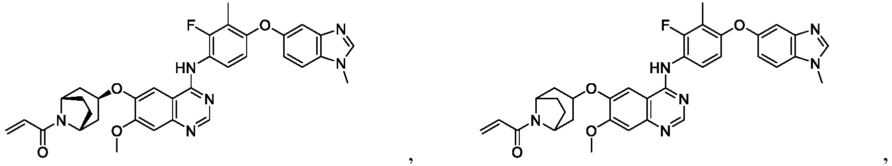

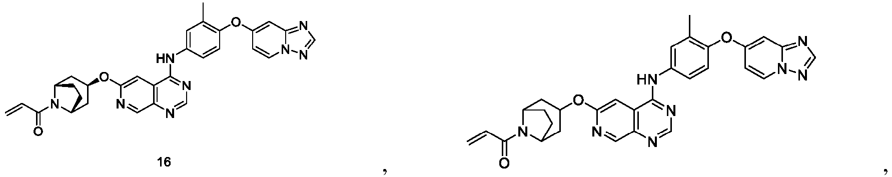

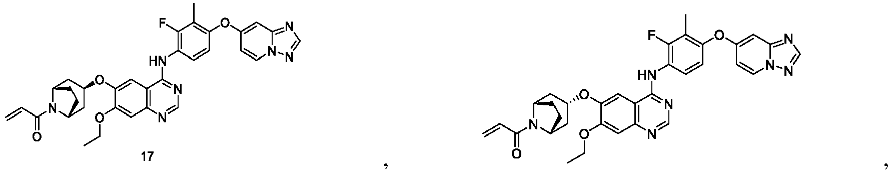

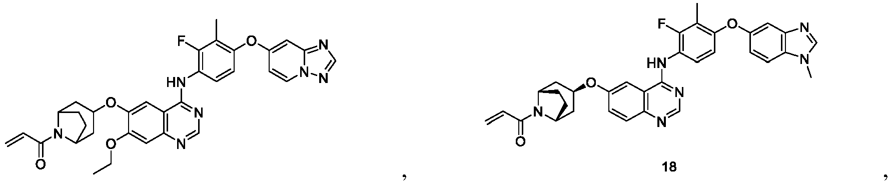

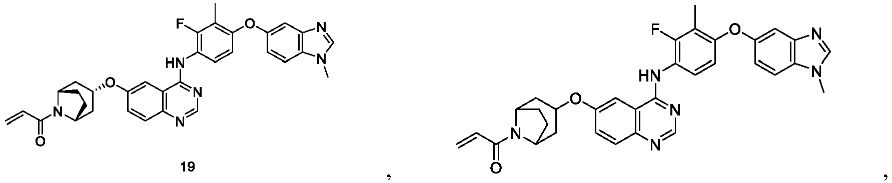

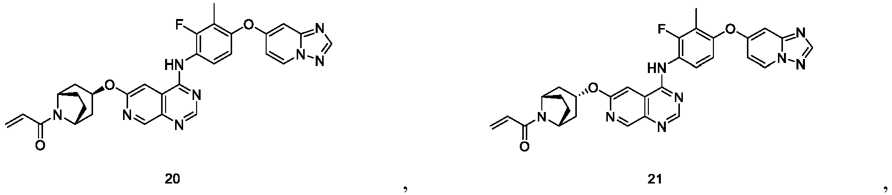

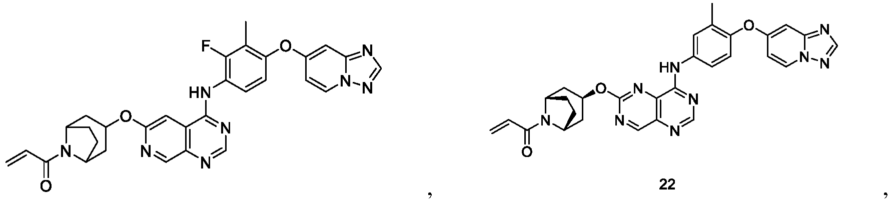

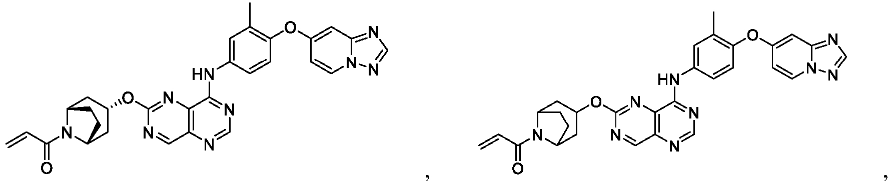

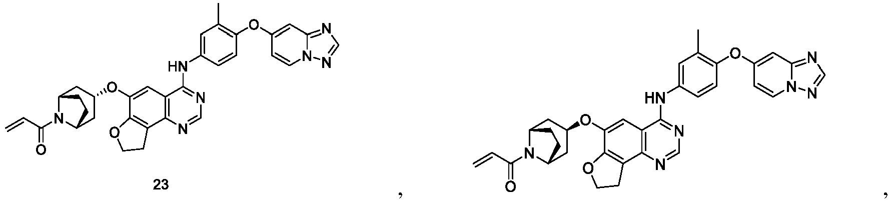

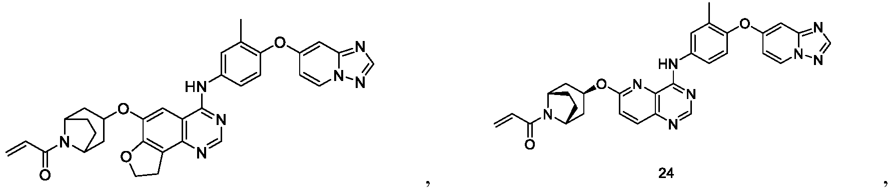

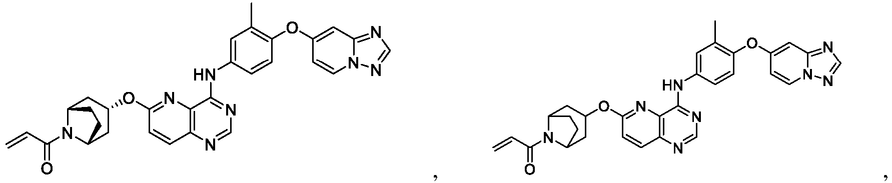

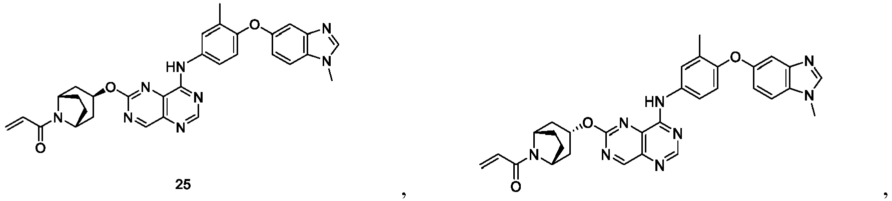

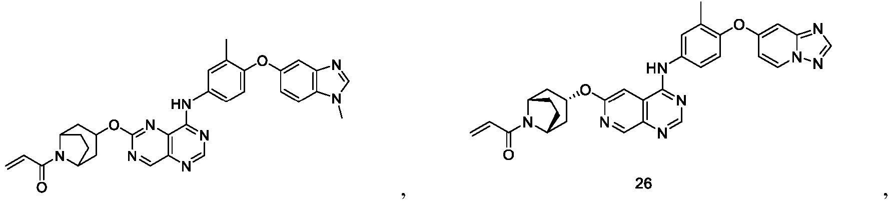

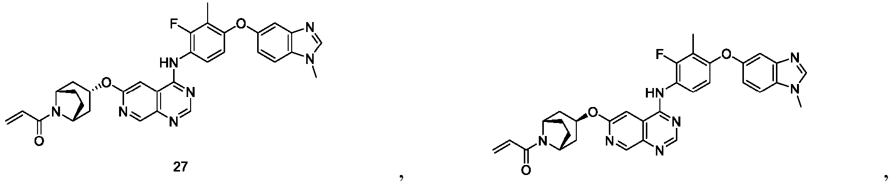

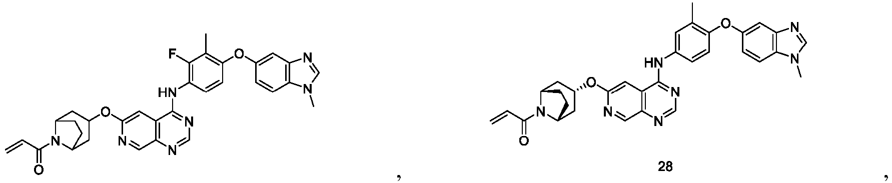

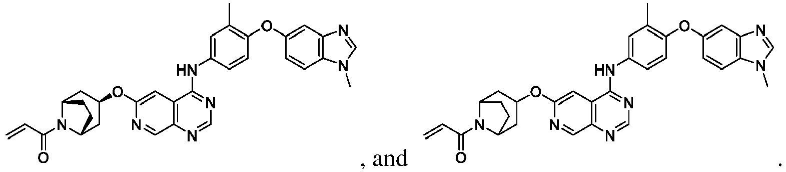

16. A compound of general formula (IIIa) or a salt thereof: 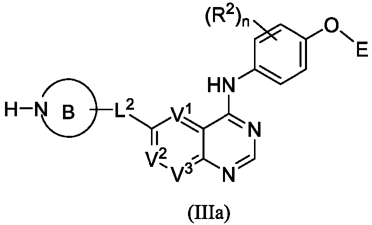 wherein:
E, ring B, R², L², V¹, V², V³, and n are as defined in claim 11.

17. The compound or the salt thereof according to claim 16, being selected from the group consisting of the following compounds: 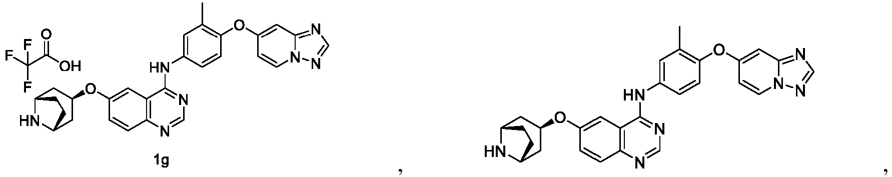 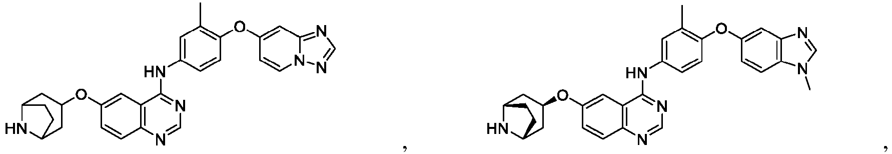 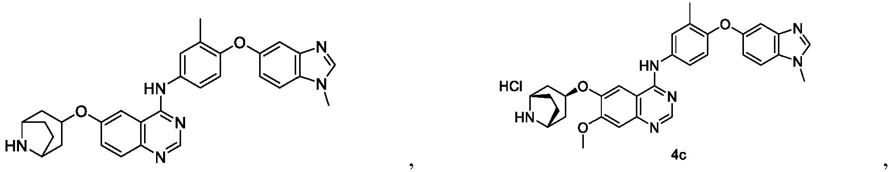 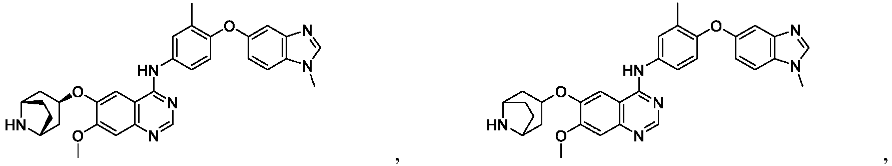 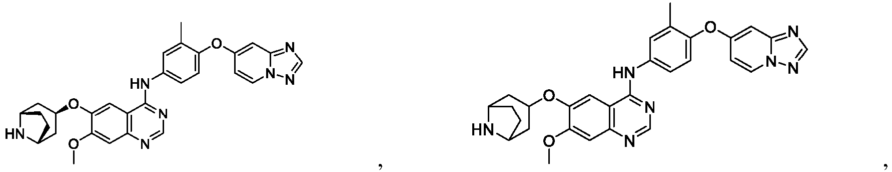 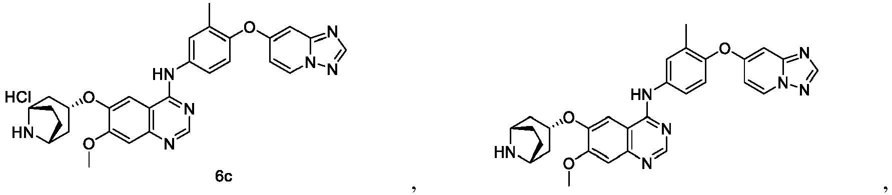 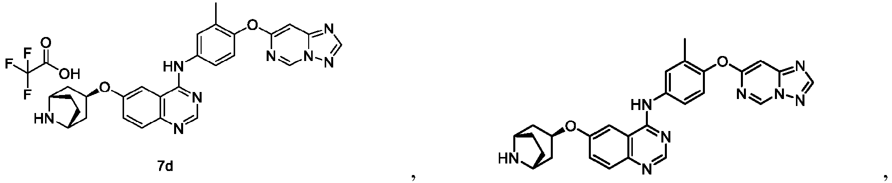 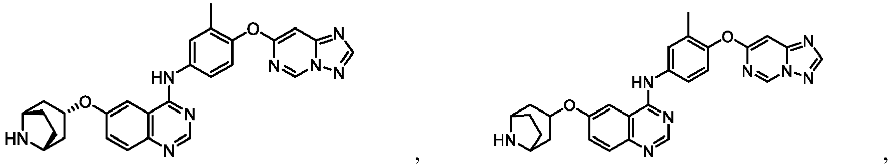 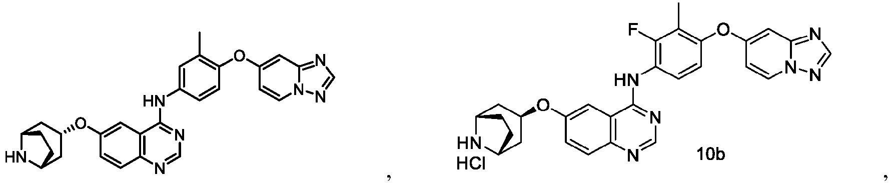 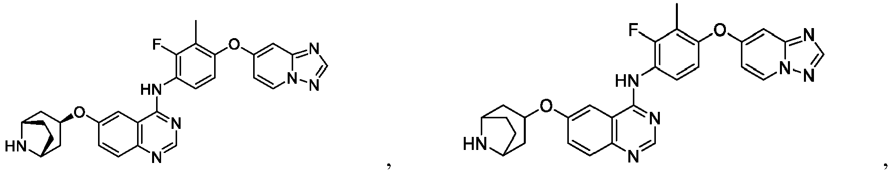 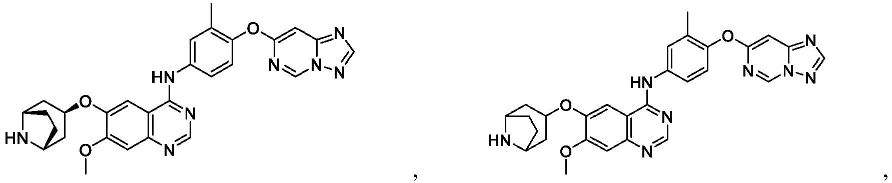 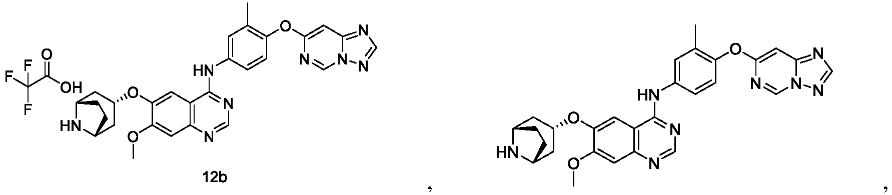 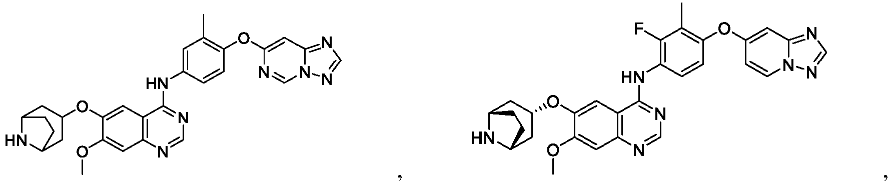 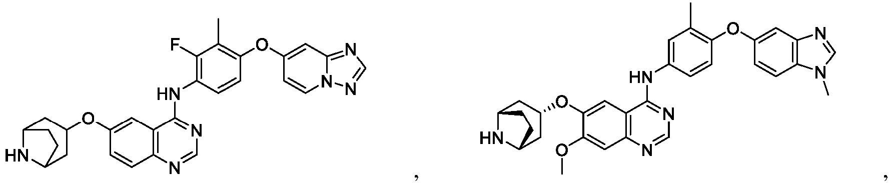 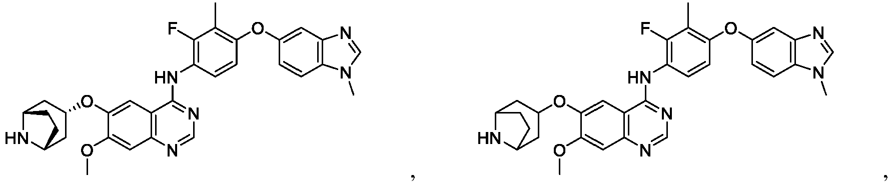 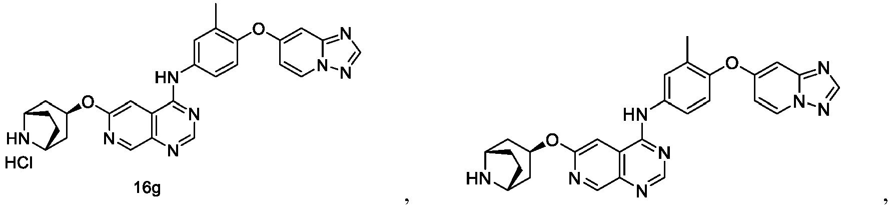 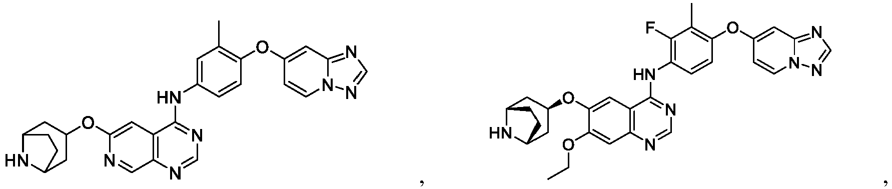 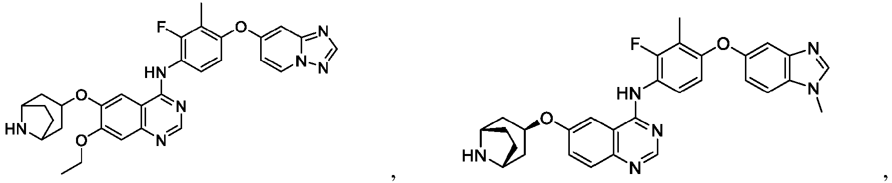 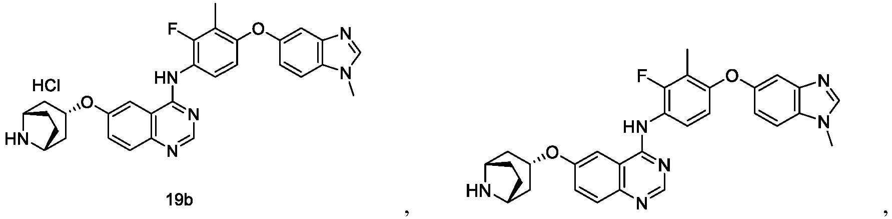 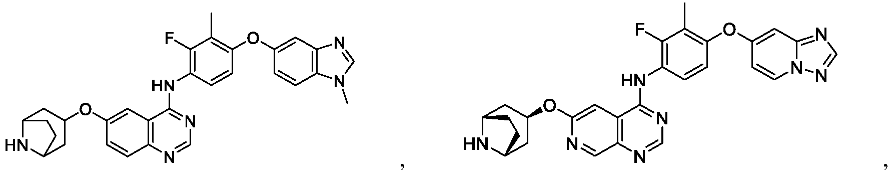 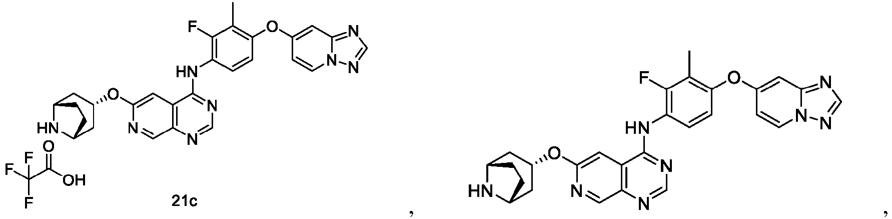 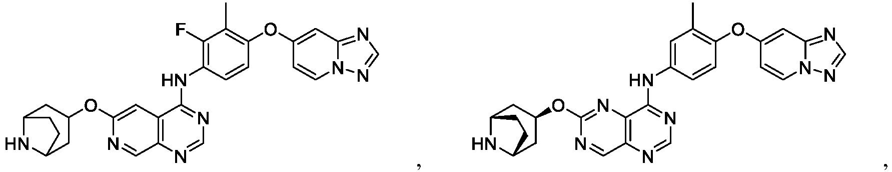 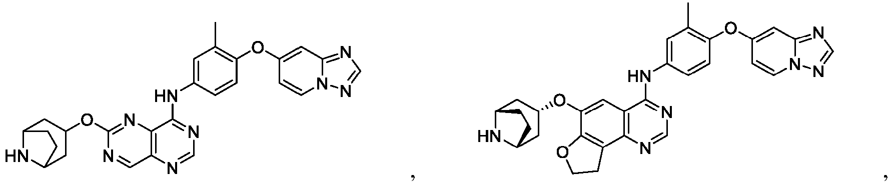 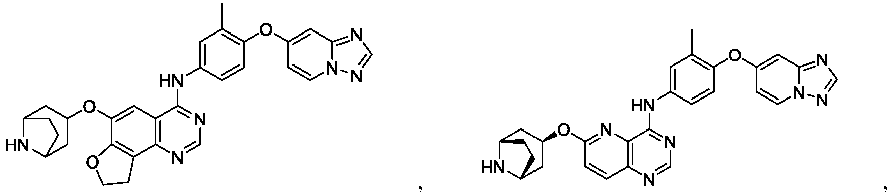 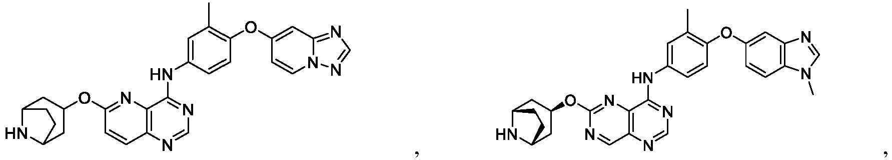 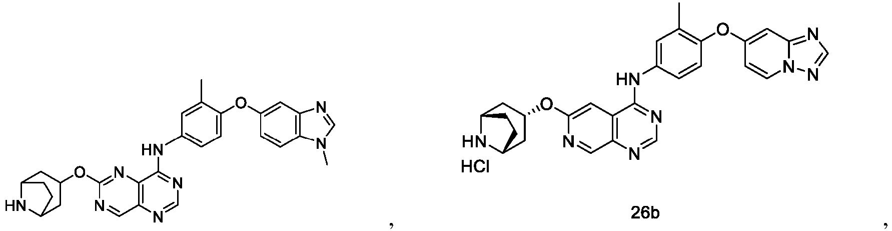 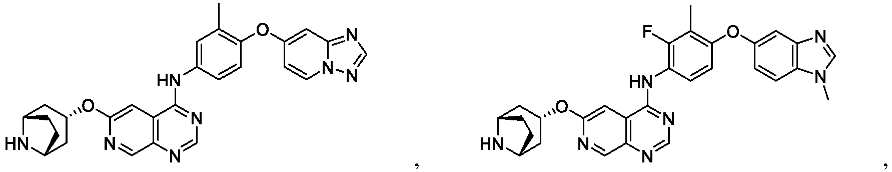 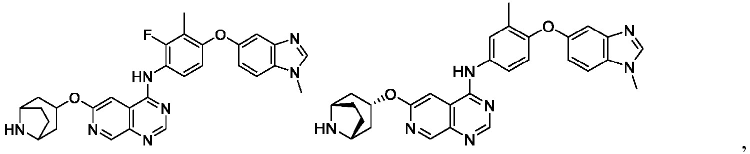 and

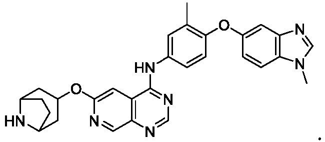

18. A method for preparing a compound of general formula (II) or a pharmaceutically acceptable salt thereof, comprising the following step: 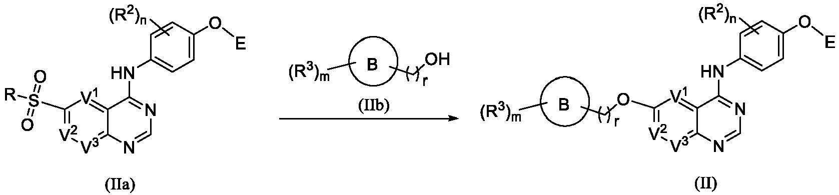 conducting a nucleophilic substitution reaction of a compound of general formula (IIa) or a salt thereof with a compound of general formula (IIb) or a salt thereof to give the compound of general formula (II) or the pharmaceutically acceptable salt thereof;
wherein:
R is C₁₋₆ alkyl; preferably, R is methyl;
E, ring B, R², R³, V¹, V², V³, r, m, and n are as defined in claim 8.

19. A method for preparing a compound of general formula (III) or a pharmaceutically acceptable salt thereof, comprising the following step: 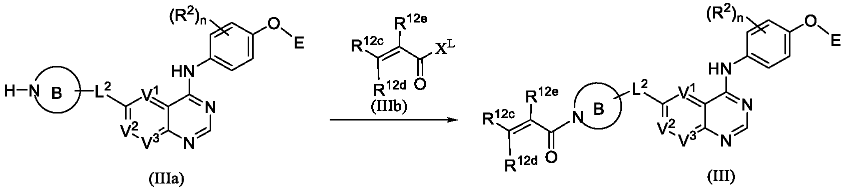
conducting a condensation reaction of a compound of general formula (IIIa) or a salt thereof with a compound of general formula (IIIb) or a salt thereof to give the compound of general formula (III) or the pharmaceutically acceptable salt thereof;
wherein:
X^{L} is halogen; preferably, X^{L} is chlorine;
E, ring B, R², L², V¹, V², V³, R^{12c}, R^{12d}, R^{12e}, and n are as defined in claim 11.

20. A pharmaceutical composition, wherein the pharmaceutical composition comprises the compound or the pharmaceutically acceptable salt thereof according to any one of claims 1 to 15, and one or more pharmaceutically acceptable carriers, diluents, or excipients.

21. Use of the compound or the pharmaceutically acceptable salt thereof according to any one of claims 1 to 15 or the pharmaceutical composition according to claim 20 in the preparation of a HER2 inhibitor.

22. Use of the compound or the pharmaceutically acceptable salt thereof according to any one of claims 1 to 15 or the pharmaceutical composition according to claim 20 in the preparation of a drug for treating and/or preventing a disease or disorder by inhibiting HER2, wherein preferably, the disease or disorder is cancer.

23. Use of the compound or the pharmaceutically acceptable salt thereof according to any one of claims 1 to 15 or the pharmaceutical composition according to claim 20 in the preparation of a drug for treating and/or preventing cancer, wherein the cancer is preferably selected from the group consisting of brain cancer, breast cancer, ovarian cancer, lung cancer, anal cancer, melanoma, neuroblastoma, colorectal cancer, cervical cancer, fallopian tube cancer, endometrial cancer, prostate cancer, gastric cancer, head and neck cancer, nasopharyngeal cancer, oral cancer, bile duct cancer, esophageal cancer, liver cancer, skin cancer, mesothelioma, bladder cancer, renal cell cancer, renal pelvis cancer, ureter cancer, small intestine cancer, pancreatic cancer, thyroid cancer, parathyroid cancer, vaginal cancer, vulva cancer, leukemia, adrenal cancer, cancer of the urinary tract, penile cancer, testicular cancer, bone cancer, osteosarcoma, myeloma, soft tissue sarcoma, pituitary adenoma, brain stem neuroglioma, spinal tumor, and lymphoma; more preferably, the cancer is selected from the group consisting of breast cancer, gastric cancer, lung cancer, colorectal cancer, pancreatic cancer, prostate cancer, bladder cancer, and ovarian cancer.
